# EUROPEAN PATENT APPLICATION

(11) **EP 4 810 516 A1**
(43) Date of publication of application: **23.09.2026**
(21) Application number: 24890803.0
(22) Date of filing: 15.11.2024
(51) Int. Cl.: C07K 16/28, C07K 16/30, C12N 15/13, C12N 15/85, A61K 39/395, A61P 35/00

(54) **TRGV9 BINDING PROTEINS AND MEDICAL USE THEREOF**

(30) Priority: 15.11.2023 CN 202311518247
(71) Applicant: Shanghai Shengdi Pharmaceutical Co., Ltd., Shanghai 201210 (CN); Jiangsu Hengrui Pharmaceuticals Co., Ltd., Lianyungang, Jiangsu 222047 (CN)
(72) Inventor: HU, Yali, Shanghai 201210 (CN); SHI, Jiajie, Shanghai 201210 (CN); CHEN, Simeng, Shanghai 201210 (CN); ZHANG, Wei, Shanghai 201210 (CN); LIAO, Cheng, Lianyungang, Jiangsu 222047 (CN)
(74) Representative: Regimbeau
(86) International application number: PCT/CN2024/132266
(87) International publication number: WO 2025/103451

(57) **Abstract**

The present disclosure relates to TRGV9 binding proteins and the medical use thereof. Specifically, the present disclosure relates to a TRGV9 binding protein, a GPC3/TRGV9 binding protein, a method for treating cancer using same, and the pharmaceutical use thereof.

## Description

The present application claims priority to Chinese Patent Application No. CN202311518247.8 filed on November 15, 2023.

### TECHNICAL FIELD

The present disclosure relates to the field of biopharmaceuticals and particularly to a TRGV9-binding protein and a GPC3/TRGV9-binding protein, a method for treating cancer using same, and pharmaceutical use thereof.

### BACKGROUND

γδ T cells are a group of T cells that naturally recognize and can kill tumor cells. They have both an antigen-presenting function and a killing function, bridging the innate immunity and the adaptive immunity. Unlike the αβ TCR, the γδ T cell TCR consists of two chains: a γ chain and a δ chain, and their recognition of antigens is not restricted by the MHC (Exp Mol Med. 2021 Mar;53(3):318-327). The number of γδ T cells is relatively small-they account for about 1%-5% of PBMCs. According to δ chain, they can be classified into four classes: δ1, δ2, δ3, and δ5 (Front Immunol. 2022 Jun 16;13:915837). δ1 and δ2 are the most abundant of the four classes. δ1 can pair with different γ chains to form different TCRs and is mainly distributed in tissues such as skin and mucosae. δ2 is mainly paired with γ9 to form a γ9δ2 subtype, is mainly distributed in peripheral blood, and accounts for up to 95% of the total number of γδ T cells in peripheral blood (Front Immunol. 2022 Jun 16;13:915837).

γδ T cells play an important role in combating tumors. They have a variety of mechanisms to kill tumor cells, including recognizing the BTN2A/BTN3A complex activated by phosphorylated antigens on target cells through TCRs and initiating a killing signal to fulfill a killing function. In addition, there are killing pathways similar to NK cells, in which receptors such as NKG2D are expressed on the cell surface, and tumor-killing effects are produced by binding to corresponding ligands on tumor cells. γδ T cells infiltrate various tumors to different extents. According to a large-sample analysis of 25 tumor types and 5782 tumors, the presence of γδ T cell infiltration is associated with a favorable prognosis for a patient, and they are the most favorable of all immune cell populations. Similarly, an analysis of 3238 samples from 14 non-brain cancer solid tumors shows that their infiltration of tumors is indicative of a good prognosis (Oncoimmunology. 2017 Feb 6;6(3):e1284723). Early clinical studies on γδ T cells mainly focused on either *in vivo* activation or reinfusion of *in vitro* expanded cells. Although different studies showed relatively limited anti-tumor effects, no serious side effects were reported, suggesting that γδ T cells have good safety.

Glypican-3 (GPC3) is a potential target for the treatment of liver cancer. In 2020, there were 900,000 new cases of liver cancer and 830,000 deaths from liver cancer worldwide, and there were 410,000 new cases and 390,000 deaths in China, which means that about half of the world's liver cancer cases were in China. In recent years, significant progress has been made in first- or second-line treatments for advanced liver cancer; however, there is still a lack of effective treatment options for later lines of therapy. GPC3 is a heparan sulfate proteoglycan consisting of 580 amino acids, anchored to the cell membrane via glycosylphosphatidylinositol. GPC3 is highly expressed in various tumor tissues. For example, the positivity rate of GPC3 in hepatocellular carcinoma is up to 90%. In contrast, GPC3 expression in normal tissues is very restricted, limited to the placenta and endometrium. Moreover, its expression level in the endometrium is much lower than that in tumor tissues, making it an ideal anti-tumor target (Sci Transl Med. 2017 Oct 4;9(410):eaal4291).

The present disclosure provides a γδTCR-targeting antibody with a new sequence structure, and uses the antibody and a tumor-associated antigen (e.g., GPC3)-binding domain (e.g., an antibody) to construct a bispecific antibody. One end of the bispecific antibody binds to the TAA (e.g., GPC3), and the other end binds to the γδTCR, recruiting and activating γδ T cells to specifically kill TAA (e.g., GPC3)-positive tumor cells, which significantly improves the effectiveness of γδ T cell therapy. The bispecific antibody of the present disclosure has good tumor-killing activity, safety, and drug developability.

### SUMMARY

The present disclosure provides a T cell receptor (TCR)-binding protein and a glypican-3 (GPC3) and T cell receptor (TCR)-binding protein, coding nucleic acids therefor, a preparation method therefor, and a method and use thereof for treating diseases.

### T Cell Receptor γ Variable Region 9 (TRGV9)-Binding Protein

The present disclosure provides a TRGV9-binding protein. In some embodiments, the protein is capable of binding to a TCR. In some embodiments, the protein is capable of binding to a γδTCR. In some embodiments, the protein is capable of binding to a γ9 chain of the TCR. In some embodiments, the protein is capable of binding to a γ9δ2 TCR. In some other embodiments, the protein is capable of binding to a γ9δ1 TCR.

In some embodiments, provided is a TRGV9-binding protein comprising:
1) an immunoglobulin single variable domain comprising a CDR1, a CDR2, and/or a CDR3 from the amino acid sequence set forth in any one of SEQ ID NOs: 5 and 26-29, for example, an immunoglobulin single variable domain comprising a CDR3 from the amino acid sequence set forth in any one of SEQ ID NOs: 5 and 26-29; or
2) a heavy chain variable region (VH) and/or a light chain variable region (VL), wherein the VH comprises a HCDR1, a HCDR2, and a HCDR3 from the amino acid sequence set forth in any one of SEQ ID NOs: 9 and 30-32, and the VL comprises a LCDR1, a LCDR2, and a LCDR3 from the amino acid sequence set forth in any one of SEQ ID NOs: 10 and 33-35,
wherein the CDRs are defined according to the Kabat, IMGT, Chothia, AbM, or Contact numbering scheme, for example, according to the Kabat numbering scheme.

In some embodiments, provided is a TRGV9-binding protein comprising:
1) an immunoglobulin single variable domain comprising a CDR1, a CDR2, and/or a CDR3, wherein the CDR1 comprises the amino acid sequence set forth in SEQ ID NO: 6, the CDR2 comprises the amino acid sequence set forth in SEQ ID NO: 7, and the CDR3 comprises the amino acid sequence set forth in SEQ ID NO: 8; or
2) a VH and/or a VL, wherein the VH comprises a HCDR1, a HCDR2, and/or a HCDR3, wherein the HCDR1 comprises the amino acid sequence set forth in SEQ ID NO: 11, the HCDR2 comprises the amino acid sequence set forth in SEQ ID NO: 12, and the HCDR3 comprises the amino acid sequence set forth in SEQ ID NO: 13; the VL comprises a LCDR1, a LCDR2, and/or a LCDR3, wherein the LCDR1 comprises the amino acid sequence set forth in SEQ ID NO: 14, the LCDR2 comprises the amino acid sequence set forth in SEQ ID NO: 15, and the LCDR3 comprises the amino acid sequence set forth in SEQ ID NO: 16.

In some embodiments, provided is a TRGV9-binding protein comprising:
1) an immunoglobulin single variable domain comprising a CDR1, a CDR2, and a CDR3 whose amino acid sequences are set forth in SEQ ID NOs: 6-8, respectively; or
2) a VH and a VL, wherein the VH comprises a HCDR1, a HCDR2, and a HCDR3 whose amino acid sequences are set forth in SEQ ID NOs: 11-13, respectively, and the VL comprises a LCDR1, a LCDR2, and a LCDR3 whose amino acid sequences are set forth in SEQ ID NOs: 14-16, respectively.

In some embodiments, the immunoglobulin single variable domain or the VH or the VL in the aforementioned protein is independently engineered by humanization, back mutation, affinity maturation, T cell epitope (TCE) removal/reduction, reduction of antibody deamidation, and/or reduction of antibody isomerization.

In some embodiments, heavy chain framework regions of a human germline template used for humanization of the immunoglobulin single variable domain in the aforementioned protein are derived from IGHV3-64; heavy chain framework regions of a human germline template used for humanization engineering of the VH are derived from IGHV3-21, and light chain framework regions of a human germline template used for humanization engineering of the VL are derived from IGKV1-12.

In some embodiments, provided is a TRGV9-binding protein comprising:
1) an immunoglobulin single variable domain comprising the amino acid sequence set forth in any one of SEQ ID NOs: 5 and 26-29 or an amino acid sequence having at least 80% or at least 90% identity thereto; or
2) a VH and a VL, wherein the VH comprises the amino acid sequence set forth in any one of SEQ ID NOs: 9 and 30-33 or an amino acid sequence having at least 80% or at least 90% identity thereto, and the VL comprises the amino acid sequence set forth in any one of SEQ ID NOs: 10 and 33-35 or an amino acid sequence having at least 80% or at least 90% identity thereto.

In some specific embodiments, the TRGV9-binding protein comprises the following VH and VL:
2-1) the VH comprises the amino acid sequence set forth in SEQ ID NO: 9 or an amino acid sequence having at least 80% or at least 90% identity thereto, and the VL comprises the amino acid sequence set forth in SEQ ID NO: 10 or an amino acid sequence having at least 80% or at least 90% identity thereto;
2-2) the VH comprises the amino acid sequence set forth in SEQ ID NO: 30 or an amino acid sequence having at least 80% or at least 90% identity thereto, and the VL comprises the amino acid sequence set forth in any one of SEQ ID NOs: 33-35 or an amino acid sequence having at least 80% or at least 90% identity thereto;
2-3) the VH comprises the amino acid sequence set forth in SEQ ID NO: 31 or an amino acid sequence having at least 80% or at least 90% identity thereto, and the VL comprises the amino acid sequence set forth in any one of SEQ ID NOs: 33-35 or an amino acid sequence having at least 80% or at least 90% identity thereto;
2-4) the VH comprises the amino acid sequence set forth in SEQ ID NO: 32 or an amino acid sequence having at least 80% or at least 90% identity thereto, and the VL comprises the amino acid sequence set forth in any one of SEQ ID NOs: 33-35 or an amino acid sequence having at least 80% or at least 90% identity thereto.

In the present disclosure, "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity; "at least 90% (sequence) identity" encompasses at least 90%, at least 91%, at least 92%, at least 93%, at least 94%, at least 95%, at least 96%, at least 97%, at least 98%, at least 99%, or 100% (sequence) identity.

In some embodiments, the aforementioned TRGV9-binding protein comprises or is an anti-TRGV9 antibody or an antigen-binding fragment thereof. In some embodiments, the anti-TRGV9 antibody or the antigen-binding fragment thereof is a recombinant antibody or a fragment thereof. In some embodiments, the anti-TRGV9 antibody is a monospecific antibody, a bispecific antibody, or a multispecific antibody (e.g., a trispecific antibody). When the aforementioned TRGV9-binding protein comprises an immunoglobulin single variable domain, the immunoglobulin single variable domain may be a camelid antibody, a chimeric antibody, a humanized antibody, or a fully human antibody, or an antigen-binding fragment thereof. For example, the immunoglobulin single variable domain is a single-domain antibody or a VHH. As an example, the aforementioned TRGV9-binding protein itself is a single-domain antibody or a VHH. In some specific embodiments, the antibody or the antigen-binding fragment thereof is a linear antibody, a single-chain antibody, a nanobody, a peptibody, a domain antibody, a diabody, a triabody, a tetrabody, a tandem di-scFv, or a tandem tri-scFv.

When the aforementioned TRGV9-binding protein comprises a VH and a VL, the protein may be a murine-derived antibody, a chimeric antibody, a humanized antibody, or a fully human antibody, or an antigen-binding fragment thereof. In some specific embodiments, the antigen-binding fragment includes, but is not limited to, a Fab, an Fv, an sFv, a Fab', a F(ab')2, a linear antibody, a single-chain antibody, an scFv, an sdAb, an sdFv, a nanobody, a peptibody, a domain antibody, a diabody, a triabody, a tetrabody, a tandem di-scFv, and a tandem tri-scFv. In some specific embodiments, the antigen-binding fragment includes a Fab, an Fv, an sFv, a Fab', and a F(ab')2.

In some embodiments, the aforementioned TRGV9-binding protein may comprise one or more (e.g., 2, 3, 4, 5, 6, 7, or 8) aforementioned immunoglobulin single variable domains. The immunoglobulin single variable domains may form a dimeric or multimeric molecule. The immunoglobulin single variable domains may be homodimers or heterodimers.

In some embodiments, the aforementioned TRGV9-binding protein may comprise one or more (e.g., 2, 3, 4, 5, 6, 7, or 8) aforementioned VHs, VLs, or combinations thereof.

In some embodiments, the aforementioned TRGV9-binding protein further comprises a human immunoglobulin Fc region; for example, the Fc region is a human IgG1, IgG2, IgG3, or IgG4 Fc region. In some embodiments, the Fc region is a human IgG1 Fc region; for example, the Fc region is set forth in SEQ ID NO: 51 or has at least 80% or at least 90% sequence identity thereto. In some specific embodiments, the Fc region may be an Fc region with a reduced effector function; for example, the Fc region may comprise a mutation, and exemplary IgG Fc regions with a reduced effector function comprise the following substitutions: N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); L234F/L235E (IgG1); L234F or L235E (IgG1); or L234A or L235A (IgG1) or S267E/L328F (IgG1). For example, L234A/L235A means that the sequence comprises L234A and L235A.

In some embodiments, the immunoglobulin single variable domain or the VH and the VL and the Fc region in the aforementioned TRGV9-binding protein are linked, either directly or by a linker. The linker may be a non-functional amino acid sequence of 1-20 or more amino acids lacking a secondary or higher-order structure. For example, the linker is represented by (GₘSₙ)ₕ or (GₘQₙ)ₕ or (GGNGT)ₕ (SEQ ID NO: 62) or (YGNGT)ₕ (SEQ ID NO: 63) or (EPKSS)ₕ (SEQ ID NO: 64) or (AₘSₙ)ₕ, wherein m and n are each independently selected from the group consisting of integers of 1-8, and h is independently selected from the group consisting of integers of 1-20. For example, the linker is selected from the group consisting of G₄S (SEQ ID NO: 65), GS, GAP, (G₄S)₂ (SEQ ID NO: 66), (G₄S)₃ (SEQ ID NO: 67), (G₄S)₄ (SEQ ID NO: 68), (G₄S)₅ (SEQ ID NO: 69), ASGS (SEQ ID NO: 70), A₃S (SEQ ID NO: 71), etc.

In some embodiments, the aforementioned TRGV9-binding protein is: a protein that specifically binds to the γδTCR, or an anti-γδTCR antibody or an antigen-binding fragment thereof; a protein that specifically binds to the γ9 chain of the TCR, or an antibody against the γ9 chain of the TCR or an antigen-binding fragment thereof; a protein that specifically binds to a variable region of the γ9 chain of the TCR (Vγ9), or an anti-Vγ9 TCR antibody or an antigen-binding fragment thereof; a protein that specifically binds to the γ9δ2 TCR, or an anti-γ9δ2 TCR antibody or an antigen-binding fragment thereof; a protein that specifically binds to the γ9δ1 TCR, or an anti-γ9δ1 TCR antibody or an antigen-binding fragment thereof; or a protein that specifically binds to TRGV9, or an anti-TRGV9 antibody or an antigen-binding fragment thereof.

In some embodiments, the aforementioned TRGV9-binding protein comprises:
1) the amino acid sequence set forth in SEQ ID NO: 36 or an amino acid sequence having at least 80% or at least 90% sequence identity thereto; and
2) a heavy chain and a light chain, wherein the heavy chain is set forth in SEQ ID NO: 37 or has at least 80% or at least 90% sequence identity thereto, and the light chain is set forth in SEQ ID NO: 38 or has at least 80% or at least 90% sequence identity thereto. In some embodiments, the aforementioned TRGV9-binding protein has at least one function or property selected from the group consisting of the following:
   (a) binding to γ9δ2 T cells with an EC₅₀ of ≤10 nM, e.g., ≤5 nM, ≤4 nM, ≤3 nM, ≤2 nM, ≤1 nM, ≤0.5 nM, ≤0.2 nM, or ≤0.1 nM, wherein the EC₅₀ is determined by FACS, which is a common affinity determination method in the art, e.g., the method described in Example 5 of the present disclosure;
   (b) specific binding to the γ9 chain of the TCR, e.g., specific binding to the γ9δ1 TCR or the γ9δ2 TCR;
   (c) no binding (undetectable by a test method) to a γ8 chain of the TCR, e.g., no binding to a γ8δ2 TCR;
   (d) specific binding to the variable region of the γ9 chain of the TCR (TRGV9);
   (e) specific binding to a VγδCαβ chimeric TCR and no binding to a VαβCγδ chimeric TCR; and
   (f) binding to a cynomolgus monkey γ9δ2 TCR;
wherein (b)-(e) can be known by a conventional detection method in the art, e.g., the method described in Example 4 of the present disclosure.

In some embodiments, the aforementioned protein may bind to TRGV9 with a K_{D} value of ≤1 × 10⁻⁷ M, e.g., ≤1 × 10⁻⁸ M, or ≤1 × 10⁻⁹ M, or ≤1 × 10⁻¹⁰ M.

In some embodiments, the aforementioned TRGV9-binding protein encompasses a variant of the immunoglobulin single variable domain, wherein the variant comprises one or more amino acid mutations compared to any one of SEQ ID NOs: 5 and 26-29. In some embodiments, the aforementioned TRGV9-binding protein encompasses a variant of the VH and/or a variant of the VL, wherein the variant of the VH comprises one or more amino acid mutations compared to any one of SEQ ID NOs: 9 and 30-32, and the variant of the VL comprises one or more amino acid mutations compared to any one of SEQ ID NOs: 10 and 33-35. "More" encompasses 1, 2, 3, 4, 5, 6, 7, 8, 9, and 10. The amino acid mutations may be conservative replacements, substitutions, or modifications, and/or deletions or additions that do not affect function; the amino acid mutations may occur in the CDRs and/or the FRs.

In some embodiments, provided is a protein that binds to or competes for binding to the same epitope with the immunoglobulin single variable domain in the aforementioned TRGV9-binding protein of the present disclosure.

In some embodiments, provided is a protein that binds to or competes for binding to the same epitope with the VH and the VL in the aforementioned TRGV9-binding protein of the present disclosure.

In some embodiments, provided is a protein whose binding to TRGV9 is blocked by the immunoglobulin single variable domain or the VH and the VL in the aforementioned TRGV9-binding protein of the present disclosure.

In some embodiments, provided is a protein or molecule comprising any one or more (e.g., 1, 2, 3, 4, 5, 6, 7, 8, 9, or 10) aforementioned immunoglobulin single variable domains of the present disclosure; for example, the immunoglobulin single variable domains comprise a CDR1, a CDR2, and a CDR3 set forth in SEQ ID NOs: 6-8, or comprise the sequence set forth in any one of SEQ ID NOs: 5 and 26-29. The protein or molecule may be a conjugate or fusion protein formed with other compounds or other polypeptides, and the conjugate may comprise, for example, any detectable label.

In some embodiments, provided is a protein or molecule comprising any one or more (e.g., 1, 2, 3, 4, 5, 6, 7, 8, 9, or 10) aforementioned VHs and VLs of the present disclosure; for example, the VHs comprise a HCDR1, a HCDR2, and a HCDR3 set forth in SEQ ID NOs: 11-13, and the VLs comprise a LCDR1, a LCDR2, and a LCDR3 whose amino acid sequences are set forth in SEQ ID NOs: 14-16, respectively. The protein or molecule may be a conjugate or fusion protein formed with other compounds or other polypeptides. For example, the conjugate may comprise any detectable label.

### GPC3/TRGV9-Binding Protein

The present disclosure provides a protein that binds to GPC3 and a TCR. In some embodiments, provided is a protein that binds to GPC3 and a γδTCR. In some embodiments, provided is a protein that binds to GPC3 and a γ9 chain of the TCR. In some embodiments, provided is a protein that binds to GPC3 and a variable region of the γ9 chain of the TCR (Vγ9). In some embodiments, provided is a protein that binds to GPC3 and a γ9δ2 TCR. In some embodiments, provided is a protein that binds to GPC3 and a γ9δ1 TCR. In some embodiments, provided is a protein that binds to GPC3 and TRGV9. In some embodiments, the "binding" is simultaneous or sequential binding.

The present disclosure provides a binding protein that binds to a tumor-associated antigen (TAA), a tumor-specific antigen, and TRGV9, comprising a binding domain that binds to the TAA (or the tumor-specific antigen) and a binding domain that binds to TRGV9. In some embodiments, the binding protein comprises a first antigen-binding domain that specifically binds to the TAA (or the tumor-specific antigen) and a second antigen-binding domain that specifically binds to TRGV9. In some embodiments, the second antigen-binding domain that specifically binds to TRGV9 is the aforementioned TRGV9-binding protein of the present disclosure. In some embodiments, the second antigen-binding domain that specifically binds to TRGV9 is the immunoglobulin single variable domain and/or a combination of the VH and the VL in the aforementioned TRGV9-binding protein of the present disclosure.

In some embodiments, provided is a GPC3/TRGV9-binding protein comprising a first antigen-binding domain that specifically binds to GPC3 and a second antigen-binding domain that specifically binds to TRGV9.

In some specific embodiments, the first antigen-binding domain comprises a heavy chain variable region (VH1) and/or a light chain variable region (VL1), wherein the VH1 comprises a HCDR1, a HCDR2, and a HCDR3 from the amino acid sequence set forth in SEQ ID NO: 39, and the VL1 comprises a LCDR1, a LCDR2, and a LCDR3 from the amino acid sequence set forth in SEQ ID NO: 40. In some specific embodiments, the first antigen-binding domain comprises a heavy chain variable region (VH1) and a light chain variable region (VL1), wherein the VH1 comprises a HCDR1, a HCDR2, and a HCDR3 from the amino acid sequence set forth in SEQ ID NO: 39, and the VL1 comprises a LCDR1, a LCDR2, and a LCDR3 from the amino acid sequence set forth in SEQ ID NO: 40; the CDRs are defined according to the Kabat, IMGT, Chothia, AbM, or Contact numbering scheme, e.g., according to the Kabat numbering scheme.

In some specific embodiments, the first antigen-binding domain comprises a heavy chain variable region (VH1) and a light chain variable region (VL1), wherein the VH1 comprises a HCDR1, a HCDR2, and a HCDR3 comprising the amino acid sequences set forth in SEQ ID NOs: 41-43, respectively, and the VL1 comprises a LCDR1, a LCDR2, and a LCDR3 comprising the amino acid sequences set forth in SEQ ID NOs: 44-46, respectively.

In some specific embodiments, the first antigen-binding domain comprises a heavy chain variable region (VH1) and a light chain variable region (VL1), wherein the VH1 comprises a HCDR1, a HCDR2, and a HCDR3 whose amino acid sequences are set forth in SEQ ID NOs: 41-43, respectively, and the VL1 comprises a LCDR1, a LCDR2, and a LCDR3 whose amino acid sequences are set forth in SEQ ID NOs: 44-46, respectively.

In some specific embodiments, the amino acid sequence of the first antigen-binding domain comprises any one or more sequences selected from the group consisting of SEQ ID NOs: 41-46.

In some specific embodiments, the VH1 and/or the VL1 are/is engineered by humanization, back mutation, affinity maturation, T cell epitope (TCE) removal/reduction, reduction of antibody deamidation, and/or reduction of antibody isomerization.

In some specific embodiments, the VH1 comprises the amino acid sequence set forth in SEQ ID NO: 39 or an amino acid sequence having at least 80% or at least 90% sequence identity thereto, and/or the VL1 comprises the amino acid sequence set forth in SEQ ID NO: 40 or an amino acid sequence having at least 80% or at least 90% sequence identity thereto.

In some specific embodiments, the amino acid sequence of the VH1 is set forth in SEQ ID NO: 39, and the amino acid sequence of the VL1 is set forth in SEQ ID NO: 40.

In some embodiments, provided is a GPC3/TRGV9-binding protein comprising any of the aforementioned first antigen-binding domains that specifically bind to GPC3 and a second antigen-binding domain that specifically binds to TRGV9, wherein the second antigen-binding domain that specifically binds to TRGV9 comprises:
1) an immunoglobulin single variable domain comprising a CDR1, a CDR2, and/or a CDR3 from the amino acid sequence set forth in any one of SEQ ID NOs: 5 and 26-29, for example, an immunoglobulin single variable domain comprising a CDR3 from the amino acid sequence set forth in any one of SEQ ID NOs: 5 and 26-29; or
2) a heavy chain variable region (VH2) and/or a light chain variable region (VL2), wherein the VH2 comprises a HCDR1, a HCDR2, and a HCDR3 from the amino acid sequence set forth in any one of SEQ ID NOs: 9 and 30-32, and the VL2 comprises a LCDR1, a LCDR2, and a LCDR3 from the amino acid sequence set forth in any one of SEQ ID NOs: 10 and 33-35,
wherein the CDRs are defined according to the Kabat, IMGT, Chothia, AbM, or Contact numbering scheme, for example, according to the Kabat numbering scheme.

In some embodiments, provided is a GPC3/TRGV9-binding protein comprising any of the aforementioned first antigen-binding domains that specifically bind to GPC3 and a second antigen-binding domain that specifically binds to TRGV9, wherein the second antigen-binding domain that specifically binds to TRGV9 comprises:
1) an immunoglobulin single variable domain comprising a CDR1, a CDR2, and/or a CDR3, wherein the CDR1 comprises the amino acid sequence set forth in SEQ ID NO: 6, the CDR2 comprises the amino acid sequence set forth in SEQ ID NO: 7, and the CDR3 comprises the amino acid sequence set forth in SEQ ID NO: 8; or
2) a VH2 and/or a VL2, wherein the VH2 comprises a HCDR1, a HCDR2, and/or a HCDR3, wherein the HCDR1 comprises the amino acid sequence set forth in SEQ ID NO: 11, the HCDR2 comprises the amino acid sequence set forth in SEQ ID NO: 12, and the HCDR3 comprises the amino acid sequence set forth in SEQ ID NO: 13; the VL comprises a LCDR1, a LCDR2, and/or a LCDR3, wherein the LCDR1 comprises the amino acid sequence set forth in SEQ ID NO: 14, the LCDR2 comprises the amino acid sequence set forth in SEQ ID NO: 15, and the LCDR3 comprises the amino acid sequence set forth in SEQ ID NO: 16.

In some embodiments, provided is a GPC3/TRGV9-binding protein comprising any of the aforementioned first antigen-binding domains that specifically bind to GPC3 and a second antigen-binding domain that specifically binds to TRGV9, wherein the second antigen-binding domain that specifically binds to TRGV9 comprises:
1) an immunoglobulin single variable domain comprising a CDR1, a CDR2, and a CDR3 whose amino acid sequences are set forth in SEQ ID NOs: 6-8, respectively; and/or
2) a VH2 and a VL2, wherein the VH2 comprises a HCDR1, a HCDR2, and a HCDR3 whose amino acid sequences are set forth in SEQ ID NOs: 11-13, respectively, and the VL2 comprises a LCDR1, a LCDR2, and a LCDR3 whose amino acid sequences are set forth in SEQ ID NOs: 14-16, respectively.

In some embodiments, the aforementioned immunoglobulin single variable domain or VH or VL is engineered by humanization, back mutation, affinity maturation, T cell epitope (TCE) removal/reduction, reduction of antibody deamidation, and/or reduction of antibody isomerization.

In some embodiments, heavy chain framework regions of a human germline template used for humanization of the aforementioned immunoglobulin single variable domain are derived from IGHV3-64; heavy chain framework regions of a human germline template used for humanization engineering of the VH are derived from IGHV3-21, and light chain framework regions of a human germline template used for humanization engineering of the VL are derived from IGKV1-12.

In some embodiments, provided is a GPC3/TRGV9-binding protein comprising any of the aforementioned first antigen-binding domains that specifically bind to GPC3 and a second antigen-binding domain that specifically binds to TRGV9, wherein the second antigen-binding domain that specifically binds to TRGV9 comprises:
1) an immunoglobulin single variable domain comprising the amino acid sequence set forth in any one of SEQ ID NOs: 5 and 26-29 or an amino acid sequence having at least 80% or at least 90% identity thereto; or
2) a VH2 and/or a VL2, wherein the VH2 comprises the amino acid sequence set forth in any one of SEQ ID NOs: 9 and 30-32 or an amino acid sequence having at least 80% or at least 90% identity thereto, and the VL2 comprises the amino acid sequence set forth in any one of SEQ ID NOs: 10 and 33-35 or an amino acid sequence having at least 80% or at least 90% identity thereto.

In some specific embodiments, a combination of a VH2 and a VL2 is selected from the group consisting of the following:
2-1) the VH2 comprises the amino acid sequence set forth in SEQ ID NO: 9 or an amino acid sequence having at least 80% or at least 90% identity thereto, and the VL2 comprises the amino acid sequence set forth in SEQ ID NO: 10 or an amino acid sequence having at least 80% or at least 90% identity thereto;
2-2) the VH2 comprises the amino acid sequence set forth in SEQ ID NO: 30 or an amino acid sequence having at least 80% or at least 90% identity thereto, and the VL2 comprises the amino acid sequence set forth in any one of SEQ ID NOs: 33-35 or an amino acid sequence having at least 80% or at least 90% identity thereto;
2-3) the VH2 comprises the amino acid sequence set forth in SEQ ID NO: 31 or an amino acid sequence having at least 80% or at least 90% identity thereto, and the VL2 comprises the amino acid sequence set forth in any one of SEQ ID NOs: 33-35 or an amino acid sequence having at least 80% or at least 90% identity thereto;
2-4) the VH2 comprises the amino acid sequence set forth in SEQ ID NO: 32 or an amino acid sequence having at least 80% or at least 90% identity thereto, and the VL2 comprises the amino acid sequence set forth in any one of SEQ ID NOs: 33-35 or an amino acid sequence having at least 80% or at least 90% identity thereto.

In some embodiments, the aforementioned GPC3/TRGV9-binding protein comprises or is an anti-GPC3/TRGV9 antibody or an antigen-binding fragment thereof. In some embodiments, the anti-GPC3/TRGV9 antibody or the antigen-binding fragment thereof is a recombinant antibody or a fragment thereof. In some embodiments, the anti-GPC3/TRGV9 antibody is a bispecific antibody or a multispecific antibody (e.g., a trispecific antibody). In some embodiments, an immunoglobulin single variable domain in the anti-GPC3/TRGV9 antibody or the antigen-binding fragment thereof is a single-domain antibody or a VHH. In some specific embodiments, the antigen-binding fragment includes, but is not limited to, a Fab, an Fv, an sFv, a Fab', a F(ab')2, a linear antibody, a single-chain antibody, an scFv, an sdAb, an sdFv, a nanobody, a peptibody, a domain antibody, a diabody, a triabody, a tetrabody, a tandem di-scFv, and a tandem tri-scFv.

In some embodiments, the aforementioned GPC3/TRGV9-binding protein comprises one or more (e.g., 2, 3, 4, 5, 6, 7, or 8) aforementioned immunoglobulin single variable domains. In some embodiments, the aforementioned GPC3/TRGV9-binding protein comprises one or more (e.g., 2, 3, 4, 5, 6, 7, or 8) combinations of the aforementioned VH1 and VL1. In some embodiments, the aforementioned GPC3/TRGV9-binding protein comprises one or more (e.g., 2, 3, 4, 5, 6, 7, or 8) combinations of the aforementioned VH2 and VL2.

In some embodiments, the aforementioned GPC3/TRGV9-binding protein further comprises a human immunoglobulin Fc region, e.g., a human IgG1, IgG2, IgG3, or IgG4 Fc region.

In some embodiments, the aforementioned GPC3/TRGV9-binding protein further comprises a human immunoglobulin Fc region; for example, the Fc region is a human IgG1, IgG2, IgG3, or IgG4 Fc region. In some embodiments, the Fc region is a human IgG1 Fc region; for example, the Fc region is set forth in SEQ ID NO: 51 or has at least 80% or at least 90% sequence identity thereto. In some specific embodiments, the Fc region may be an Fc region with a reduced effector function; for example, the Fc region may comprise a mutation, and exemplary IgG Fc regions with a reduced effector function comprise the following substitutions: N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); L234F/L235E (IgG1); L234F or L235E (IgG1); or L234A or L235A (IgG1) or S267E/L328F (IgG1).

In some specific embodiments, the Fc region comprises a first subunit (Fc1) and a second subunit (Fc2). In some specific embodiments, a mutation that causes the two subunits (Fc1 and Fc2) of the Fc region to be paired to form a dimer, or a mutation that reduces homodimerization, is introduced. In some specific embodiments, the first subunit and the second subunit comprise knob-into-hole mutations. For example, within a CH3/CH3 interface, one, two, or more amino acid residues in a CH3 domain of the Fc1 are mutated into one or more amino acid residues with larger side-chain volumes, thereby producing protuberances (or knobs) on the surface of the CH3 domain of the Fc1. Accordingly, one, two, or more amino acid residues in a CH3 domain of the Fc2 that interact with the CH3 domain of the Fc1 are mutated into amino acid residues with smaller side-chain volumes, thereby producing cavities (or holes) in the surface of the CH3 domain of the Fc2. The first subunit (Fc1) and the second subunit (Fc2) are only used to distinguish between two different subunits; thus, the two are interchangeable.

In some specific embodiments, the Fc1 comprises one or more amino acid substitutions at positions selected from the group consisting of 354, 356, 358, and 366, and the Fc2 comprises one or more amino acid substitutions at positions selected from the group consisting of 349, 356, 358, 366, 368, and 407. In some specific embodiments, the Fc1 comprises a mutation at position 366, and the Fc2 comprises a mutation selected from the group consisting of positions 366, 368, and 407 or any combination thereof. In some specific embodiments, the Fc1 comprises a mutation at position 354 or 356, and the Fc2 comprises a mutation at position 349. In some specific embodiments, the Fc1 comprises a mutation at position 354 or 356, and the Fc2 comprises mutations at positions 349, 366, 368, and 407.

In some specific embodiments, the Fc1 comprises one or more amino acid substitutions selected from the group consisting of 354C, 356E, 358M, and 366W, and the Fc2 comprises one or more amino acid substitutions selected from the group consisting of 349C, 356E, 358M, 366S, 368A, and 407V. In some specific embodiments, the Fc1 comprises mutation 366W, and the Fc2 comprises a mutation selected from the group consisting of 366S, 368A, and 407V or any combination thereof. In some specific embodiments, the Fc1 comprises mutation 354C or 356C, and the Fc2 comprises mutation 349C. Alternatively, in some specific embodiments, the Fc1 comprises mutations 354C/366W, and the Fc2 comprises mutations 349C/366S/368A/407V.

In some specific embodiments, the Fc1 comprises mutation T366W, and the Fc2 comprises a mutation selected from the group consisting of T366S, L368A, and Y407V or any combination thereof; the Fc1 comprises mutation S354C or E356C, and the Fc2 comprises mutation Y349C; or the Fc1 comprises mutations S354C/T366W, and the Fc2 comprises mutations Y349C/T366S/L368A/Y407V. In some specific embodiments, the amino acid sequence of the Fc1 is set forth in SEQ ID NO: 52, and the amino acid sequence of the Fc2 is set forth in SEQ ID NO: 53.

In some embodiments, the aforementioned GPC3/TRGV9-binding protein comprises reduced mispairing between light and heavy chains achieved by mutating amino acids at the interface between the heavy chain CH1 and the light chain CL in terms of amino acid size and charge. Illustratively, Roche swapped the domains of CH1 and CL and created the CrossMab platform (Schaefer et al., Proceedings of the National Academy of Sciences of the United States of America, 108(27), pp. 11187-11192 (2011)), MedImmune mutated the heavy chain (F126C) and light chain (S121C) to introduce a disulfide bond (Mazor et al., mAbs, 7(2), pp. 377-389 (2015)), Amgen further performed an electrostatic modification of the CH1-CL region (Liu et al., Journal of Biological Chemistry, 290(12), pp. 7535-7562 (2015)), and Lilly (Lewis et al., Nature Biotechnology, 32(2), pp. 191-198

(2014)) and Genentech (Dillon et al., mAbs, 9(2), pp. 213-230 (2017)) introduced mutations into both variable and constant domains." WuXi Biologics replaced a constant region of an antibody with a constant region of a TCR, which is described in CN109535257A (which is incorporated herein by reference in its entirety).

In some embodiments, the aforementioned GPC3/TRGV9-binding protein comprises an Obscurin-O chain (set forth in SEQ ID NO: 47) and a Titin-T chain (set forth in SEQ ID NO: 48) to prevent or reduce mispairing between different VHs and VLs.

In some embodiments, the aforementioned GPC3/TRGV9-binding protein comprises a linker.

In some embodiments, the immunoglobulin single variable domain or the VH and the VL and the Fc region in the aforementioned GPC3/TRGV9-binding protein are linked, either directly or by a linker. The linker may be a non-functional amino acid sequence of 1-20 or more amino acids lacking a secondary or higher-order structure. For example, the linker is represented by (GₘSₙ)ₕ or (GₘQₙ)ₕ or (GGNGT)ₕ or (YGNGT)ₕ or (EPKSS)ₕ or (AₘSₙ)ₕ, wherein m and n are each independently selected from the group consisting of integers of 1-8, and h is independently selected from the group consisting of integers of 1-20. For example, the linker is selected from the group consisting of G₄S, GS, GAP, (G₄S)₂, (G₄S)₃, (G₄S)₄, (G₄S)₅, ASGS, A₃S, etc.

In some embodiments, the GPC3/TRGV9-binding protein comprises a combination selected from the group consisting of the following:
1) a first heavy chain, a second heavy chain, and a light chain, wherein:
   the first heavy chain, from the N-terminus to the C-terminus, is [immunoglobulin single variable domain]-[linker 1]-[Fc1],
   the second heavy chain, from the N-terminus to the C-terminus, is [VH1]-[linker 2]-[CH1]-[linker 3]-[Fc2], and
   the light chain, from the N-terminus to the C-terminus, is [VL1]-[linker 4]-[CL];
      or
   the first heavy chain, from the N-terminus to the C-terminus, is [immunoglobulin single variable domain]-[linker 1]-[Fc2],
   the second heavy chain, from the N-terminus to the C-terminus, is [VH1]-[linker 2]-[CH1]-[linker 3]-[Fc1], and
   the light chain, from the N-terminus to the C-terminus, is [VL1]-[linker 4]-[CL];
   wherein - represents a peptide bond, and the linker 1, the linker 2, the linker 3, and the linker 4 may be identical or different, may independently be present or absent, and may be independently selected from the group consisting of the aforementioned linker of the present disclosure;
   in some specific embodiments, the linker 1 is AAAS, and the linker 2, the linker 3, and the linker 4 are absent;
2) a first heavy chain, a first light chain, a second heavy chain, and a second light chain, wherein:
   the first heavy chain, from the N-terminus to the C-terminus, is [VH2]-[linker 1]-[Obscurin-O chain]-[linker 2]-[Fc1],
   the first light chain, from the N-terminus to the C-terminus, is [VL2]-[linker 3]-[Titin-T chain],
   the second heavy chain, from the N-terminus to the C-terminus, is [VH1]-[linker 4]-[CH1]-[linker 5]-[Fc2], and
   the second light chain, from the N-terminus to the C-terminus, is [VL1]-[linker 6]-[CL]; or
   the first heavy chain, from the N-terminus to the C-terminus, is [VH1]-[linker 1]-[Obscurin-O chain]-[linker 2]-[Fc1],
   the first light chain, from the N-terminus to the C-terminus, is [VL1]-[linker 3]-[Titin-T chain],
   the second heavy chain, from the N-terminus to the C-terminus, is [VH2]-[linker 4]-[CH1]-[linker 5]-[Fc2], and
   the second light chain, from the N-terminus to the C-terminus, is [VL2]-[linker 6]-[CL], wherein - represents a peptide bond, and the linker 1, the linker 2, the linker 3, the linker 4, the linker 5, and the linker 6 may be identical or different, may independently be present or absent, and may be independently selected from the group consisting of the aforementioned linker of the present disclosure;
   in some specific embodiments, the linker 1 and the linker 3 are GGGGS, and the linker 2, the linker 4, the linker 5, and the linker 6 are absent;
3) a first heavy chain, a first light chain, a second heavy chain, and a second light chain, wherein:
   the first heavy chain, from the N-terminus to the C-terminus, is [VH2]-[linker 1]-[Obscurin-O chain]-[linker 2]-[Fc1],
   the first light chain, from the N-terminus to the C-terminus, is [VL2]-[linker 3]-[Titin-T chain],
   the second heavy chain, from the N-terminus to the C-terminus, is [VH1]-[linker 4]-[CH1]-[linker 5]-[VH1]-[linker 6]-[CH1]-[linker 7]-[Fc2], and
   the second light chain, from the N-terminus to the C-terminus, is [VL1]-[linker 8]-[CL]; or
   the first heavy chain, from the N-terminus to the C-terminus, is [VH1]-[linker 1]-[Obscurin-O chain]-[linker 2]-[Fc1],
   the first light chain, from the N-terminus to the C-terminus, is [VL1]-[linker 3]-[Titin-T chain],
   the second heavy chain, from the N-terminus to the C-terminus, is [VH2]-[linker 4]-[CH1]-[linker 5]-[VH2]-[linker 6]-[CH1]-[linker 7]-[Fc2], and
   the second light chain, from the N-terminus to the C-terminus, is [VL2]-[linker 8]-[CL]; wherein - represents a peptide bond, and the linker 1, the linker 2, the linker 3, the linker 4, the linker 5, the linker 6, the linker 7, and the linker 8 may be identical or different, may independently be present or absent, and may be independently selected from the group consisting of the aforementioned linker of the present disclosure;
   in some specific embodiments, the linker 1 and the linker 3 are GGGGS, the linker 5 is GGGGSGGGGS, and the linker 2, the linker 4, the linker 6, the linker 7, and the linker 8 are absent;
   in some specific embodiments, the molar ratio of the first heavy chain to the first light chain to the second heavy chain to the second light chain is 1:1:1:2.

In some specific embodiments, the amino acid sequences of the Obscurin-O chain and the Titin-T chain are set forth in SEQ ID NOs: 47 and 48, respectively. In some specific embodiments, the CL is a Cκ, and the amino acid sequences of the CH1 and the Cκ are set forth in SEQ ID NOs: 49 and 50, respectively. In some specific embodiments, the amino acid sequences of the Fc1 and the Fc2 are set forth in SEQ ID NOs: 52 and 53, respectively.

In some embodiments, provided is a GPC3/TRGV9-binding protein selected from the group consisting of:
1) comprising a first heavy chain whose amino acid sequence is set forth in SEQ ID NO: 54 or has at least 80% or at least 90% identity thereto, a second heavy chain whose amino acid sequence is set forth in SEQ ID NO: 55 or has at least 80% or at least 90% identity thereto, and a light chain whose amino acid sequence is set forth in SEQ ID NO: 56 or has at least 80% or at least 90% identity thereto;
2) comprising a first heavy chain whose amino acid sequence is set forth in SEQ ID NO: 57 or has at least 80% or at least 90% identity thereto, a first light chain whose amino acid sequence is set forth in SEQ ID NO: 58 or has at least 80% or at least 90% identity thereto, a second heavy chain whose amino acid sequence is set forth in SEQ ID NO: 59 or has at least 80% or at least 90% identity thereto, and a second light chain whose amino acid sequence is set forth in SEQ ID NO: 56 or has at least 80% or at least 90% identity thereto; and
3) comprising a first heavy chain whose amino acid sequence is set forth in SEQ ID NO: 57 or has at least 80% or at least 90% identity thereto, a first light chain whose amino acid sequence is set forth in SEQ ID NO: 58 or has at least 80% or at least 90% identity thereto, a second heavy chain whose amino acid sequence is set forth in SEQ ID NO: 60 or has at least 80% or at least 90% identity thereto, and a second light chain whose amino acid sequence is set forth in SEQ ID NO: 61 or has at least 80% or at least 90% identity thereto.

In some embodiments, provided is a GPC3/TRGV9-binding protein selected from the group consisting of:
1) comprising a combination of polypeptides whose amino acid sequences are set forth in SEQ ID NOs: 54-56; in some embodiments, the GPC3/TRGV9-binding protein has a molar ratio of the polypeptide set forth in SEQ ID NO: 54 to the polypeptide set forth in SEQ ID NO: 55 to the polypeptide set forth in SEQ ID NO: 56 of 1:1:1;
2) comprising a combination of polypeptides whose amino acid sequences are set forth in SEQ ID NOs: 56-59; in some embodiments, the GPC3/TRGV9-binding protein has a molar ratio of the polypeptide set forth in SEQ ID NO: 56 to the polypeptide set forth in SEQ ID NO: 57 to SEQ ID NO: 58 to the polypeptide set forth in SEQ ID NO: 59 of 1:1:1:1; and
3) comprising a combination of polypeptides whose amino acid sequences are set forth in SEQ ID NOs: 57, 58, 60, and 61; in some embodiments, the GPC3/TRGV9-binding protein has a molar ratio of the polypeptide set forth in SEQ ID NO: 57 to the polypeptide set forth in SEQ ID NO: 58 to SEQ ID NO: 60 to the polypeptide set forth in SEQ ID NO: 61 of 1:1:1:2.

In some embodiments, the aforementioned GPC3/TRGV9-binding protein is: a protein that specifically binds to GPC3 and the γδTCR, or an anti-GPC3/γδTCR antibody or an antigen-binding fragment thereof; a protein that specifically binds to GPC3 and the γ9 chain of the TCR, or an anti-GPC3/TCR γ9 chain antibody or an antigen-binding fragment thereof; a protein that specifically binds to GPC3 and the variable region of the γ9 chain of the TCR (Vγ9), or an anti-GPC3/Vγ9 TCR antibody or an antigen-binding fragment thereof; a protein that specifically binds to GPC3 and the γ9δ2 TCR, or an anti-GPC3/γ9δ2 TCR antibody or an antigen-binding fragment thereof; a protein that specifically binds to GPC3 and the γ9δ1 TCR, or an anti-GPC3/γ9δ1 TCR antibody or an antigen-binding fragment thereof; a protein that specifically binds to GPC3 and TRGV9, or an anti-GPC3/TRGV9 antibody or an antigen-binding fragment thereof.

In some embodiments, the aforementioned GPC3/TRGV9-binding protein has at least one function or property selected from the group consisting of the following:
(a) binding to γ9δ2 T cells with an EC₅₀ of ≤10 nM, e.g., ≤5 nM, ≤4 nM, ≤3 nM, ≤2 nM, ≤1 nM, ≤0.5 nM, ≤0.2 nM, or ≤0.1 nM, wherein the EC₅₀ is determined by FACS, which is a common affinity determination method in the art, e.g., the method described in Example 5 of the present disclosure;
(b) specific binding to the γ9 chain of the TCR, e.g., specific binding to the γ9δ1 TCR or specific binding to the γ9δ2 TCR;
(c) no binding to a γ8 chain of the TCR, e.g., no binding to a γ8δ2 TCR;
(d) specific binding to the variable region of the γ9 chain of the TCR (TRGV9);
(e) specific binding to a VγδCαβ chimeric TCR and no binding to a VαβCγδ chimeric TCR;
   wherein (b)-(e) can be known by a conventional detection method in the art, e.g., the method described in Example 4 of the present disclosure;
(f) specific binding to a GPC3 protein or GPC3-positive cells, and no or little binding to GPC3-negative cells (e.g., PBMCs), for example, using the detection method in Example 8 of the present disclosure;
(g) binding to cynomolgus monkey GPC3 and γ9δ2 proteins;
(h) no blocking of BTN2A/BTN3A-TCR natural signals, for example, using the detection method in Example 9 of the present disclosure;
(i) mediation of killing of GPC3-positive tumor cells by γδ T cells or mediation of killing of GPC3-positive tumor cells by γδ T cells in a GPC3 expression level-dependent manner, for example, using the detection method in Example 10 of the present disclosure; mediation of killing of GPC3-positive tumor cells by γδ T cells in an effector-to-target ratio-dependent manner, for example, using the detection method in Example 12 of the present disclosure; and mediation of killing of GPC3-positive tumor cells by γδ T cells in a low-individual variability manner, for example, using the detection method in Example 11 of the present disclosure;
(j) induction of less cytokine (e.g., IFNγ and TNFα) release during killing of GPC3-positive tumor cells by γδ T cells, for example, using the detection method in Example 13 of the present disclosure;
(k) promotion of proliferation of γδ T cells in PBMCs, for example, using the detection method in Example 14 of the present disclosure; and
(l) *in vivo* inhibition of proliferation of GPC3-positive tumor cells, and/or inhibition of growth of GPC3-positive tumors, and/or elimination of GPC3-positive tumors, wherein an *in vivo* mouse model is as the detection method in Example 15 of the present disclosure. In some embodiments, the aforementioned GPC3/TRGV9-binding protein may bind to TRGV9 with a K_{D} value of ≤1 × 10⁻⁷ M, e.g., ≤1 × 10⁻⁸ M, or ≤1 × 10⁻⁹ M, or ≤1 × 10⁻¹⁰ M.

In some embodiments, the aforementioned GPC3/TRGV9-binding protein may bind to GPC3 with a K_{D} value of ≤1 × 10⁻⁷ M, e.g., ≤1 × 10⁻⁸ M, or ≤1 × 10⁻⁹ M, or ≤1 × 10⁻¹⁰ M. In some embodiments, the immunoglobulin single variable domain in the aforementioned GPC3/TRGV9-binding protein encompasses a variant, wherein the variant comprises one or more amino acid mutations compared to any one of SEQ ID NOs: 5 and 26-29. In some embodiments, the VH1 and/or the VL1 in the aforementioned GPC3/TRGV9-binding protein encompass(es) a variant, wherein the variant of the VH1 comprises one or more amino acid mutations compared to SEQ ID NO: 39, and the variant of the VL comprises one or more amino acid mutations compared to SEQ ID NO: 40. In some embodiments, the VH2 and/or the VL2 in the aforementioned GPC3/TRGV9-binding protein encompass(es) a variant, wherein the variant of the VH2 comprises one or more amino acid mutations compared to any one of SEQ ID NOs: 9 and 30-32, and the variant of the VL comprises one or more amino acid mutations compared to any one of SEQ ID NOs: 10 and 33-35. "More" is, for example, 1, 2, 3, 4, 5, 6, 7, 8, 9, or 10. The amino acid mutations may be conservative replacements, substitutions, or modifications, and/or deletions or additions that do not affect function; the amino acid mutations may occur in the CDRs and/or the FRs.

In some embodiments, provided is a protein that binds to or competes for binding to the same TRGV9 and/or GPC3 epitope(s) with the aforementioned GPC3/TRGV9-binding protein of the present disclosure.

In some embodiments, provided is a protein whose binding to TRGV9 and/or GPC3 is blocked by the aforementioned GPC3/TRGV9-binding protein of the present disclosure. In some embodiments, provided is a protein or molecule comprising any of the aforementioned GPC3/TRGV9-binding proteins of the present disclosure. The protein or molecule may be a conjugate or fusion protein formed with other compounds or other polypeptides. For example, the conjugate may comprise any detectable label.

### Polynucleotide and Vector

The present disclosure provides a polynucleotide encoding the TRGV9-binding protein or GPC3/TRGV9-binding protein of the present disclosure. The nucleic acid of the present disclosure may be an RNA, DNA, or cDNA. According to some embodiments of the present disclosure, the nucleic acid of the present disclosure is a substantially isolated nucleic acid.

The nucleic acid of the present disclosure may also take the form of a vector and may be present in the vector and/or may be part of the vector. The vector is, for example, a plasmid, cosmid, YAC, or viral vector. The vector may especially be an expression vector, i.e., a vector that can provide expression of the TRGV9-binding protein or the GPC3/TRGV9-binding protein *in vitro* and/or *in vivo* (i.e., in a suitable host cell, host organism, and/or expression system). The expression vector generally comprises at least one of the nucleic acids of the present disclosure, which is operably linked to one or more suitable expression regulatory elements (e.g., promoters, enhancers, and terminators). The selection of the elements and sequences thereof for expression in a particular host is common knowledge of those skilled in the art. Regulatory elements and other elements useful or necessary for expression of the TRGV9-binding protein and the GPC3/TRGV9-binding protein of the present disclosure include, for example, promoters, enhancers, terminators, integration factors, selection labels, leader sequences, and reporter genes.

The nucleic acid of the present disclosure may be prepared or obtained by known means (e.g., by automatic DNA synthesis and/or recombinant DNA techniques) based on information about the amino acid sequences of the polypeptides of the present disclosure, and/or may be isolated from a suitable natural source.

In some embodiments, provided is a polynucleotide encoding a TRGV9-binding protein, wherein the TRGV9-binding protein comprises:
1) an immunoglobulin single variable domain comprising a CDR1, a CDR2, and/or a CDR3 from the amino acid sequence set forth in any one of SEQ ID NOs: 5 and 26-29, for example, an immunoglobulin single variable domain comprising a CDR3 from the amino acid sequence set forth in any one of SEQ ID NOs: 5 and 26-29; and/or
2) a heavy chain variable region (VH) and/or a light chain variable region (VL), wherein the VH comprises a HCDR1, a HCDR2, and/or a HCDR3 from the amino acid sequence set forth in any one of SEQ ID NOs: 9 and 30-32, and the VL comprises a LCDR1, a LCDR2, and/or a LCDR3 from the amino acid sequence set forth in any one of SEQ ID NOs: 10 and 33-35,
wherein the CDRs are defined according to the Kabat, IMGT, Chothia, AbM, or Contact numbering scheme, for example, according to the Kabat numbering scheme.

In some embodiments, provided is a polynucleotide encoding a TRGV9-binding protein, wherein the TRGV9-binding protein comprises:
1) an immunoglobulin single variable domain comprising a CDR1, a CDR2, and a CDR3 whose amino acid sequences are set forth in SEQ ID NOs: 6-8, respectively; and/or
2) a VH and a VL, wherein the VH comprises a HCDR1, a HCDR2, and a HCDR3 whose amino acid sequences are set forth in SEQ ID NOs: 11-13, respectively, and the VL comprises a LCDR1, a LCDR2, and a LCDR3 whose amino acid sequences are set forth in SEQ ID NOs: 14-16, respectively.

In some embodiments, provided is a polynucleotide encoding a TRGV9-binding protein, wherein the TRGV9-binding protein comprises: 1) an immunoglobulin single variable domain comprising the amino acid sequence set forth in any one of SEQ ID NOs: 5 and 26-29 or an amino acid sequence having at least 80% or at least 90% identity thereto; and/or
2) a VH and/or a VL, wherein the VH comprises the amino acid sequence set forth in any one of SEQ ID NOs: 9 and 30-33 or an amino acid sequence having at least 80% or at least 90% identity thereto, and the VL comprises the amino acid sequence set forth in any one of SEQ ID NOs: 10 and 33-35 or an amino acid sequence having at least 80% or at least 90% identity thereto.

In some specific embodiments, provided is a polynucleotide encoding a TRGV9-binding protein, wherein the TRGV9-binding protein comprises the following VH and VL:
2-1) the VH comprises the amino acid sequence set forth in SEQ ID NO: 9 or an amino acid sequence having at least 80% or at least 90% identity thereto, and the VL comprises the amino acid sequence set forth in SEQ ID NO: 10 or an amino acid sequence having at least 80% or at least 90% identity thereto;
2-2) the VH comprises the amino acid sequence set forth in SEQ ID NO: 30 or an amino acid sequence having at least 80% or at least 90% identity thereto, and the VL comprises the amino acid sequence set forth in any one of SEQ ID NOs: 33-35 or an amino acid sequence having at least 80% or at least 90% identity thereto;
2-3) the VH comprises the amino acid sequence set forth in SEQ ID NO: 31 or an amino acid sequence having at least 80% or at least 90% identity thereto, and the VL comprises the amino acid sequence set forth in any one of SEQ ID NOs: 33-35 or an amino acid sequence having at least 80% or at least 90% identity thereto;
2-4) the VH comprises the amino acid sequence set forth in SEQ ID NO: 32 or an amino acid sequence having at least 80% or at least 90% identity thereto, and the VL comprises the amino acid sequence set forth in any one of SEQ ID NOs: 33-35 or an amino acid sequence having at least 80% or at least 90% identity thereto.

In some specific embodiments, provided is a polynucleotide encoding a TRGV9-binding protein, wherein the TRGV9-binding protein comprises:
1) the amino acid sequence set forth in SEQ ID NO: 36 or an amino acid sequence having at least 80% or at least 90% sequence identity thereto; and
2) a heavy chain and a light chain, wherein the heavy chain is set forth in SEQ ID NO: 37 or has at least 80% or at least 90% sequence identity thereto, and the light chain is set forth in SEQ ID NO: 38 or has at least 80% or at least 90% sequence identity thereto.

In some embodiments, provided is a polynucleotide encoding a GPC3/TRGV9-binding protein, wherein the GPC3/TRGV9-binding protein comprises a first antigen-binding domain that specifically binds to GPC3 and a second antigen-binding domain that specifically binds to TRGV9.

In some specific embodiments, provided is a polynucleotide encoding a GPC3/TRGV9-binding protein, wherein the aforementioned first antigen-binding domain comprises a heavy chain variable region (VH1) and/or a light chain variable region (VL1), wherein the VH1 comprises a HCDR1, a HCDR2, and a HCDR3 from the amino acid sequence set forth in SEQ ID NO: 39, and the VL1 comprises a LCDR1, a LCDR2, and a LCDR3 from the amino acid sequence set forth in SEQ ID NO: 40.

In some specific embodiments, provided is a polynucleotide encoding a GPC3/TRGV9-binding protein, wherein the aforementioned first antigen-binding domain comprises a heavy chain variable region (VH1) and a light chain variable region (VL1), wherein the VH1 comprises a HCDR1, a HCDR2, and a HCDR3 whose amino acid sequences are set forth in SEQ ID NOs: 41-43, respectively, and the VL1 comprises a LCDR1, a LCDR2, and a LCDR3 whose amino acid sequences are set forth in SEQ ID NOs: 44-46, respectively.

In some specific embodiments, provided is a polynucleotide encoding a GPC3/TRGV9-binding protein, wherein the aforementioned first antigen-binding domain comprises a heavy chain variable region (VH1) and a light chain variable region (VL1), wherein the VH1 comprises the amino acid sequence set forth in SEQ ID NO: 39 or an amino acid sequence having at least 80% or at least 90% sequence identity thereto, and/or the VL1 comprises the amino acid sequence set forth in SEQ ID NO: 40 or an amino acid sequence having at least 80% or at least 90% sequence identity thereto.

In some specific embodiments, provided is a polynucleotide encoding a GPC3/TRGV9-binding protein, wherein the aforementioned second antigen-binding domain that specifically binds to TRGV9 comprises:
1) an immunoglobulin single variable domain comprising a CDR1, a CDR2, and/or a CDR3 from the amino acid sequence set forth in any one of SEQ ID NOs: 5 and 26-29, for example, an immunoglobulin single variable domain comprising a CDR3 from the amino acid sequence set forth in any one of SEQ ID NOs: 5 and 26-29; or
2) a heavy chain variable region (VH2) and/or a light chain variable region (VL2), wherein the VH2 comprises a HCDR1, a HCDR2, and a HCDR3 from the amino acid sequence set forth in any one of SEQ ID NOs: 9 and 30-32, and the VL2 comprises a LCDR1, a LCDR2, and a LCDR3 from the amino acid sequence set forth in any one of SEQ ID NOs: 10 and 33-35.

In some specific embodiments, provided is a polynucleotide encoding a GPC3/TRGV9-binding protein, wherein the aforementioned second antigen-binding domain that specifically binds to TRGV9 comprises:
1) an immunoglobulin single variable domain comprising a CDR1, a CDR2, and a CDR3 whose amino acid sequences are set forth in SEQ ID NOs: 6-8, respectively; and/or
2) a VH2 and a VL2, wherein the VH2 comprises a HCDR1, a HCDR2, and a HCDR3 whose amino acid sequences are set forth in SEQ ID NOs: 11-13, respectively, and the VL2 comprises a LCDR1, a LCDR2, and a LCDR3 whose amino acid sequences are set forth in SEQ ID NOs: 14-16, respectively.

In some specific embodiments, provided is a polynucleotide encoding a GPC3/TRGV9-binding protein, wherein the aforementioned second antigen-binding domain that specifically binds to TRGV9 comprises:
1) an immunoglobulin single variable domain comprising the amino acid sequence set forth in any one of SEQ ID NOs: 5 and 26-29 or an amino acid sequence having at least 80% or at least 90% identity thereto; or
2) a VH2 and/or a VL2, wherein the VH2 comprises the amino acid sequence set forth in any one of SEQ ID NOs: 9 and 30-32 or an amino acid sequence having at least 80% or at least 90% identity thereto, and the VL2 comprises the amino acid sequence set forth in any one of SEQ ID NOs: 10 and 33-35 or an amino acid sequence having at least 80% or at least 90% identity thereto.

In some specific embodiments, provided is a polynucleotide encoding a GPC3/TRGV9-binding protein, wherein the aforementioned second antigen-binding domain that specifically binds to TRGV9 comprises the following VH2 and VL2:
2-1) the VH2 comprises the amino acid sequence set forth in SEQ ID NO: 9 or an amino acid sequence having at least 80% or at least 90% identity thereto, and the VL2 comprises the amino acid sequence set forth in SEQ ID NO: 10 or an amino acid sequence having at least 80% or at least 90% identity thereto;
2-2) the VH2 comprises the amino acid sequence set forth in SEQ ID NO: 30 or an amino acid sequence having at least 80% or at least 90% identity thereto, and the VL2 comprises the amino acid sequence set forth in any one of SEQ ID NOs: 33-35 or an amino acid sequence having at least 80% or at least 90% identity thereto;
2-3) the VH2 comprises the amino acid sequence set forth in SEQ ID NO: 31 or an amino acid sequence having at least 80% or at least 90% identity thereto, and the VL2 comprises the amino acid sequence set forth in any one of SEQ ID NOs: 33-35 or an amino acid sequence having at least 80% or at least 90% identity thereto;
2-4) the VH2 comprises the amino acid sequence set forth in SEQ ID NO: 32 or an amino acid sequence having at least 80% or at least 90% identity thereto, and the VL2 comprises the amino acid sequence set forth in any one of SEQ ID NOs: 33-35 or an amino acid sequence having at least 80% or at least 90% identity thereto.

In some specific embodiments, provided is a polynucleotide encoding a GPC3/TRGV9-binding protein, wherein the GPC3/TRGV9-binding protein is selected from the group consisting of:
1) comprising a first heavy chain whose amino acid sequence is set forth in SEQ ID NO: 54 or has at least 80% or at least 90% identity thereto, a second heavy chain whose amino acid sequence is set forth in SEQ ID NO: 55 or has at least 80% or at least 90% identity thereto, and a light chain whose amino acid sequence is set forth in SEQ ID NO: 56 or has at least 80% or at least 90% identity thereto;
2) comprising a first heavy chain whose amino acid sequence is set forth in SEQ ID NO: 57 or has at least 80% or at least 90% identity thereto, a first light chain whose amino acid sequence is set forth in SEQ ID NO: 58 or has at least 80% or at least 90% identity thereto, a second heavy chain whose amino acid sequence is set forth in SEQ ID NO: 59 or has at least 80% or at least 90% identity thereto, and a second light chain whose amino acid sequence is set forth in SEQ ID NO: 56 or has at least 80% or at least 90% identity thereto; and
3) comprising a first heavy chain whose amino acid sequence is set forth in SEQ ID NO: 57 or has at least 80% or at least 90% identity thereto, a first light chain whose amino acid sequence is set forth in SEQ ID NO: 58 or has at least 80% or at least 90% identity thereto, a second heavy chain whose amino acid sequence is set forth in SEQ ID NO: 60 or has at least 80% or at least 90% identity thereto, and a second light chain whose amino acid sequence is set forth in SEQ ID NO: 61 or has at least 80% or at least 90% identity thereto.

### Host Cell

The present disclosure provides a recombinant host cell that expresses or is capable of expressing one or more of the TRGV9-binding proteins and GPC3/TRGV9-binding proteins of the present disclosure and/or comprises the polynucleotide or vector of the present disclosure. In some embodiments, the host cell is a bacterial cell, a fungal cell, or a mammalian cell.

Bacterial cells include, for example, cells of gram-negative bacterial strains (e.g., *Escherichia coli* strains, *Proteus* strains, and *Pseudomonas* strains) and gram-positive bacterial strains (e.g., *Bacillus* strains, *Streptomyces* strains, *Staphylococcus* strains, and *Lactococcus* strains).

Fungal cells include, for example, cells of the species of *Trichoderma*, *Neurospora*, and *Aspergillus*; or cells of the species of *Saccharomyces* (e.g., *Saccharomyces cerevisiae*), *Schizosaccharomyces* (e.g., *Schizosaccharomyces pombe*), *Pichia* (e.g., *Pichia pastoris* and *Pichia methanolica*), and *Hansenula*.

Mammalian cells include, for example, HEK293 cells, CHO cells, BHK cells, HeLa cells, and COS cells.

However, amphibian cells, insect cells, plant cells, and any other cells used in the art for expressing heterologous proteins may also be used in the present disclosure.

### Preparation Method

The present disclosure provides a method for preparing a TRGV9-binding protein or a GPC3/TRGV9-binding protein, comprising expressing the target protein in the aforementioned host cell and isolating the target protein from the host cell. Optionally, the method may further comprise a purification step, for example, purifying using an A or G Sepharose FF column containing an adjusted buffer to wash off non-specifically bound components, then eluting the bound antibody using a pH gradient method, detecting using SDS-PAGE, and collecting. Optionally, filtration and concentration are performed by conventional methods. Soluble mixtures and multimers can also be removed by conventional methods, such as molecular sieves and ion exchange. The resulting products need to be immediately frozen, such as at -70 °C, or lyophilized.

Methods for producing and purifying antibodies are well known in the prior art and can be found in, for example, Antibodies: A Laboratory Manual, Cold Spring Harbor Press (chapters 5-8 and 15).

The engineered antibodies or antigen-binding fragments of the present disclosure can be prepared and purified by conventional methods. For example, cDNA sequences encoding heavy and light chains can be cloned and recombined into an expression vector. CHO cells can be stably transfected with recombinant immunoglobulin expression vectors. Mammalian expression systems may result in glycosylation of antibodies, particularly at the highly conserved N-terminus of the Fc region. Stable clones are obtained by expression of antibodies specifically binding to human antigens. Positive clones are expanded in a serum-free culture medium in a bioreactor to produce antibodies. The culture solution with the secreted antibodies can be purified and collected by conventional techniques. Antibodies can be filtered and concentrated by conventional methods. Soluble mixtures and multimers can also be removed by conventional methods, such as molecular sieves and ion exchange.

### Composition

The present disclosure provides a composition comprising the aforementioned TRGV9-binding protein and/or GPC3/TRGV9-binding protein of the present disclosure. For example, provided is a pharmaceutical composition comprising an amount of the aforementioned TRGV9-binding protein and/or GPC3/TRGV9-binding protein that is effective in treating, alleviating, or preventing a disease, and at least one pharmaceutically acceptable excipient, diluent, or auxiliary material.

In some specific embodiments, a unit dose of the pharmaceutical composition may comprise 0.01 to 99 wt% of the TRGV9-binding protein and/or the GPC3/TRGV9-binding protein, or a unit dose of the pharmaceutical composition comprises 0.1-2000 mg of the TRGV9-binding protein and/or the GPC3/TRGV9-binding protein. In some specific embodiments, the unit dose comprises 1-1000 mg.

In some embodiments, provided is a product (e.g., a kit) comprising the aforementioned TRGV9-binding protein and/or GPC3/TRGV9-binding protein. Optionally, the product comprises a container and a label. The container is, for example, a bottle, a syringe, and a test tube. The container accommodates a pharmaceutical composition effective in treating a disorder. The label on or associated with the container indicates that the pharmaceutical composition is used for treating the disorder of choice.

In some embodiments, the aforementioned disease is a cell proliferative disease or cancer.

### Treatment Method and Pharmaceutical Use

The present disclosure provides a method for treating, alleviating, preventing, or diagnosing a disease or disorder by using the aforementioned TRGV9-binding protein, GPC3/TRGV9-binding protein, coding polynucleotide therefor, or composition thereof (including the pharmaceutical composition).

In some embodiments, provided is a method for ameliorating, alleviating, treating, or preventing a disease, comprising administering to a subject an amount of the following that is effective in ameliorating, alleviating, treating, or preventing the disease:
1) the aforementioned TRGV9-binding protein or coding polynucleotide therefor or pharmaceutical composition thereof of the present disclosure; or
2) the aforementioned GPC3/TRGV9-binding protein or coding polynucleotide therefor or pharmaceutical composition thereof of the present disclosure.

In some embodiments, provided is use of the aforementioned items 1) to 2) for the manufacture of a medicament for ameliorating, alleviating, treating, or preventing a disease.

In some embodiments, provided is a method for treating a disease by using the TRGV9-binding protein of the present disclosure in combination with γδ T cells. In some embodiments, provided is a method for treating a disease by using the GPC3/TRGV9-binding protein of the present disclosure in combination with γδ T cells. In some embodiments, the γδ T cells are autologous or allogeneic.

In some embodiments, any of the aforementioned TRGV9-binding proteins of the present disclosure is used for treating a disease, including use in combination with γδ T cells. In some embodiments, the γδ T cells are used for treating a disease, including use in combination with any of the aforementioned TRGV9-binding proteins of the present disclosure.

In some embodiments, any of the aforementioned GPC3/TRGV9-binding proteins of the present disclosure is used for treating a disease, including use in combination with γδ T cells. In some embodiments, the γδ T cells are used for treating a disease, including use in combination with any of the aforementioned GPC3/TRGV9-binding proteins of the present disclosure.

In some embodiments, provided is a method for treating a disease, comprising administering to a subject in need thereof a therapeutically effective amount of any of the aforementioned TRGV9-binding proteins of the present disclosure and γδ T cells. In some embodiments, provided is a method for treating a disease, comprising administering to a subject in need thereof a therapeutically effective amount of any of the aforementioned GPC3/TRGV9-binding proteins of the present disclosure and γδ T cells.

In some embodiments, the aforementioned disease is a disease or disorder caused by GPC3 overexpression.

In some embodiments, the aforementioned disease is a cell proliferative disease or cancer.

In some specific embodiments, the aforementioned disease is GPC3-positive cancer.

In some embodiments, the aforementioned disease is liver cancer.

In some specific embodiments, the aforementioned disease is GPC3-positive liver cancer.

### Detection

The present disclosure provides use of the TRGV9-binding protein, the GPC3/TRGV9-binding protein, the coding polynucleotide therefor, or the composition for detection. The present disclosure further provides a method, system, or device for detecting GPC3 or TRGV9 *in vivo* or *in vitro*, comprising treating a sample with the aforementioned binding protein, polynucleotide, or composition of the present disclosure.

In some embodiments, further provided is a kit comprising the aforementioned TRGV9-binding protein, GPC3/TRGV9-binding protein, coding polynucleotide therefor, or composition thereof, wherein the kit may further comprise instructions for diagnostic use. The kit may further comprise at least one additional reagent, such as a label or an additional diagnostic agent. For *in vivo* use, the TRGV9-binding protein, the GPC3/TRGV9-binding protein, and the coding polynucleotide therefor may be formulated as pharmaceutical compositions.

### BRIEF DESCRIPTION OF THE DRAWINGS

FIG. 1 shows schematic diagrams of the structures of anti-GPC3/γδTCR bispecific antibodies.
FIGs. 2A to 2D show the FACS binding activity of anti-GPC3/γδTCR bispecific antibodies to GPC3-positive cells and human γ9δ2 T cells, wherein FIG. 2A shows the binding of anti-GPC3/γδTCR bispecific antibodies on HepG2 cells, FIG. 2B shows the binding of anti-GPC3/γδTCR bispecific antibodies on DLD-1 cells, FIG. 2C shows the binding of anti-GPC3/γδTCR bispecific antibodies on human γδ T cells, and FIG. 2D shows the binding of anti-GPC3/γδTCR bispecific antibodies on PBMCs.
FIGs. 3A to 3B show the effects of anti-γδTCR antibodies on BTN2A/BTN3A-TCR natural signals. FIG. 3A shows the effect of SDP01378 on BTN2A/BTN3A-TCR natural signals, and FIG. 3B shows the effect of SDP01315 on BTN2A/BTN3A-TCR natural signals.
FIGs. 4A to 4D show the killing activity of anti-γδTCR antibodies against tumor cells with different GPC3 expression levels, wherein FIG. 4A shows the killing activity against HepG2 cells, FIG. 4B shows the killing activity against Huh-7 cells, FIG. 4C shows the killing activity against MKN-45 cells, and FIG. 4D shows the killing activity against DLD-1 cells.
FIGs. 5A to 5D show the killing activity of γδ T cells derived from different donors against HepG2, wherein the γδ T cells used in FIGs. 5A-5D were derived from induced expansion of donors #SC12004, #SC12392, #XC11053, and #XC11061, respectively.
FIGs. 6A to 6C show the killing of tumor cells with low antigen expression by anti-GPC3/γδTCR bispecific antibodies at different effector-to-target ratios, wherein the effector-to-target ratio in FIG. 6A is 1:1, the effector-to-target ratio in FIG. 6B is 10:1, and the effector-to-target ratio in FIG. 6C is 30:1.
FIGs. 7A to 7D show the killing activity after an increase in the proportion of γδ T cells in PBMCs. FIG. 7A shows killing by PBMCs of donor #XC11053, and FIG. 7B shows the killing activity after addition of 30% γδ T cells to PBMCs of donor #XC11053. FIG. 7C shows killing by PBMCs of donor #SC12392, and FIG. 7D shows the killing activity after addition of 30% γδ T cells to PBMCs of donor #SC12392.
FIGs. 8A to 8D show the release of cytokines, wherein FIGs. 8A-8B show the release of IFNγ and TNFα, respectively, during killing by donor #XC11053, and FIGs. 8C-8D show the release of IFNγ and TNFα, respectively, during killing by donor #SC12392.
FIG. 9 shows the promotion of the proliferation of γδ T cells in PBMCs by anti-GPC3/γδTCR bispecific antibodies.
FIGs. 10A to 10B show the anti-tumor activity of SDP01716 in a Huh-7 xenograft tumor model, wherein FIG. 10A shows changes in mouse tumor volume, and FIG. 10B shows changes in mouse body weight.
FIGs. 11A to 11B show the anti-tumor activity of different anti-GPC3/γδTCR bispecific antibodies in a Huh-7 xenograft tumor model, wherein FIG. 11A shows changes in mouse tumor volume, and FIG. 11B shows changes in mouse body weight.

### DETAILED DESCRIPTION

### Definitions

In order to facilitate the understanding of the present disclosure, certain technical and scientific terms are specifically defined below. Unless otherwise specifically defined herein, all other technical and scientific terms used herein have the meanings generally understood by those of ordinary skill in the art to which the present disclosure belongs. The three-letter and single-letter codes for amino acids used in the present disclosure are as described in J. biol. chem., 243, p3558 (1968).

"TRGV9" refers to a polypeptide that is capable of forming a T cell receptor when expressed on the surface of a γδ T cell. TRGV9-expressing γδ T cells are among the earliest T cells to develop in the human fetus and represent a major subset of γδ T cells in peripheral blood cells of healthy adults. "TRGV9" includes any TRGV9 variants, isotypes, and species homologs that are naturally expressed by cells, including T cells, or are capable of being expressed on cells transfected with a gene or cDNA encoding the polypeptide. In a specific embodiment, TRGV9 is human TRGV9. An exemplary human TRGV9 amino acid sequence is provided by GenBank Accession No. NG_001336.2.

"GPC3" refers to glypican 3, which is anchored to the cell membrane by phosphatidylinositol and is a marker of hepatocellular carcinoma. An exemplary human GPC3 amino acid sequence is provided by Uniprot Accession No. P51654.

"TRGV9-binding protein" encompasses any protein capable of specifically binding to TRGV9 or any molecule comprising the protein, including but not limited to antibodies targeting TRGV9 as defined in the present disclosure and antigen-binding fragments thereof or fusion proteins thereof. In some embodiments, the "TRGV9-binding protein" may comprise at least one (e.g., 1, 2, 3, 4, 5, 6, or more) single-domain antibody or VHH that specifically binds to TRGV9 in the examples of the present disclosure. In some embodiments, the "TRGV9-binding protein" may comprise at least one (e.g., 1, 2, 3, 4, 5, 6, or more) VH and VL combination that specifically binds to TRGV9 in the examples of the present disclosure. In some embodiments, the "TRGV9-binding protein" of the present disclosure may comprise, in addition to an immunoglobulin single variable domain or a VH and VL combination of TRGV9, a linker and/or a moiety with an effector function, such as a half-life extending moiety (e.g., an immunoglobulin single variable domain that binds to serum albumin) and/or a fusion partner (e.g., serum albumin) and/or a conjugated polymer (e.g., PEG) and/or an Fc region. In some embodiments, the "TRGV9-binding protein" encompasses the anti-γδTCR antibodies or antigen-binding fragments thereof in the examples of the present disclosure.

"GPC3/TRGV9-binding protein" encompasses any protein capable of specifically binding to GPC3 and TRGV9 or any molecule comprising the protein, including but not limited to antibodies, polypeptides, fusion proteins of antibodies and polypeptides, or conjugates thereof. In some embodiments, the "GPC3/TRGV9-binding protein" encompasses the anti-GPC3/γδTCR bispecific antibodies in the examples of the present disclosure.

"Antibody" encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties), so long as they exhibit the desired antigen-binding activity. An antibody may refer to an immunoglobulin, which is a four-peptide-chain structure formed by linking two heavy chains and two light chains by interchain disulfide bonds. The heavy chain constant regions of immunoglobulins differ in amino acid composition and arrangement; therefore, they also differ in antigenicity. Accordingly, immunoglobulins can be divided into five classes, or isotypes of immunoglobulins, namely IgM, IgD, IgG, IgA, and IgE, and their corresponding heavy chains are µ chains, δ chains, γ chains, α chains, and ε chains, respectively. Igs of the same class can be divided into different subclasses based on the amino acid composition of their hinge regions and the number and positions of heavy chain disulfide bonds; for example, IgGs can be divided into IgG1, IgG2, IgG3, and IgG4. Light chains are divided into κ or λ chains based on their constant regions. Each of the five Ig classes may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable region comprises 3 hypervariable regions (HVRs) and 4 framework regions (FRs) with relatively conservative sequences. The 3 hypervariable regions determine the specificity of the antibody and thus are also known as complementarity-determining regions (CDRs).

Each light chain variable region (VL) or heavy chain variable region (VH) consists of 3 CDRs and 4 FRs arranged from the amino-terminus to the carboxyl-terminus in the following order: FR1, CDR1, FR2, CDR2, FR3, CDR3, and FR4. The 3 CDRs of the light chain refer to LCDR1, LCDR2, and LCDR3, and the 3 CDRs of the heavy chain refer to HCDR1, HCDR2, and HCDR3.

The antibodies of the present disclosure may be polyclonal, monoclonal, xenogeneic, allogeneic, syngeneic, or modified forms thereof, with the monoclonal antibodies being especially applicable in various examples. Generally, the antibodies of the present disclosure are recombinant antibodies. As used herein, "recombinant" generally refers to such products as a cell, a nucleic acid, a protein, or a vector, and indicates that the cell, the nucleic acid, the protein, or the vector has been modified by introduction of a heterologous nucleic acid or protein or alteration of a natural nucleic acid or protein, or that the cell is derived from a cell modified in this way. For example, recombinant cells express genes that are not found within the native (non-recombinant) cellular form or express native genes that are originally abnormally expressed, expressed at low levels, or not expressed at all.

For the determination or definition of CDRs, the deterministic depiction of CDRs and identification of residues comprising binding sites of an antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition. The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the location of certain structural loop regions (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues does not significantly affect the antigen binding. As used in the present disclosure, a CDR may refer to a CDR defined by any method known in the art, including combinations of methods. The correspondence between the various numbering schemes is well known to those skilled in the art.

A skilled person understands that although a particular numbering scheme (such as Kabat) is employed in a specific example or in a specific SEQ ID NO, the skilled person can determine the sequences to which the sequence corresponds under other numbering schemes, and such sequences are still considered to fall within the scope of the present disclosure.

A "domain" of a polypeptide or protein refers to a folded protein structure that is capable of maintaining its tertiary structure independently of the rest of the protein. In general, a domain is responsible for a single functional property of a protein and, in many cases, may be added, deleted, or transferred to other proteins without loss of functions of the rest of the protein and/or the domain of the protein.

"Immunoglobulin domain" refers to a globular region of an antibody chain (e.g., a chain of a conventional antibody with a four-peptide-chain structure or of a heavy-chain antibody) or a polypeptide essentially consisting of such globular regions. Immunoglobulin domains are characterized by their maintenance of the immunoglobulin folding feature of the antibody molecule.

"Immunoglobulin variable domain" substantially consists of four "framework regions" referred to in the art and hereinafter as "framework region 1" or "FR1", "framework region 2" or "FR2", "framework region 3" or "FR3", and "framework region 4" or "FR4", and three "complementarity determining regions" or "CDRs" referred to in the art and hereinafter as "complementarity determining region 1" or "CDR1", "complementarity determining region 2" or "CDR2", and "complementarity determining region 3" or "CDR3". The general structure or sequence of immunoglobulin variable domains can be expressed as: FR1-CDR1-FR2-CDR2-FR3-CDR3-FR4. An immunoglobulin variable domain possesses specificity for an antigen by virtue of having an antigen-binding site. "Antibody framework (FR)" refers to a portion of a variable domain that is used as a scaffold for the antigen-binding loops (CDRs).

"Immunoglobulin single variable domain" is generally used to refer to an immunoglobulin variable domain (which may be a heavy or light chain domain, including a VH, VHH, or VL domain) that can form a functional antigen-binding site without interacting with other variable domains (e.g., without VH/VL interactions as are required between the VH and VL domains of conventional four-chain monoclonal antibodies). Examples of "immunoglobulin single variable domain" include nanobodies (including VHHs, humanized VHHs, and/or camelized VHs, e.g., camelized human VHs), IgNARs, domains, (single-domain) antibodies as VH domains or derived from VH domains (such as dAbs^{™}), and (single-domain) antibodies as VL domains or derived from VL domains (such as dAbs^{™}). Immunoglobulin single variable domains based on and/or derived from heavy chain variable domains (such as VH or VHH domains) are generally preferred. A specific example of an immunoglobulin single variable domain is a "VHH domain" (or simply "VHH") as defined below.

"VHH", also known as a heavy-chain single-domain antibody, VHH, V_{H}H domain, VHH antibody fragment, VHH antibody, or nanobody, is a variable domain of an antigen-binding immunoglobulin known as a "heavy-chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). "VHH" is used to distinguish the variable domain from the heavy chain variable domain (which is referred to in the present disclosure as a "VH domain" or VH) and the light chain variable domain (which is referred to in the present disclosure as a "VL domain" or VL) present in conventional antibodies with a four-peptide-chain structure. A VHH domain specifically binds to an epitope without the need for an additional antigen-binding domain; such binding behavior is different from that of the VH or VL domain in a conventional antibody with a four-peptide-chain structure, in which case the VL domain and the VH domain recognize the epitope together. A VHH domain is a small, stable, and highly efficient antigen recognition unit formed by a single immunoglobulin domain. The terms "heavy-chain single-domain antibody", "VHH domain", "VHH", "V_{H}H domain", "VHH antibody fragment", "VHH antibody", "Nanobody^{®}", and "Nanobody^{®} domain" ("Nanobody" is a trademark of Ablynx N.V., Ghent, Belgium) are used interchangeably. VHHs include, but are not limited to, natural antibodies produced by camelids, antibodies produced by camelids and then humanized, or antibodies obtained by screening using phage display techniques. The total number of amino acid residues in a VHH is generally in the range of 110 to 120, typically between 112 and 115. However, it should be noted that shorter and longer sequences may also be suitable for the purposes described in the present disclosure. Methods for obtaining VHHs that bind to a particular antigen or epitope have been previously disclosed in the following documents: R. van der Linden et al., Journal of Immunological Methods, 240(2000) 185-195; Li et al., J Biol Chem., 287(2012) 13713-13721; Deffar et al., African Journal of Biotechnology Vol. 8(12), pp. 2645-2652, 17 June, 2009; and WO94/04678.

As is well known in the art for VH domains and VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, in general, the numbering according to Kabat may or may not correspond to the actual numbering of the amino acid residues in the actual sequence. Other numbering systems or numbering schemes include Chothia, IMGT, and AbM.

"Humanized antibody", also known as a CDR-grafted antibody, refers to an antibody produced by grafting non-human CDR sequences into the framework of variable regions of a human antibody. Humanized antibodies can overcome the issue of strong immune responses induced by chimeric antibodies, which arise due to their significant non-human protein content. To avoid a decrease in activity caused by a decrease in immunogenicity, the variable regions of a fully human antibody can be subjected to minimum reverse mutation to maintain the activity. Examples of "humanization" include "humanization" of VHH domains derived from Camelidae by replacing one or more amino acid residues in the amino acid sequence of the original VHH sequence with one or more amino acid residues present at the corresponding positions in a VH domain of a human conventional antibody with a four-peptide-chain structure (also referred to in the present disclosure as "sequence optimization"; in addition to humanization, "sequence optimization" may also encompass other modifications to the sequence by one or more mutations providing improved properties of the VHH, such as removal of potential post-translational modification sites). A humanized VHH domain may comprise one or more fully human framework region sequences, and in some specific embodiments, may comprise the human framework region sequence of IGHV3. Methods for humanization include, for example, protein surface amino acid humanization (resurfacing) and universal framework grafting method for antibody humanization (CDR grafting to a universal framework), i.e., "grafting" CDRs onto other "scaffolds" (including but not limited to human scaffolds or non-immunoglobulin scaffolds). Scaffolds and techniques suitable for such CDR grafting are known in the art. For example, germline DNA sequences of genes of the human heavy and light chain variable regions can be found in the VBase human species sequence database, as well as in Kabat, E. A. et al., 1991 Sequences of Proteins of Immunological Interest, 5th Edition. The humanized antibodies of the present disclosure also include humanized antibodies that are further subjected to CDR affinity maturation by phage display. In addition, in order to avoid a decrease in activity caused by a decrease in immunogenicity, the framework sequence in the human antibody variable region can be subjected to minimum reverse mutation or back mutation to maintain activity.

An "affinity-matured" antibody refers to an antibody that has one or more changes in one or more hypervariable regions (HVRs) that result in an improvement in the affinity of the antibody for the antigen, compared to the parental antibody that does not have such changes. For example, an "affinity-matured" TRGV9-binding protein or anti-TRGV9 antibody comprises one or more changes in one or more CDRs that result in an increase in the affinity for the antigen compared to its parental antibody. Affinity-matured antibodies can be prepared, for example, by methods known in the art as described below: Marks et al., 1992, Biotechnology 10:779-783 or Barbas et al., 1994, Proc. Nat. Acad. Sci, USA 91:3809-3813; Shier et al., 1995, Gene 169:147-155; Yelton et al., 1995, Immunol. 155:1994-2004; Jackson et al., 1995, J. Immunol. 154(7):3310-9; Hawkins et al., 1992, J. MoI. Biol. 226(3):889896; KS Johnson and RE Hawkins, "Affinity maturation of antibodies using phage display", Oxford University Press 1996.

Typically, the GPC3/TRGV9-binding protein or TRGV9-binding protein of the present disclosure may bind to the antigen or target protein to be bound (i.e., GPC3 or TRGV9) with a dissociation constant (K_{D}) of preferably 10⁻⁷ to 10⁻¹⁰ mol/L (M), more preferably 10⁻⁸ to 10⁻¹⁰ mol/L, and even more preferably 10⁻⁹ to 10⁻¹⁰ or less, and/or with an association constant (KA) of at least 10⁻⁷ M, preferably at least 10⁻⁸ M, more preferably at least 10⁻⁹ M, and more preferably at least 10⁻¹⁰ M, as measured in a Biacore, KinExA, or Fortibio assay. Any K_{D} value greater than 10⁻⁴ M is generally considered to indicate non-specific binding. Specific binding of an antigen-binding protein to an antigen or epitope can be measured in any suitable manner known, including, for example, surface plasmon resonance (SPR) assay, Scatchard assay, and/or competitive binding assay (e.g., radioimmunoassay (RIA), enzyme immunoassay (EIA), and sandwich competitive assay) described in the present disclosure.

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation constant (K_{D}). K_{D} can be determined by measuring the kinetics of complex formation and dissociation by using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods, and calculations can even be performed for complex mixtures by, for example, those methods described in Caceci et al (1984, Byte 9:340-362). For example, K_{D} can be determined by using a dual filtration nitrocellulose filter binding assay such as that disclosed by Wong & Lohman (1993, Proc. Natl. Acad. Sci. USA 90:5428-5432). Other standard assays for assessing the binding ability of an antibody to a target antigen are known in the art and include, for example, ELISA, western blot, RIA, and flow cytometry, as well as other assays exemplified elsewhere in the present disclosure. The binding kinetics and binding affinity of the antibody can also be evaluated by standard assays known in the art, such as surface plasmon resonance (SPR), for example, by using the Biacore^{™} system or KinExA. The binding affinities associated with different molecular interactions, e.g., the binding affinities of different antibodies for a given antigen, can be compared by comparing the K_{D} values of antibody/antigen complexes. Similarly, the specificity of an interaction can be evaluated by determining and comparing the K_{D} value for the interaction of interest (e.g., a specific interaction between an antibody and an antigen) with the K_{D} value for an interaction not of interest (e.g., a control antibody known not to bind to IGF-1R or TRGV9).

"Conservative replacement" refers to replacement by another amino acid residue having similar properties to the original amino acid residue. For example, lysine, arginine, and histidine have similar properties in that they have basic side chains, and aspartic acid and glutamic acid have similar properties in that they have acidic side chains. In addition, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity", or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The percent homology between two sequences is a function of the number of matching or homologous positions shared by the two sequences divided by the number of compared positions × 100%. For example, if 6 out of 10 positions are matched or homologous when two sequences are optimally aligned, the two sequences are 60% homologous. In general, a comparison is performed when two sequences are aligned to obtain the maximum percent homology.

"Titin-T chain" or "T chain" refers to a peptide fragment of 78-118 amino acids comprising a Titin Ig-like 152 domain in a Titin protein or a functional variant thereof, and the Titin-T chain is capable of binding to the Obscurin Ig-like 1 domain to form a dimeric complex. The functional variant of the T chain is obtained by mutating some amino acids of a wild-type T chain, but still has a polypeptide that binds to the Obscurin Ig-like 1 domain to form a dimeric complex. For example, a suitable number of amino acids are added to or removed from the C-terminus and/or the N-terminus of the Titin Ig-like 152 domain; 1, 2, 3, 4, 5, 6, 7, 8, 9, or 10 amino acid residues may be added or removed; for example, the 5 amino acids "KAGIR" in 5 wild-type Titin proteins which are immediately adjacent to the N-terminus of the Titin Ig-like 152 domain are added to the N-terminus of the Titin Ig-like 152 domain, and it still has the function of binding to the Obscurin Ig-like 1 domain to form a complex. Amino acids of the Titin Ig-like 152 domain may also be subjected to other mutations; for example, certain amino acids may be mutated to improve interchain disulfide bonds, enhance complex stability, etc.

"Obscurin-O chain" or "O chain" refers to a peptide fragment of 87-117 amino acids comprising an Obscurin Ig-like 1 domain in an Obscurin protein or a functional variant thereof, and the Obscurin-O chain is capable of binding to the Titin Ig-like 152 domain to form a dimeric complex. The functional variant of the Obscurin-O chain is obtained by mutating some amino acids of a wild-type O chain, but still has a polypeptide that binds to the Titin Ig-like 152 domain to form a dimeric complex. For example, a suitable number of amino acids are added to or removed from the C-terminus and/or the N-terminus of the Obscurin-O domain, for example, 1, 2, 3, 4, 5, 6, 7, 8, 9, or 10 amino acids are added or removed; for example, the 5 amino acids "DQPQF" in 5 wild-type Obscurin proteins which are immediately adjacent to the N-terminus of the Obscurin Ig-like 1 domain are added to the N-terminus of the Obscurin-O domain, and it still has the function of binding to the Titin Ig-like 152 domain to form a dimeric complex. Some amino acids of the Obscurin Ig-like 1 domain may also be subjected to other mutations; for example, certain amino acids may be mutated to improve interchain disulfide bonds, enhance antibody stability, etc.

"Nucleic acid" and "polynucleotide" are used interchangeably in the present disclosure to refer to any DNA or RNA molecule, either single- or double-stranded, and, if single-stranded, the molecule of its complementary sequence, preferably a double-stranded DNA. A nucleic acid is "operably linked" when it is placed into a functional relationship with another nucleic acid sequence. For example, a promoter or enhancer is operably linked to a coding sequence if it affects the transcription of the coding sequence.

"Host cell" includes individual cells or cell cultures, which may be or have been the recipient of a vector for incorporation of a polynucleotide insert. The host cell includes the progeny of a single host cell, and the progeny may not necessarily be identical (in morphology or genomic DNA complement) to the original parental cell due to natural, accidental, or intentional mutations. The host cell includes cells transfected and/or transformed *in vivo* with the polynucleotide of the present disclosure. "Cell", "cell line", and "cell culture" are used interchangeably, and all such designations include their progeny. It should also be understood that all progeny may not be precisely identical in DNA content due to intentional or unintentional mutations. Mutant progeny that have the same function or biological activity as those selected in the initially transformed cells are included.

"Inhibition" and "blocking" are used interchangeably and encompass both partial and complete inhibition/blocking. "Inhibition of growth" (e.g., involving cells) is intended to include any measurable reduction in cell growth.

"Giving", "administering", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids, e.g., therapeutic, pharmacokinetic, diagnostic, research, and experimental methods. Treatment of cells comprises contact between a reagent and cells, and contact between the reagent and a fluid, wherein the fluid is in contact with the cells. "Giving", "administering", and "treating" also mean treating, for example, a cell by a reagent, diagnosis, a binding composition or by another cell *in vitro* and *ex vivo*. When applied to humans, veterinary medicine, or research subjects, they refer to therapeutic treatment, preventive or prophylactic measures, and research and diagnostic applications.

"Treating" or "treatment" refers to administering a therapeutic agent, such as a therapeutic agent comprising any one of the binding proteins of the present disclosure or a pharmaceutical composition thereof, either internally or externally, to a subject who has had, is suspected of having, or is predisposed to having one or more proliferative diseases or symptoms thereof on which the therapeutic agent is known to have a therapeutic effect. Typically, the therapeutic agent is administered in an amount effective in alleviating one or more disease symptoms in the subject or population being treated, whether by inducing regression of such symptoms or inhibiting the progression of such symptoms to any clinically measurable degree. The amount of therapeutic agent effective in alleviating any specific disease symptom (also referred to as the "therapeutically effective amount") may vary depending on factors such as the disease state, age, and body weight of the subject, and the ability of the drug to produce a desired therapeutic effect in the subject. Whether a disease symptom has been palliated can be evaluated by any clinical test methods commonly used by doctors or other health care professionals to evaluate the severity or progression of the symptom. Although embodiments of the present disclosure (e.g., treatment methods or articles of manufacture) may be ineffective in alleviating a disease symptom of interest in a certain subject, they shall palliate the disease symptom of interest in a statistically significant number of subjects as determined by any statistical testing method known in the art, such as a Student's t-test, a Chi-square test, a U-test by Mann and Whitney, a Kruskal-Wallis test (H-test), a Jonckheere-Terpstra test, and a Wilcoxon test.

"Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method, route, and dose of administration, and the severity of side effects. An effective amount may be the maximum dose or administration regimen to avoid significant side effects or toxic effects. A subject of the present disclosure may be an animal or a human subject. "Optional" or "optionally" means that the event or circumstance subsequently described may, but does not necessarily, occur. This description includes the instance where the event or circumstance occurs or does not occur. "And/or" should be taken as a specific disclosure of each of the two specified features or components, with or without the other. Thus, the term "and/or" as used in a phrase such as "A and/or B" in the present disclosure includes "A and B", "A or B", "A" (alone), and "B" (alone). Unless the context clearly requires otherwise, throughout the description and the claims, the words "comprise", "have", "include", etc., are to be understood in an inclusive sense as opposed to an exclusive or exhaustive sense, that is to say, in the sense of "including but not limited to". In the context of the mutations contained in the Fc region of the present disclosure, "/" represents "and"; for example, "354C/366W" represents "354C and 366W"; that is, the Fc comprises mutations 354C and 366W; the amino acid positions of the mutations in the Fc region of the present disclosure are numbered according to the EU numbering scheme. The "subject" or "patient" in the present disclosure means a mammal, particularly a primate, and especially a human.

### Examples

The present disclosure is further described with reference to the following examples; however, these examples are not intended to limit the scope of the present disclosure.

Experimental procedures without specific conditions indicated in the examples or test examples of the present disclosure are generally conducted under conventional conditions, or under conditions recommended by the manufacturers of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific sources indicated were commercially available conventional reagents.

### Example 1. Design and Preparation of Antigens

The TCR on the surface of a γδ T cell (γδ T cell receptor) is a heterodimeric membrane protein formed by the complexation of a γ chain and a δ chain. The amino acids at positions 1-242 of the human γδTCR extracellular region protein γ9 chain (Protein data bank, No. 1HXM, chain B) were selected, and a 3C enzyme cutting site, a leucine zipper, and a FLAG tag were sequentially added to the C-terminus; the amino acids at positions 1-229 of the human γδTCR extracellular region protein δ2 chain (Protein data bank, No. 1HXM, chain A) were selected, and a 3C enzyme cutting site, a leucine zipper, and a His8 tag were sequentially added to the C-terminus. In addition, mutations Q180C and V168C were introduced into the constant regions of the γ chain and the δ chain, respectively, to form an interchain disulfide bond.

A recombinant monkey (Macaca mulatta) γ9δ2 TCR protein: the amino acids at positions 1-241 of the monkey γ9 chain (see SEQ ID NO: 42 in Published Patent No. US2019144540A1 for the sequence) were selected, and a 3C enzyme cutting site, a leucine zipper, and a FLAG tag were sequentially added to the C-terminus; the amino acids at positions 1-229 of the monkey δ2 chain (see SEQ ID NO: 37 in Published Patent No. US20190144540A1 for the sequence) were selected, and a 3C enzyme cutting site, a leucine zipper, and a His8 tag were sequentially added to the C-terminus; in addition, mutations Q179C and V168C were introduced into the constant regions of the γ chain and the δ chain, respectively, to form an interchain disulfide bond.
> Recombinant human γ9 chain 
AGHLEQPQISSTKTLSKTARLECVVSGITISATSVYWYRERPGEVIQFLVSISYDG
TVRKESGIPSGKFEVDRIPETSTSTLTIHNVEKQDIATYYCALWEAQQELGKKIK
VFGPGTKLIITDKQLDADVSPKPTIFLPSIAETKLQKAGTYLCLLEKFFPDVIKIH
WEEKKSNTILGSCEGNTMKTNDTYMKFSWLTVPEKSLDKEHRCIVRHENNKNG
VDQEIIFPPIKTDVITMDPKDNGSGLEVLFQGPGSGAQLEKELQALEKENAQLE
WELQALEKELAQKGSGDYKDDDDK (SEQ ID NO: 1)
> Recombinant human δ2 chain 
AIELVPEHQTVPVSIGVPATLRCSMKGEAIGNYYINWYRKTQGNTMTFIYREKDI
YGPGFKDNFQGDIDIAKNLAVLKILAPSERDEGSYYCACDTLGMGGEYTDKLIF
GKGTRVTVEPRSQPHTKPSVFVMKNGTNVACLVKEFYPKDIRINLVSSKKITEFD
PAICISPSGKYNAVKLGKYEDSNSVTCSVQHDNKTVHSTDFEVKTDSTDHVKPK
ETENTKQPSKSGSGLEVLFQGPGSGAQLKKKLQALKKKNAQLKWKLQALKKK
LAQKGSGHHHHHHHH (SEQ ID NO: 2)
> Recombinant monkey γ9 chain 
AGHLEQPQISSTKMLSKTARLECVVSGVTISETSIYWYRERPGEVIQFLVCIFYDG
TVKKESSIPSGKFEVDRIPKTSTSTLTIHNVEKQDIATYYCALWEVQQFGRKVKL
 
FGPGTKLIITDKHLDADVSPKPTIFLPSIAETNLHKAGTYLCLLENFFPDVIKIHW
QEKKSNTILGSCEGNTVKTNDTYMKFSWLTVPEKSLDKEHRCIVRHENNKNGV
DQEIIFPPIKTDVTTMDPKDNGSGLEVLFQGPGSGAQLEKELQALEKENAQLEW
ELQALEKELAQKGSGDYKDDDDK (SEQ ID NO: 3)
> Recombinant monkey δ2 chain

AVELVPEHQTVIVSVGDPATLKCSMKGEAISNYYINWYRKTQGNTMTFIYREKG
IYGPGFKDNFQGDIDTEENQAVLKILAPSERDEGSYYCASDILSWVDSYTDKLIF
GKGTRVTVEPKRQPHTKPSVFVMKNGTNVACLVKDFYGKDIRINLESSKKITEF
DPAICVSPSGKYNAVKLGQYADSNSVTCSVQHNKEVVYSTDFEVKTNSTDHLK
PTETENTKQPSKSGSGLEVLFQGPGSGAQLKKKLQALKKKNAQLKWKLQALK
KKLAQKGSGHHHHHHHH (SEQ ID NO: 4)

Plasmids carrying genes encoding the target proteins were separately synthesized. The human γ9 chain (SEQ ID NO: 1) and human δ2 chain (SEQ ID NO: 2) plasmids were mixed in a 1:1 ratio, and the monkey γ9 chain (SEQ ID NO: 3) and monkey δ2 chain (SEQ ID NO: 4) plasmids were mixed in a 1:1 ratio. The resulting mixtures were separately transiently transfected into Expi293 cells (purchased from Thermo) and expressed for 7 days. After separation and purification, a human γ9δ2 TCR protein and a monkey γ9δ2 TCR protein were obtained. The proteins were stored at -80 °C for later use.

### Example 2. Screening for Anti-γδTCR Antibodies

### 1. Screening for anti-γδTCR single-domain antibody

*In vitro* expanded human γ9δ2 T cells were used as an immunogen to immunize adult healthy alpacas. The recombinant human γ9δ2 TCR protein in Example 1 was used to screen for a specific antibody by phage display.

Specifically, the primary immunization dose was 2E7 cells per alpaca. Three weeks after the primary immunization dose, a boost immunization dose (2E7 cells per alpaca) was administered. Subsequent boost immunization doses were administered at intervals of 3 weeks. Serum samples were collected one week after each boost immunization dose, and the antibody titer in the alpaca serum was determined by Protein ELISA and FACS.

The specific Protein ELISA was as follows: The recombinant human γ9δ2 TCR protein was diluted to 2 µg/mL with a 0.05 M carbonate buffer (pH 9.6), and a plate was coated with the solution (100 µL/well) at 4 °C overnight. The plate was blocked with a PBST buffer containing 5% skim milk for 1 h and washed 3 times. The alpaca serum was serially diluted two-fold in the blocking buffer (starting at 1:2000). After 45 min of incubation at 37 °C, the plate was washed 5 times. 100 µL of a horseradish peroxidase-labeled goat anti-alpaca secondary antibody (AlpVHHs, 053-404-005, diluted with PBS in a 1:10,000 ratio) was added to each well, and the plate was incubated at 37 °C for 45 min and washed 5 times. Finally, 100 µL of a TMB chromogenic solution was added to each well for color development. After 5 min, 50 µL of a stop solution was added to stop the reaction. The absorbance at 450 nm was measured using a microplate reader.

The specific FACS assay was as follows: Cells were collected and resuspended to 4E6 cells/mL, and 50 µL of the cell suspension was added to the serially diluted alpaca serum. The resulting mixture was incubated at 4 °C for 1 h. The cells were washed twice with a 1% BSA/PBS buffer, and the supernatant was then discarded. Anti-alpaca IgG iFluor647 (AlpVHHs, 053-404-009, diluted in a 1:200 ratio) was added, and the cells were incubated in a dark place at 4 °C for 45 min. The cells were washed twice with a 1% BSA/PBS buffer, resuspended in 200 µL of the buffer, and then assayed by FACS.

According to the assay, the serum titer of the immunized alpacas was greater than 128 k. After three immunization doses, 50 mL of peripheral blood was collected one week after each immunization dose, and lymphocytes (PBMCs) were isolated. The total RNA of the PBMCs was extracted using an RNAiso Plus reagent to establish a phage library with a titer of 3.08 × E13 cfu/mL.

Antibodies against the recombinant human γ9δ2 TCR protein were panned by phage display. Antibodies that cross-bound to the monkey γ9δ2 TCR protein were identified by ELISA. The binding activity to human γ9δ2 T cells expanded *in vitro* was determined by FACS. A positive monoclonal single-domain antibody was selected using the above ELISA and FACS methods. Its sequence is shown below.
> The variable region of SDP01346 (clone No. ac-025)

QVQLVESGGGLVQAGGSLRLSCTVSGSTFSDFAMGWLRQAPGKEREFVAAISW
TGGRTYYADSVKGRFAISRDNGENTVYLQMNSLKSEDTAIYYCAASRDCSGPG
CRVHEYDYWGQGTQVTVSS (SEQ ID NO: 5)

**Table 1. The CDR sequences of an anti-human γδTCR single-domain antibody (the Kabat numbering scheme)**

| Antibody No. | CDR sequences | | Sequence No. |
|---|---|---|---|
| SDP01346 | CDR1 | DFAMG | SEQ ID NO: 6 |
| | CDR2 | AISWTGGRTYYADSVKG | SEQ ID NO: 7 |
| | CDR3 | SRDCSGPGCRVHEYDY | SEQ ID NO: 8 |

### 2. Screening for murine-derived anti-γδTCR antibody

*In vitro* expanded human γ9δ2 T cells were used as an immunogen to immunize Balb/c and SJL mice aged 6-8 weeks. The recombinant human γ9δ2 TCR protein in Example 1 was used to screen for a specific antibody by hybridoma fusion.

Specifically, the primary immunization dose was 1E7 cells per mouse. Two weeks after the primary immunization dose, a boost immunization dose (1E7 cells per mouse) was administered. Subsequent boost immunization doses were administered at intervals of 2-3 weeks. Serum samples were collected one week after each boost immunization dose, and the antibody activity in the mouse serum was assessed by Protein ELISA and FACS. The specific Protein ELISA was as follows: A plate was coated with 1 µg/mL recombinant human γ9δ2 TCR protein at 4 °C overnight, blocked with a PBST buffer containing 1% BSA for 1 h, and washed 3 times. The mouse serum was serially diluted three-fold in the blocking buffer (starting at 1:200). After 1 h of incubation at 37 °C, the plate was washed 3 times and incubated with an anti-mouse IgG-Fc-HRP secondary antibody (Sigma, AP127P, diluted in a 1:10,000 ratio) for 1 h. The plate was washed 3 times with PBST, and 100 µL of a TMB chromogenic solution was added to each well for color development.

After 15 min, the reaction was stopped using a stop solution. The absorbance at 450 nm was measured using a microplate reader.

The specific FACS assay was as follows: Cells were plated at 1E5 cells per well. The plate was centrifuged, and the supernatant was discarded. The serially diluted mouse serum was added. The plate was incubated at 4 °C for 1 h. The cells were washed twice with a 1% BSA/PBS buffer, and the supernatant was then discarded. The Alexa Flour 488 goat anti-mouse secondary antibody (Jackson immuno research, 115-545-071, diluted in a 1:500 ratio) was added, and the cells were incubated in a dark place at 4 °C for 30 min. The cells were washed twice with a 1% BSA/PBS buffer, resuspended in 200 µL of the buffer, and then assayed by FACS. The serum titer of the immunized mice was greater than 72,900.

As the last immunization dose, 50 µg of the recombinant human γ9δ2 TCR protein was injected intraperitoneally. After 4 days, the mice were sacrificed, and their spleens were collected and ground. Spleen cells were collected and mixed with mouse myeloma cells (SP2/0), and the cell mixture was subjected to electrofusion to obtain hybridoma cells. Positive clones with OD 450 nm > 0.2 were additionally tested by FACS, and positive clones that bound to human γ9δ2 T cells with an MFI value > 5000 were subcloned. After primary screening and retesting of subclones, a murine-derived anti-γδTCR antibody was selected using the above ELISA and FACS methods. The sequences of SDP01315 (clone No. bph-003) are shown below.
> The VH of SDP01315 (clone No. bph-003) 
EVKLVESGGGLVKPGGSLKLSCAASGFTFSSYAMSWVRQTPEKRLEWVASISSG
GSTYYPDSVKGRFTISRDNARNILYLQMSSLRSEDTAMYYCARDGYPPFDYWG
QGTTLTVSS (SEQ ID NO: 9)
> The VL of SDP01315 (clone No. bph-003)

DIVMTQSQKFMSTSVGDRVSVTCKASQNVGTNVAWYQQKPGQSPKALIYSASY
RYSGVPDRFTGSGSGTDFTLTISNVQSEDLAEYFCQQYNSFPLTFGSGTKLEIK
(SEQ ID NO: 10)

**Table 2. The CDR sequences of a murine-derived anti-γδTCR antibody (the Kabat numbering scheme)**

| Antibody No. | CDR sequences | | Sequence No. |
|---|---|---|---|
| SDP01315 | HCDR1 | SYAMS | SEQ ID NO: 11 |
| | HCDR2 | SISSGGSTYYPDSVKG | SEQ ID NO: 12 |
| | HCDR3 | DGYPPFDY | SEQ ID NO: 13 |
| | LCDR1 | KASQNVGTNVA | SEQ ID NO: 14 |
| | LCDR2 | SASYRYS | SEQ ID NO: 15 |
| | LCDR3 | QQYNSFPLT | SEQ ID NO: 16 |

### Example 3. Construction of Anti-γδTCR Chimeric Antibodies and Functional Effect Verification

### 1. Expression of chimeric antibodies

Single-domain antibody: A nucleotide sequence encoding the antibody was cloned into a pTT5 vector, and ExpiCHO cells were then transfected with the vector. After 8 days, the cells were removed by centrifugation, and the cell culture solution was collected and filtered. The collected cell culture solution was purified using a nickel affinity column (HisTrap excel, GE), and the bound antibody was eluted with 300 mM imidazole, desalted, and buffer-exchanged into PBS to obtain the target antibody.
> The full-length sequence of SDP01346

QVQLVESGGGLVQAGGSLRLSCTVSGSTFSDFAMGWLRQAPGKEREFVAAISW
TGGRTYYADSVKGRFAISRDNGENTVYLQMNSLKSEDTAIYYCAASRDCSGPG
CRVHEYDYWGQGTQVTVSSGGGGSHHHHHH
(SEQ ID NO: 17)

Murine-derived antibody: Nucleotide sequences encoding the antibody were cloned into a pTT5 vector, and ExpiCHO cells were then transfected with the vector. After 8 days, the cells were removed by centrifugation, and the cell culture solution was collected and filtered. The collected cell culture solution was purified using a Protein A affinity column (MabSelect SuRe, GE), and the bound antibody was eluted with glycine. The eluate was neutralized with 1 M Tris and then desalted to obtain the target antibody.
> The full-length heavy chain of SDP01315 
EVKLVESGGGLVKPGGSLKLSCAASGFTFSSYAMSWVRQTPEKRLEWVASISSG
GSTYYPDSVKGRFTISRDNARNILYLQMSSLRSEDTAMYYCARDGYPPFDYWG
QGTTLTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHT
CPPCPAPEFEGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK
(SEQ ID NO: 18)
> The full-length light chain of SDP01315

DIVMTQSQKFMSTSVGDRVSVTCKASQNVGTNVAWYQQKPGQSPKALIYSASY
RYSGVPDRFTGSGSGTDFTLTISNVQSEDLAEYFCQQYNSFPLTFGSGTKLEIKRT
VAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
(SEQ ID NO: 19)

### 2. Binding of chimeric antibodies to γ9δ2 T cells

The process of measuring the cell-level affinity of the anti-γδTCR single-domain antibody was as follows: The serially diluted antibody molecule was incubated with 1 × 10⁵ γ9δ2 T cells at 4 °C for 1 h, and the excess antibody was washed off. The DyLight 405-AffiniPure Goat Anti-Alpaca IgG, VHH domain antibody (Jackson, Cat#128-475-232) was added, and the cells were incubated at 4 °C for 30 min. After the excess antibody was washed off, the cells were resuspended in 200 µL of a 2% FBS/PBS buffer, and the fluorescence signals on the cell surface were read using a Thermo Attune NxT flow cytometer. The results are shown in Table 3.

The process of measuring the cell-level affinity of the murine-derived anti-γδTCR antibody was as follows: The serially diluted antibody molecule was incubated with 1 × 10⁵ γ9δ2 T cells at 4 °C for 1 h, and the excess antibody was washed off. The murine-derived Alexa Flour 647 labeled anti-human Fc antibody (Jackson, Cat#209-605-098) was added, and the cells were incubated at 4 °C for 30 min. After the excess antibody was washed off, the cells were resuspended in 200 µL of a 2% FBS/PBS buffer, and the fluorescence signals on the cell surface were read using a Thermo Attune NxT flow cytometer. The results are shown in Table 3.

**Table 3. The binding ability of anti-γδTCR chimeric antibodies**

| Antibody No. | Binding to γ9δ2 T cells | |
|---|---|---|
| | EC₅₀ (nM) | Max MFI |
| SDP01346 | 0.44 | 3279 |
| SDP01315 | 0.99 | 11123 |

The results show that SDP01346 and SDP01315 exhibited good binding activity on γ9δ2 T cells.

**Example 4. Identification of Antigen Regions to Which Anti-γδTCR Antibodies Bind** In this example, the antigen regions to which anti-γδTCR antibodies bind were identified by protein-based ELISA. A recombinant human γ9δ1 TCR protein (a dimer protein of SEQ ID NO: 1 and SEQ ID NO: 20), a recombinant human γ8δ2 TCR protein (a dimer protein of SEQ ID NO: 21 and SEQ ID NO: 2), a recombinant human VγδCαβ chimeric TCR protein (a dimer protein of SEQ ID NO: 22 and SEQ ID NO: 23), and a recombinant human VαβCγδ chimeric TCR protein (a dimer protein of SEQ ID NO: 24 and SEQ ID NO: 25) were prepared using a method similar to that in Example 1.

The binding activity of the anti-γδTCR antibodies to the antigens described above was assessed by ELISA to identify the general regions to which the antibodies bind. The specific Protein ELISA was as follows: A plate was coated with 1 µg/mL recombinant TCR protein at 4 °C overnight, blocked with a PBST buffer containing 1% BSA for 1 h, and washed 3 times. The antibodies were diluted to 30 nM with a blocking buffer. After 1 h of incubation at 37 °C, the plate was washed 3 times and incubated with an anti-mouse IgG-Fc-HRP secondary antibody (Sigma, AP127P, diluted in a 1:10,000 ratio) for 1 h. The plate was washed 3 times with PBST, and 100 µL of a TMB chromogenic solution was added to each well for color development. After 15 min, the reaction was stopped using a stop solution. The absorbance at 450 nm was measured using a microplate reader.
> Recombinant human δ1 chain 
AQKVTQAQSSVSMPVRKAVTLNCLYETSWWSYYIFWYKQLPSKEMIFLIRQGS
DEQNAKSGRYSVNFKKAAKSVALTISALQLEDSAKYFCALGVRAFLRDWGIRV
LIFGKGTRVTVEPRSQPHTKPSVFVMKNGTNVACLVKEFYPKDIRINLVSSKKITE
FDPAICISPSGKYNAVKLGKYEDSNSVTCSVQHDNKTVHSTDFEVKTDSTDHVK
PKETENTKQPSKSGSGLEVLFQGPGSGAQLKKKLQALKKKNAQLKWKLQALK
KKLAQKGSGHHHHHHHH (SEQ ID NO: 20)
> Recombinant human γ8 chain 
ADLSSNLEGRTKSVTRPTGSSAVITCDLPVENAVYTHWYLHQEGKAPQRLLYYD
SYNSRVVLESGISREKYHTYASTGKSLKFILENLIERDSGVYYCATWASSDWIKT
FAKGTRLIVTSPDKQLDADVSPKPTIFLPSIAETKLQKAGTYLCLLEKFFPDIIKIH
WQEKKSNTILGSCEGNTMKTNDTYMKFSWLTVPEESLDKEHRCIVRHENNKNG
IDQEIIFPPIKTDVTTVDPKYNYSKDANDVITMDPKDNGSGLEVLFQGPGSGAQL
EKELQALEKENAQLEWELQALEKELAQKGSGDYKDDDDK (SEQ ID NO: 21)
> Recombinant human Vγ9Cβ chain 
AGHLEQPQISSTKTLSKTARLECVVSGITISATSVYWYRERPGEVIQFLVSISYDG
TVRKESGIPSGKFEVDRIPETSTSTLTIHNVEKQDIATYYCALWEVHELGKKIKVF
GPGTKLIITEDLKNVFPPEVAVFEPSEAEISHTQKATLVCLATGFYPDHVELSWW
VNGKEVHSGVCTDPQPLKEQPALNDSRYSLSSRLRVSATFWQNPRNHFRCQVQ
FYGLSENDEWTQDRAKPVTQIVSAEAWGRADSGSGLEVLFQGPGSGAQLEKEL
QALEKENAQLEWELQALEKELAQKGSGDYKDDDDK (SEQ ID NO: 22)
> Recombinant human Vδ2Cα chain 
AIELVPEHQTVPVSIGVPATLRCSMKGEAIGNYYINWYRKTQGNTMTFIYREKDI
YGPGFKDNFQGDIDIAKNLAVLKILAPSERDEGSYYCACDPVQVTGGYKVDKLI
FGKGTRVTVEPNIQNPDPAVYQLRDSKSSDKSVCLFTDFDSQTNVSQSKDSDVYI
TDKCVLDMRSMDFKSNSAVAWSNKSDFACANAFNNSIIPEDTFFPSPESSSGSGL
EVLFQGPGSGAQLKKKLQALKKKNAQLKWKLQALKKKLAQKGSGHHHHHH
H (SEQ ID NO: 23)
> Recombinant human Vβ7Cγ chain 
GVSQTPSNKVTEKGKYVELRCDPISGHTALYWYRQSLGQGPEFLIYFQGTGAAD
DSGLPNDRFFAVRPEGSVSTLKIQRTERGDSAVYLCATSALGDTQYFGPGTRLTV
LDKQLDADVSPKPTIFLPSIAETKLQKAGTYLCLLEKFFPDVIKIHWEEKKSNTIL
GSCEGNTMKTNDTYMKFSWLTVPEKSLDKEHRCIVRHENNKNGVDQEIIFPPIK
TDVITMDPKDNGSGLEVLFQGPGSGAQLEKELQALEKENAQLEWELQALEKEL
AQKGSGDYKDDDDK (SEQ ID NO: 24)
> Recombinant human Vα13Cδ chain

ENVEQHPSTLSVQEGDSAVIKCTYSDSASNYFPWYKQELGKRPQLIIDIRSNVGE
KKDQRIAVTLNKTAKHFSLHITETQPEDSAVYFCAASSFGNEKLTFGTGTRLTIIPS
QPHTKPSVFVMKNGTNVACLVKEFYPKDIRINLVSSKKITEFDPAICISPSGKYNA
VKLGKYEDSNSVTCSVQHDNKTVHSTDFEVKTDSTDHVKPKETENTKQPSKSG
SGLEVLFQGPGSGAQLKKKLQALKKKNAQLKWKLQALKKKLAQKGSGHHHH
HHHH (SEQ ID NO: 25)

The ELISA results are shown in Table 4. The binding absorbance values of antibodies SDP01346 and SDP01315 to different antigens were different. Both antibodies bound to the γ9δ1 TCR protein and not to the γ8δ2 TCR protein, indicating that the antibodies bound to the γ9 chain. The two antibodies bound to the VγδCαβ chimeric TCR protein and not to the VαβCγδ chimeric TCR, indicating that the antibodies bound to the TCR variable region (V region). In summary, it was clear that antibodies SDP01346 and SDP01315 bound to TRGV9.

**Table 4. The results of the identification of antigen regions to which chimeric antibodies bind**

| Antibody No. | Human γ9δ2 TCR | Monkey γ9δ2 TCR | Human γ9δ1 TCR | Human γ8δ2 TCR | VγδCαβ chimeric TCR | VαβCγδ chimeric TCR |
|---|---|---|---|---|---|---|
| SDP01346 | 3.52 | 0.769 | 2.444 | 0.108 | 2.345 | 0.094 |
| SDP01315 | 3.71 | 1.646 | 3.057 | 0.192 | 3.045 | 0.176 |

### Example 5. Humanization Engineering of Anti-γδTCR Antibodies

The variable region sequences were compared with an antibody germline database to obtain human germline templates with high homology. The human germline heavy chain template used for SDP01346 was IGHV3-30. The human germline heavy chain template used for SDP01315 was IGHV3-21, and the human germline light chain template was IGKV1-12. After the structures of monoclonal antibodies were predicted by homology modeling, the VH and VL of the murine-derived antibody or the CDRs of the single-domain antibody were grafted onto a suitable human GermLine framework (Bioinformation. 2014; 10(4):180-186; Methods Mol Biol. 2019;1904:213-230), and back mutations were subsequently introduced.

The humanized molecule sequences obtained are shown below:
> SDP01346 VH1 
EVQLVESGGGVVQPGGSLRLSCAASGSTFSDFAMGWLRQAPGKGREFVAAISW
TGGRTYYADSVKGRFTISRDNSKNTVYLQMNSLRAEDTAVYYCAASRDCSGPG
CRVHEYDYWGQGTLVTVSS (SEQ ID NO: 26)
> SDP01346 VH2 
EVQLVESGGGVVQPGGSLRLSCAVSGSTFSDFAMGWLRQAPGKEREFVAAISW
TGGRTYYADSVKGRFTISRDNSKNTVYLQMNSLRAEDTAVYYCAASRDCSGPG
CRVHEYDYWGQGTLVTVSS (SEQ ID NO: 27)
> SDP01346 VH3 
EVQLVESGGGVVQPGGSLRLSCAVSGSTFSDFAMGWLRQAPGKEREFVAAISW
TGGRTYYADSVKGRFTISRDNGKNTVYLQMNSLKSEDTAVYYCAASRDCSGPG
CRVHEYDYWGQGTLVTVSS (SEQ ID NO: 28)
> SDP01346 VH4 
EVQLVESGGGLVQPGGSLRLSCSVSGSTFSDFAMGWLRQAPGKEREFVAAISWT
GGRTYYADSVKGRFTISRDNSKNTVYLQMNSLRAEDTAVYYCAASRDCSGPGC
RVHEYDYWGQGTLVTVSS (SEQ ID NO: 29)
> SDP01315 VH1 
EVQLVESGGGLVKPGGSLRLSCAASGFTFSSYAMSWVRQAPGKRLEWVSSISSG
GSTYYPDSVKGRFTISRDNAKNSLYLQMNSLRAEDTAVYYCARDGYPPFDYWG
QGTTVTVSS (SEQ ID NO: 30)
> SDP01315 VH2 
EVQLVESGGGLVKPGGSLRLSCAASGFTFSSYAMSWVRQAPGKRLEWVASISSG
GSTYYPDSVKGRFTISRDNAKNSLYLQMNSLRAEDTAVYYCARDGYPPFDYWG
 QGTTVTVSS (SEQ ID NO: 31)
> SDP01315 VH3 
EVQLVESGGGLVQPGGSLRLSCAASGFTFSSYAMSWVRQAPGKRLEWVASISSG
GSTYYPDSVKGRFTISRDNAKNTLYLQMNSLRAEDTAVYYCARDGYPPFDYWG
QGTLVTVSS (SEQ ID NO: 32)
> SDP01315 VL1 
DIQMTQSPSSVSASVGDRVTITCKASQNVGTNVAWYQQKPGKAPKLLIYSASYR
YSGVPSRFSGSGSGTDFTLTISSLQPEDFATYYCQQYNSFPLTFGQGTKLEIK
(SEQ ID NO: 33)
> SDP01315 VL2 
DIQMTQSPSSVSASVGDRVTITCKASQNVGTNVAWYQQKPGKAPKALIYSASYR
YSGVPSRFSGSGSGTDFTLTISSLQPEDFATYYCQQYNSFPLTFGQGTKLEIK
(SEQ ID NO: 34)
> SDP01315 VL3

DIQMTQSPSSVSASVGDRVTVTCKASQNVGTNVAWYQQKPGKSPKALIYSASY
RYSGVPSRFSGSGSGTDFTLTISSLQPEDFATYFCQQYNSFPLTFGQGTKLEIK
(SEQ ID NO: 35)

After the single-domain antibody was humanized, a full-length antibody was constructed; GGGGSHHHHHH (SEQ ID NO: 72) was added after the corresponding VHH. Alternatively, after the single-domain antibody was humanized, a full-length antibody was constructed; a linker and an Fc were added after the corresponding VHH.

After the murine-derived antibody was humanized, a full-length antibody was constructed; the light chain constant region used was a Cκ, and the heavy chain constant region used was IgG1 with mutations L234F and L235E.

**Table 5. The correspondence between antibody names and antibody structures**

| Antibody No. | Heavy chain variable region | Light chain variable region |
|---|---|---|
| SDP01375 | SDP01346 VH1 | / |
| SDP01376 | SDP01346 VH2 | / |
| SDP01377 | SDP01346 VH3 | / |
| SDP01378 | SDP01346 VH4 | / |
| SDP05576 | SDP01346 VH4 | / |
| SDP01365 | SDP01315 VH1 | SDP01315 VL1 |
| SDP01366 | SDP01315 VH2 | SDP01315 VL1 |
| SDP01367 | SDP01315 VH3 | SDP01315 VL1 |
| SDP01368 | SDP01315 VH1 | SDP01315 VL2 |
| SDP01369 | SDP01315 VH2 | SDP01315 VL2 |
| SDP01370 | SDP01315 VH3 | SDP01315 VL2 |
| SDP01371 | SDP01315 VH1 | SDP01315 VL3 |
| SDP01372 | SDP01315 VH2 | SDP01315 VL3 |
| SDP01373 | SDP01315 VH3 | SDP01315 VL3 |

Illustratively, the full-length sequences of SDP01378, SDP05576, and SDP01369 are shown below.
> The full-length sequence of SDP01378 
EVQLVESGGGLVQPGGSLRLSCSVSGSTFSDFAMGWLRQAPGKEREFVAAISWT
GGRTYYADSVKGRFTISRDNSKNTVYLQMNSLRAEDTAVYYCAASRDCSGPGC
RVHEYDYWGQGTLVTVSSGGGGSHHHHHH
(SEQ ID NO: 36)
> SDP05576 
EVQLVESGGGLVQPGGSLRLSCSVSGSTFSDFAMGWLRQAPGKEREFVAAISWT
GGRTYYADSVKGRFTISRDNSKNTVYLQMNSLRAEDTAVYYCAASRDCSGPGC
RVHEYDYWGQGTLVTVSSAAASDKTHTCPPCPAPELLGGPSVFLFPPKPKDTLM
ISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSKLTVD
KSRWQQGNVFSCSVMHEALHNHYTQKSLSLSPGK
(SEQ ID NO: 73)
> The full-length heavy chain of SDP01369 
EVQLVESGGGLVKPGGSLRLSCAASGFTFSSYAMSWVRQAPGKRLEWVASISSG
GSTYYPDSVKGRFTISRDNAKNSLYLQMNSLRAEDTAVYYCARDGYPPFDYWG
QGTTVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHT
CPPCPAPEFEGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK
(SEQ ID NO: 37)
> The full-length light chain of SDP01369

DIQMTQSPSSVSASVGDRVTITCKASQNVGTNVAWYQQKPGKAPKALIYSASYR
YSGVPSRFSGSGSGTDFTLTISSLQPEDFATYYCQQYNSFPLTFGQGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
(SEQ ID NO: 38)

Below, the binding of humanized monoclonal antibodies to γ9δ2 T cells was assessed by FACS.

Affinity assay after humanization of single-domain antibody SDP01346: The serially diluted antibody molecule was incubated with 1 × 10⁵ γ9δ2 T cells at 4 °C for 1 h, and the excess antibody was washed off. The DyLight 405-AffiniPure Goat Anti-Alpaca IgG, VHH domain antibody (Jackson, Cat#128-475-232) was added, and the cells were incubated at 4 °C for 30 min. After the excess antibody was washed off, the cells were resuspended in 200 µL of a 2% FBS/PBS buffer, and the fluorescence signals on the cell surface were read using a Thermo Attune NxT flow cytometer. The results are shown in Table 6.

Affinity assay after humanization of murine-derived chimeric antibody SDP01315: The serially diluted antibody molecule was incubated with 1 × 10⁵ γ9δ2 T cells at 4 °C for 1 h, and the excess antibody was washed off. The murine-derived Alexa Flour 647 labeled anti-human Fc antibody (Jackson, Cat#209-605-098) was added, and the cells were incubated at 4 °C for 30 min. After the excess antibody was washed off, the cells were resuspended in 200 µL of a 2% FBS/PBS buffer, and the fluorescence signals on the cell surface were read using a Thermo Attune NxT flow cytometer. The results are shown in Table 6.

**Table 6. Affinity assays of humanized molecules of SDP01346 and SDP01315**

| Antibody No. | Binding to γ9δ2 T cells | |
|---|---|---|
| | EC₅₀ (nM) | Max MFI |
| SDP01375 | 1.27 | 1093 |
| SDP01376 | 0.70 | 1252 |
| SDP01377 | 0.77 | 1237 |
| SDP01378 | 1.06 | 1496 |
| SDP01365 | 2.42 | 15519 |
| SDP01366 | 2.11 | 14836 |
| SDP01367 | 1.79 | 14698 |
| SDP01368 | 1.67 | 14854 |
| SDP01369 | 1.74 | 15014 |
| SDP01370 | 2.00 | 14922 |
| SDP01371 | 1.70 | 15385 |
| SDP01372 | 1.69 | 15470 |
| SDP01373 | 2.50 | 15678 |

The results indicate that SDP01378, a humanized molecule of the single-domain antibody, exhibited the strongest affinity, and SDP01365-SDP01373, humanized molecules of SDP01346, were comparable in affinity. SDP01369, which contained a moderate number of back mutations, was selected.

**Example 6. Design and Preparation of Anti-GPC3/γδTCR Bispecific Antibodies** For the GPC3 end, antibody G with nanomolar affinity was selected. The sequences of the heavy and light chain variable regions are shown below. Antibody G binds to a C-terminal subunit and does not bind to soluble GPC3.
> GPC3 VH 
QVQLVQSGAEVKKPGASVKVSCKASGYTFTDYEMHWVRQAPGQGLEWMGAL
DPKTGDTAYSQKFKGRVTLTADKSTSTAYMELSSLTSEDTAVYYCTRFYSYTYW
 GQGTLVTVSS (SEQ ID NO: 39)
> GPC3 VL

DVVMTQSPLSLPVTPGEPASISCRSSQSLVHSNRNTYLHWYLQKPGQSPQLLIYK
VSNRFSGVPDRFSGSGSGTDFTLKISRVEAEDVGVYYCSQNTHVPPTFGQGTKL
EIK (SEQ ID NO: 40)

**Table 7. The VH and VL of antibody G (the Kabat numbering scheme)**

| Antibody No. | CDR sequences | | Sequence No. |
|---|---|---|---|
| Antibody G | HCDR1 | DYEMH | SEQ ID NO: 41 |
| | HCDR2 | ALDPKTGDTAYSQKFKG | SEQ ID NO: 42 |
| | HCDR3 | FYSYTY | SEQ ID NO: 43 |
| | LCDR1 | RSSQSLVHSNRNTYLH | SEQ ID NO: 44 |
| | LCDR2 | KVSNRFS | SEQ ID NO: 45 |
| | LCDR3 | SQNTHVPPT | SEQ ID NO: 46 |

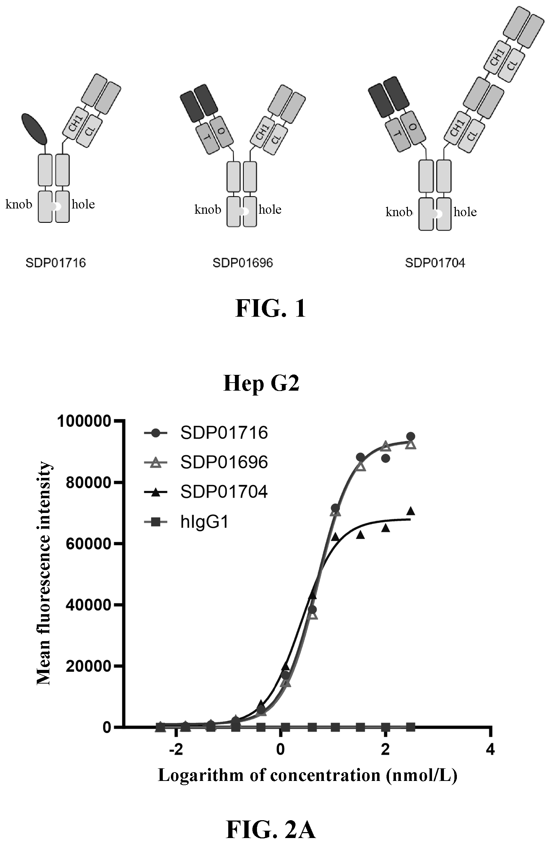

We designed a variety of GPC3/γδTCR bispecific antibodies, and finally selected a 1+1 asymmetric structure formed with SDP01346H4 (i.e., SDP01378), in which KIH (knob into hole) was used to avoid heavy chain mispairing, and a 2+1 asymmetric structure formed with SDP01315H2L2, in which KIH was used to avoid heavy chain mispairing, and HOT was used to avoid light chain mispairing. The structures are schematically shown in FIG. 1.

To prevent and reduce heavy and light chain mispairing, the Obscurin-O and Titin-T used are set forth in SEQ ID NOs: 47-48, and the CH1 and Cκ are set forth in SEQ ID NOs: 49-50. To prevent and reduce mispairing between two heavy chains, IgG mutations were used. The full-length sequences of the bispecific antibodies are shown below. In the sequences, the underlined portions are CDR sequences, the portion underlined with a wavy line is the Obscurin-O or Titin-T sequence, the portion underlined with a dotted line is the CH1 or Cκ, the italicized portions are IgG1 Fc regions, the italicized portions in bold type are point mutations in the Fc regions, and the portions in bold type are linkers.
> Obscurin-O 
SGAPRFLTRPKASVVSVGKDATLSCQIVGNPFPQVSWEKDKQPVTAGVRFRLAQ
DGDLYRLKILDLQLSDSGQYVCRARNAHGEAFACLGLQVDAEA
(SEQ ID NO: 47)
> Titin-T 
GIPPKIECLPIDISIDEGKVLTVASAFTGEPTPEVTWSTGGRKIHSQEQGRFHIENT
DDSTTLTIKDVQKQDGGLYTLTLRNEFGSDSATVNIHIRSI (SEQ ID NO: 48)
> CH1 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSC
(SEQ ID NO: 49)
> Cκ  

(SEQ ID NO: 50)
> IgG1 Fc (WT) 
DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHN
HYTQKSLSLSPGK (SEQ ID NO: 51)
> IgG1 Fc1 (with mutations S354C/T366W/L234F/L235E) 
DKTHTCPPCPAPEFEGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPCREEMTKNQVSLWCLVKGFYPSDIAVEW
ESNGQPENNYKTTPPVLDSDGSFFLYSKLTVDKSRWQQGNVFSCSVMHEALHN
HYTQKSLSLSPGK (SEQ ID NO: 52)
> IgG1 Fc2 (with mutations Y349C/T366S/L368A/Y407V/L234F/L235E) 
DKTHTCPPCPAPEFEGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVCTLPPSREEMTKNQVSLSCAVKGFYPSDIAVEW
ESNGQPENNYKTTPPVLDSDGSFFLVSKLTVDKSRWQQGNVFSCSVMHEALHN
HYTQKSLSLSPGK (SEQ ID NO: 53)
> SDP01716 H1 
EVQLVESGGGLVQPGGSLRLSCSVSGSTFSDFAMGWLRQAPGKEREFVAAISWT
GGRTYYADSVKGRFTISRDNSKNTVYLQMNSLRAEDTAVYYCAASRDCSGPGC
RVHEYDYWGQGTLVTVSSAAASDKTHTCPPCPAPEFEGGPSVFLFPPKPKDTLMI
SRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPCREEMTKNQVSLW
CLVKGFYPSDIAVEWESNGQPENNYKTTPPVLDSDGSFFLYSKLTVDKSRWQQGNVF
SCSVMHEALHNHYTQKSLSLSPGK (SEQ ID NO: 54)
> SDP01716 H2 
QVQLVQSGAEVKKPGASVKVSCKASGYTFTDYEMHWVRQAPGQGLEWMGAL
DPKTGDTAYSQKFKGRVTLTADKSTSTAYMELSSLTSEDTAVYYCTRFYSYTYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPEFEGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALP
APIEKTISKAKGQPREPQVCTLPPSREEMTKNQVSLSCAVKGFYPSDIAVEWESNGQP
ENNYKTTPPVLDSDGSFFLVSKLTVD (SEQ ID NO: 55)
> SDP01716 L 
DVVMTQSPLSLPVTPGEPASISCRSSQSLVHSNRNTYLHWYLQKPGQSPQLLIYK
VSNRFSGVPDRFSGSGSGTDFTLKISRVEAEDVGVYYCSQNTHVPPTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
 EC (SEQ ID NO: 56)
> **SDP01696** H1 
EVQLVESGGGLVKPGGSLRLSCAASGFTFSSYAMSWVRQAPGKRLEWVASISSG
GSTYYPDSVKGRFTISRDNAKNSLYLQMNSLRAEDTAVYYCARDGYPPFDYWG
QGTTVTVSSGGGGSSGAPRFLTRPKASVVSVGKDATLSCQIVGNPFPQVSWEK
DKQPVTAGVRFRLAQDGDLYRLKILDLQLSDSGQYVCRARNAHGEAFACLGLQ
VDAEADKTHTCPPCPAPEFEGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKA
LPAPIEKTISKAKGQPREPQVYTLPPCREEMTKNQVSLWCLVKGFYPSDIAVEWESNG
QPENNYKTTPPVLDSDGSFFLYSKLTVDKSRWQQGNVFSCSVMHEALHNHYTQKSL
SLSPGK (SEQ ID NO: 57)
> SDP01696 L1 
DIQMTQSPSSVSASVGDRVTITCKASQNVGTNVAWYQQKPGKAPKALIYSASYR
YSGVPSRFSGSGSGTDFTLTISSLQPEDFATYYCQQYNSFPLTFGQGTKLEIKGGG
GSGIPPKIECLPIDISIDEGKVLTVASAFTGEPTPEVTWSTGGRKIHSQEQGRFHIE
NTDDSTTLTIKDVQKQDGGLYTLTLRNEFGSDSATVNIHIRSI    (SEQ ID NO: 58)
> SDP01696 H2 
QVQLVQSGAEVKKPGASVKVSCKASGYTFTDYEMHWVRQAPGQGLEWMGAL
DPKTGDTAYSQKFKGRVTLTADKSTSTAYMELSSLTSEDTAVYYCTRFYSYTYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPEFEGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALP
APIEKTISKAKGQPREPQVCTLPPSREEMTKNQVSLSCAVKGFYPSDIAVEWESNGQP
ENNYKTTPPVLDSDGSFFLVSKLTVDKSRWQQGNVFSCSVMHEALHNHYTQKSLSLS
PGK (SEQ ID NO: 59)
> SDP01696 L2
   The same as SDP01716 L (SEQ ID NO: 56).
> SDP01704 H1
   The same as SDP01696 H1 (SEQ ID NO: 57).
> SDP01704 L1
   The same as SDP01696 L1 (SEQ ID NO: 58).
> SDP01704 H2 
QVQLVQSGAEVKKPGASVKVSCKASGYTFTDYEMHWVRQAPGQGLEWMGAL
DPKTGDTAYSQKFKGRVTLTADKSTSTAYMELSSLTSEDTAVYYCTRFYSYTYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDGGGGSGGGGSQVQLVQSGAEVKKPGASVKVSCKASGYTFTDYEMHW
VRQAPGQGLEWMGALDPKTGDTAYSQKFKGRVTLTADKSTSTAYMELSSLTSE
DTAVYYCTRFYSYTYWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYIC
NVNHKPSNTKVDKKVEPKSCDKTHTCPPCPAPEFEGGPSVFLFPPKPKDTLMISR
 
TPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSREEMTKNQVSLSCA
VKGFYPSDIAVEWESNGQPENNYKTTPPVLDSDGSFFLVSKLTVDKSRWQQGNVFSC
SVMHEALHNHYTQKSLSLSPGK (SEQ ID NO: 60)
> SDP01704 L2

DVVMTQSPLSLPVTPGEPASISCRSSQSLVHSNRNTYLHWYLQKPGQSPQLLIYK
VSNRFSGVPDRFSGSGSGTDFTLKISRVEAEDVGVYYCSQNTHVPPTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC (SEQ ID NO: 61)

Nucleotide sequences encoding the antibodies were cloned into pTT5 vectors, and ExpiCHO cells were then transfected with the vectors. After 8 days, the cells were removed by centrifugation, and the cell culture solution was collected and filtered. The collected cell culture solution was purified using a Protein A affinity column (MabSelect SuRe, GE), and the bound antibodies were eluted with glycine. The eluates were neutralized with 1 M Tris and then desalted. According to analysis, the target antibodies SDP01716, SDP01696, and SDP01704 were obtained.

### Example 7. Measurement of Binding Affinity of Anti-GPC3/γδTCR Bispecific Antibodies for Human and Monkey Antigens

A Protein A biosensor chip (Cat. # 29139121-AB, Cytiva) was used. Each test antibody was dissolved in an HBS-EP+ buffer solution as a ligand and captured by Protein A on the chip channels. The human GPC3 (Sino Biological, Cat#10088-H08H)/cynomolgus monkey GPC3 (Acrobiosystems, Cat#GP3-C5225)/human γ9δ2/cynomolgus monkey γ9δ2 antigen was used as an analyte and dissolved in an HBS-EP+ buffer solution, and the analyte was serially diluted 2-fold. The diluted antigen was allowed to flow through the experimental and reference channels at a flow rate of 30 µL/min (association: 80 seconds; disassociation: 300 seconds). 10 mM glycine pH 1.5 (GE Healthcare, BR-1003-54) was selected as a regeneration buffer and allowed to flow at a flow rate of 10 µL/min for 30 seconds. Data were analyzed using Biacore 8K evaluation software.

The results are shown in Tables 8 and 9. The antigen-binding affinity of the three antibodies to the human and cynomolgus monkey GPC3 proteins was about 2 nM, which was similar to the affinity of antibody G. The binding K_{D} values of the three antibodies to the cynomolgus monkey γ9δ2 TCR protein were about 50 nM.

**Table 8. Kinetic parameters of the binding of antibodies to the human/cynomolgus monkey GPC3 protein**

| Antibody No. | Antigen | ka (1/Ms) | kd (1/s) | K_{D} (M) |
|---|---|---|---|---|
| SDP01716 | Human GPC3 | 1.47E+05 | 2.42E-04 | 1.65E-09 |
| SDP01696 | | 1.42E+05 | 2.47E-04 | 1.74E-09 |
| SDP01704 | | 1.36E+05 | 2.50E-04 | 1.84E-09 |
| SDP01716 | Cynomolgus monkey GPC3 | 1.87E+05 | 2.75E-04 | 1.47E-09 |
| SDP01696 | | 1.96E+05 | 2.88E-04 | 1.47E-09 |
| SDP01704 | | 1.78E+05 | 2.89E-04 | 1.63E-09 |

**Table 9. Kinetic parameters of the binding of antibodies to the human/cynomolgus monkey γ9δ2 protein**

| Antibody No. | Antigen | ka (1/Ms) | kd (1/s) | K_{D} (M) |
|---|---|---|---|---|
| SDP01716 | Human γ9δ2 | 4.26E+05 | 4.27E-04 | 1.00E-09 |
| SDP01696 | | 2.54E+04 | 1.03E-03 | 4.06E-08 |
| SDP01704 | | 2.67E+04 | 1.02E-03 | 3.83E-08 |
| SDP01716 | Cynomolgus monkey γ9δ2 | 1.99E+05 | 1.37E-02 | 6.88E-08 |
| SDP01696 | | 1.40E+05 | 5.92E-03 | 4.24E-08 |
| SDP01704 | | 1.48E+05 | 6.01E-03 | 4.06E-08 |

### Example 8. Measurement of Binding Activity of Anti-GPC3/γδTCR Bispecific Antibodies to GPC3-Positive Cells and Human γ9δ2 T Cells

The binding activity of γδT bispecific antibodies to HepG2 cells that naturally express human GPC3 at high levels (purchased from ATCC) and DLD-1 cells that do not express human GPC3 (purchased from ATCC) was measured through a FACS assay. In the assay for measuring the GPC3-binding activity of the anti-GPC3/γδTCR bispecific antibodies on cells, fluorescence signals of the antibodies that bound to the cell surface were detected, and the binding strength of the antibodies was evaluated according to the intensity of the fluorescence signals. Specifically, the serially diluted antibody molecule and control molecule were incubated with 1 × 10⁵ cells at 4 °C for 1 h, and the excess antibody was washed off. The murine-derived Alexa Flour 647 labeled anti-human Fc antibody (Jackson, Cat#209-605-098) was added, and the cells were incubated at 4 °C for 30 min. After the excess antibody was washed off, the cells were resuspended in 200 µL of a 2% FBS/PBS buffer, and the fluorescence signals on the cell surface were read using a Thermo Attune NxT flow cytometer. The cells used were cells of the HepG2 cell line.

The binding activity of the anti-GPC3/γδTCR bispecific antibodies to human γ9δ2 T cells and their binding activity to human PBMCs were measured through a FACS assay. The human γ9δ2 T cells were obtained by induction with zoledronic acid and IL-2. In the assay for measuring the binding activity of the anti-GPC3/γδTCR bispecific antibodies on γδ T cells, fluorescence signals of the antibodies that bound to the cell surface were detected, and the binding strength of the antibodies was evaluated according to the intensity of the fluorescence signals. Specifically, the serially diluted antibody molecule and control molecule were incubated with 1 × 10⁵ cells at 4 °C for 1 h, and the excess antibody was washed off. The murine-derived Alexa Flour 647 labeled anti-human Fc antibody (Jackson, Cat#209-605-098) was added, and the cells were incubated at 4 °C for 30 min. After the excess antibody was washed off, the cells were resuspended in 200 µL of a 2% FBS/PBS buffer, and the fluorescence signals on the cell surface were read using a Thermo Attune NxT flow cytometer.

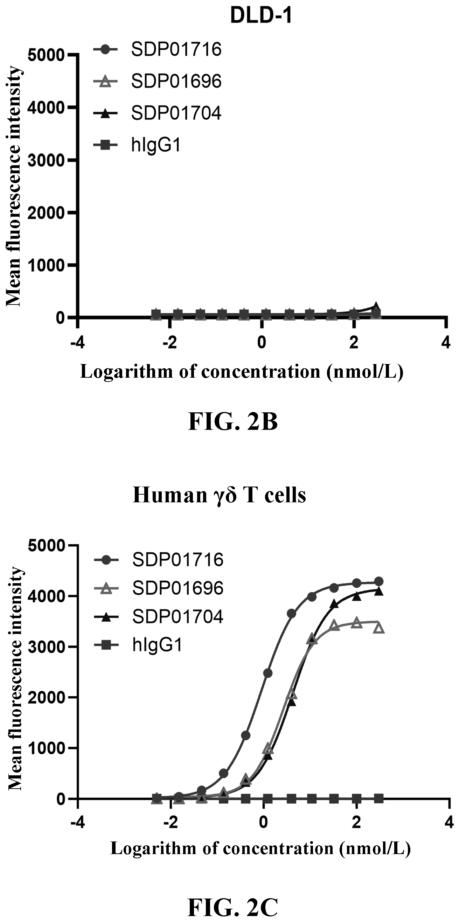

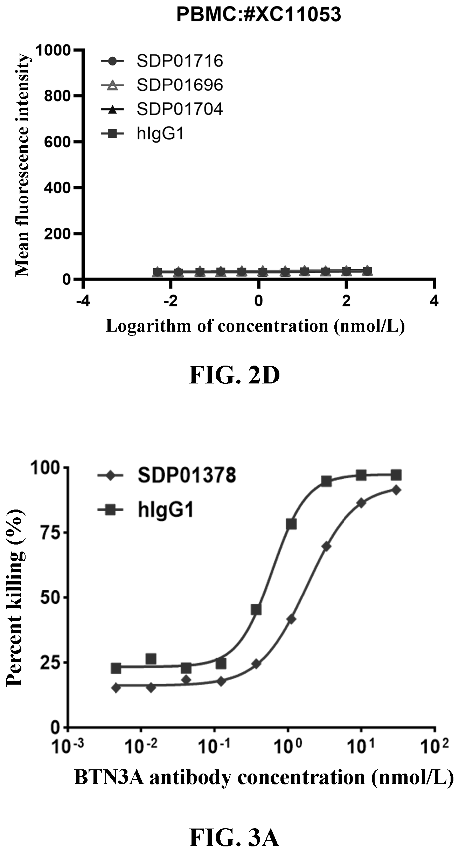

The results are shown in FIGs. 2A-2D. As shown in FIGs. 2A and 2B, the GPC3 ends of SDP01716 and SDP01696 were comparable in affinity, with their EC₅₀ values being 4.853 nM and 5.166 nM, respectively. SDP01704 showed the characteristics of bivalent binding, and its EC₅₀ value was 2.466 nM; none of the tested antibodies exhibited nonspecific binding on GPC3-negative cells. As shown in FIGs. 2C and 2D, the affinity of SDP01716 for γ9δ2 T cells was 0.9064 nM, the affinity of SDP01696 was 2.676 nM, and the affinity of SDP01704 was 4.289 nM; none of the tested antibodies exhibited nonspecific binding on PBMCs.

### Example 9. Effects of Anti-GPC3/γδTCR Bispecific Antibodies on BTN2A/BTN3A-TCR Natural Signals

In the natural state, phosphorylated antigens in cells alter the molecular tension of BTN3A after binding to BTN3A; two BTN3A molecules and two BTN2A molecules bind to form a heterotetramer, and the heterotetramer interacts with TCRs, activates the TCRs, and induces the activation, proliferation, killing, and other functions of γδ T cells. Given that anti-γδTCR antibodies also bind to TCRs, the effects of the antibodies of the present disclosure on BTN2A/BTN3A-TCR natural signals were assessed in this example. SDP01378 was a monovalent molecule of the γδT end of SDP01716, and SDP01315 was a monovalent molecule of SDP01696 and SDP01704.

The A375-Luc cell culture medium was RPMI1640 containing 10% inactivated fetal bovine serum. γδ T cells were obtained by expansion of PBMCs. The density of A375-Luc cells was adjusted, and the cells were seeded into a 96-well plate at 25 µL/well (1 × 10⁴ cells/well). γδ T cells (25 µL/well, 1 × 10⁵ cells/well) were mixed with A375-Luc cells. Then, SDP01378 or SDP01315 (final concentration: 10 nM) was added at 25 µL/well, and a serially diluted BTN3A agonistic antibody was added at 25 µL/well. The plate was incubated in an incubator at 37 °C with 5% CO₂ for 24 h. After 24 h, the Bright-GloTM Luciferase Assay System (Promega, E2650) reagent was added at 100 µL/well for detection. Refer to the instructions for the reagent for specific procedures.

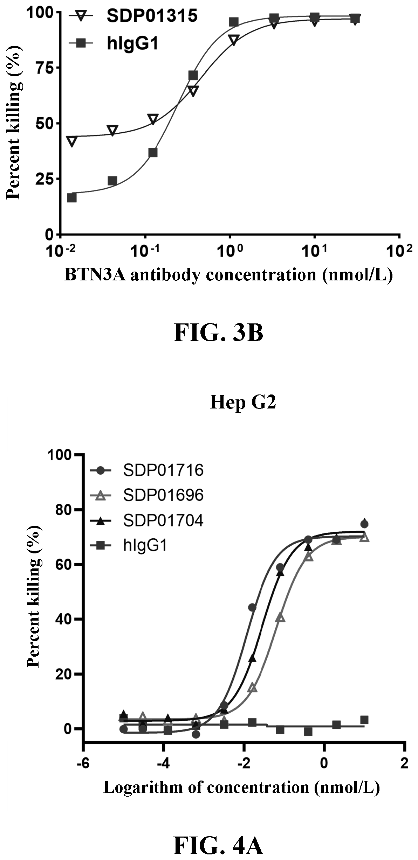

The results are shown in FIGs. 3A and 3B. Neither SDP01378 nor SDP01315 blocked BTN2A/BTN3A-TCR natural signals. Thus, it can be considered that the anti-GPC3/γδTCR bispecific antibodies of the present disclosure did not block BTN2A/BTN3A-TCR natural signals.

### Example 10. Measurement of Anti-GPC3/γδTCR Bispecific Antibody-Mediated In Vitro Killing Activity of γδ T Cells Against Tumor Cells

In this example, the antibody-mediated killing activity of γδ T cells against target cells with different GPC3 expression levels was assessed through a lactate dehydrogenase (LDH) assay.

HepG2 cells naturally express GPC3 at high levels, and the cell culture medium was DMEM (Gibco, Cat#11995-065, the same applies hereinafter) containing 15% inactivated fetal bovine serum. Huh-7 cells express GPC3 at moderate levels, and the cell culture medium was DMEM. MKN-45 cells express GPC3 at low levels, and the cell culture medium was RPMI 1640 (Gibco, Cat#10491A-01, the same applies hereinafter). DLD-1 cells do not express GPC3, and the cell culture medium was RPMI 1640. The target cells were digested and then resuspended in an RPMI1640 culture medium containing 2% serum, and the density was adjusted to 7 × 10⁴ cells/mL. Subsequently, the cells were seeded into 96-well plates at 50 µL/well, and the test antibodies (serially diluted, 50 µL) were added. An additional 50 µL of culture medium was added to each well. γδ T cells were collected and resuspended in RPMI1640 containing 2% fetal bovine serum, and the cell density was adjusted. The cells were seeded into the above assay plates at 50 µL/well, and the plates were incubated in an incubator at 37 °C with 5% CO₂ for 24 h. The cell culture plates were taken out and centrifuged at 400 g for 5 min, and the cell culture supernatants were collected and assayed for LDH levels using a CytoTox 96^{®} Non-Radioactive Cytotoxicity Assay kit (Promega, G1780). Refer to the instructions for the reagent for specific procedures.

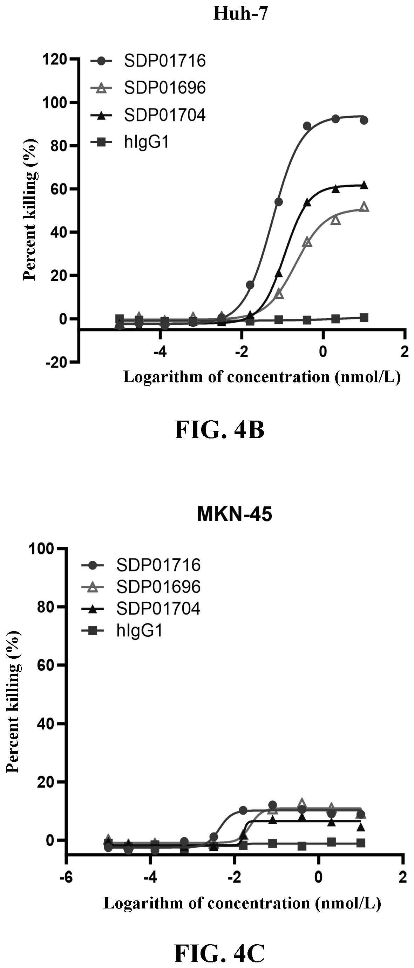

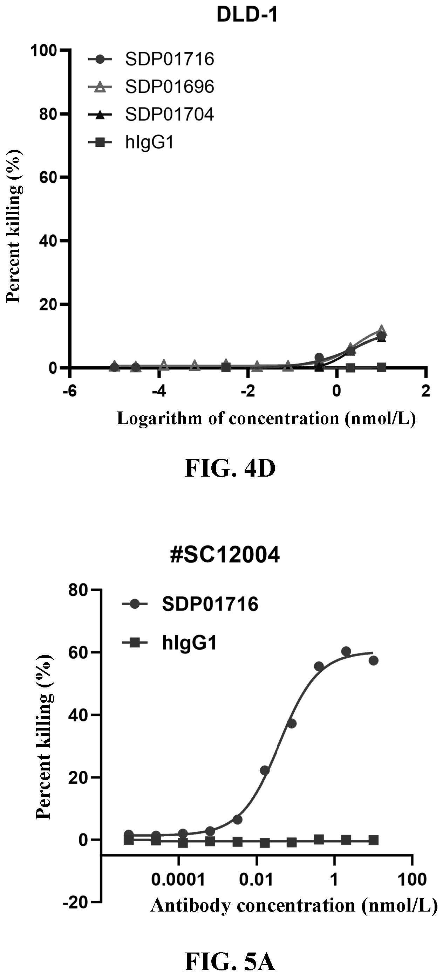

The results are shown in FIGs. 4A to 4D and Table 10. The killing activity of the bispecific antibodies of the present disclosure against tumor cells weakened as the antigen expression level decreased. The affinity of the γδT ends of the antibodies was positively correlated with the killing activity against tumor cells, and the GPC3-end bivalent molecules were superior to the monovalent molecules.

**Table 10. Killing of cells with different antigen expression levels by anti-GPC3/γδTCR bispecific antibodies**

| Antibody No. | γδT killing (EC₅₀, nM) | | | | | | | |
|---|---|---|---|---|---|---|---|---|
| | HepG2 | | Huh-7 | | MKN-45 | | DLD-1 | |
| | EC₅₀ | Emax | EC₅₀ | Emax | EC₅₀ | Emax | EC₅₀ | Emax |
| SDP01716 | 0.01 | 70.3% | 0.06 | 93.8% | 0.004 | 10.3% | - | - |
| SDP01696 | 0.06 | 70.1% | 0.21 | 50.9% | 0.024 | 11.0% | - | - |
| SDP01704 | 0.03 | 72.0% | 0.11 | 61.7% | 0.016 | 6.6% | - | - |
| hIgG1 | - | - | - | - | - | - | - | - |

| | | | | | | | | |
|---|---|---|---|---|---|---|---|---|
| Note: - means that no killing activity was detected. | | | | | | | | |

### Example 11. Effect of Anti-GPC3/γδTCR Bispecific Antibodies on Killing by γδ T Cells Derived from Different PBMC Donors

In this example, the antibody-mediated killing activity of γδ T cells against GPC3-expressing target cells was assessed through a lactate dehydrogenase (LDH) assay.

HepG2 cells were digested and then resuspended in an RPMI1640 culture medium containing 2% serum, and the cell density was adjusted to 7 × 10⁴ cells/mL. Subsequently, the cells were seeded into 96-well plates at 50 µL/well, and the test antibodies (serially diluted, 50 µL) were added. An additional 50 µL of culture medium was added to each well. Expanded γδ T cells from different donors were collected and resuspended in RPMI1640 containing 2% fetal bovine serum, and the cell density was adjusted. The cells were seeded into the above assay plates at 50 µL/well, and the plates were incubated in an incubator at 37 °C with 5% CO₂ for 24 h. The cell culture plates were taken out and centrifuged at 400 g for 5 min, and the cell culture supernatants were collected and assayed for LDH levels using a CytoTox 96^{®} Non-Radioactive Cytotoxicity Assay kit (Promega, G1780).

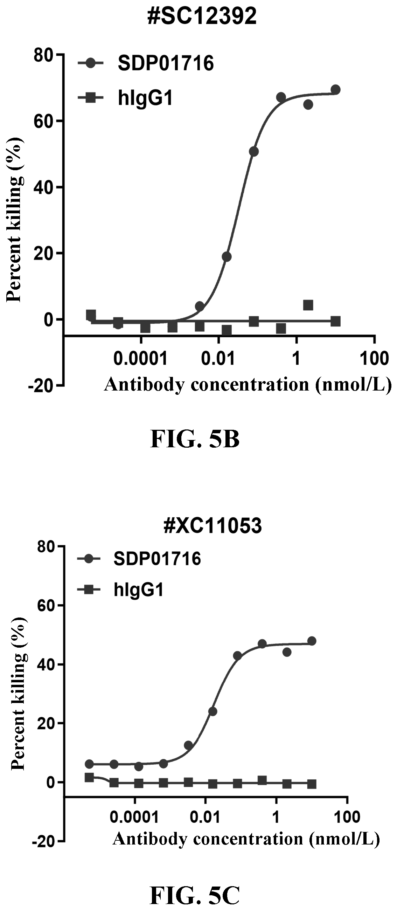

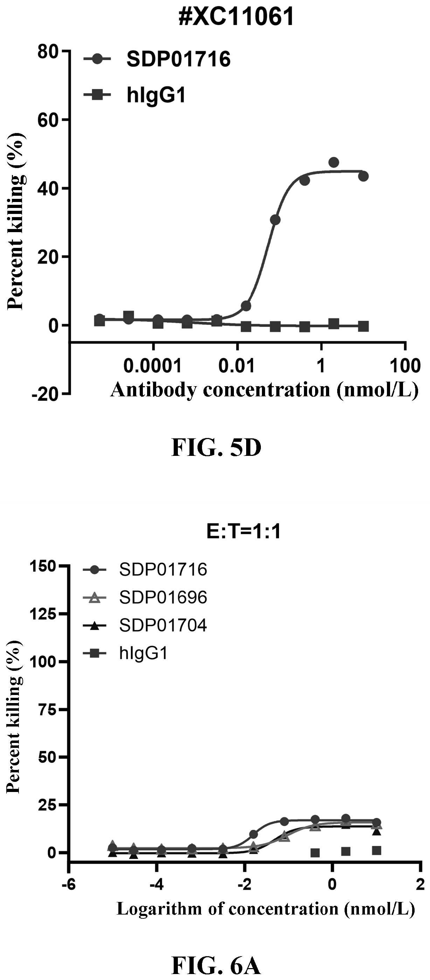

The results for SDP01716 are illustratively shown. The results are shown in FIGs. 5A to 5D and Table 11. SDP01716 could mediate the killing of tumor cells by γδ T cells derived from different donors, with no relatively significant differences in the EC₅₀ and maximum killing values.

**Table 11. The antibody-mediated killing activity of γδ T cells derived from different donors against HepG2**

| Antibody No. | Killing activity against HepG2 (EC₅₀, nM) | | | | | | | |
|---|---|---|---|---|---|---|---|---|
| | #SC12004 | | #SC12392 | | #XC11053 | | #XC11061 | |
| | EC₅₀ | Emax | EC₅₀ | Emax | EC₅₀ | Emax | EC₅₀ | Emax |
| SDP01716 | 0.038 | 60.30% | 0.033 | 68.23% | 0.017 | 46.94% | 0.054 | 44.95% |
| hIgG1 | - | - | - | - | - | - | - | - |

| | | | | | | | | |
|---|---|---|---|---|---|---|---|---|
| Note: #SC12004, #SC12392, #XC11053, and #XC11061 were expanded γδ T cells derived from different donors (healthy allogeneic human peripheral blood), and - means that no killing was detected. | | | | | | | | |

### Example 12. Measurement of Killing Activity of Anti-GPC3/γδTCR Bispecific Antibodies Against Cells with Low Antigen Expression at Different Effector-to-Target Ratios

In this example, the antibody-mediated killing activity of γδ T cells against target cells that express GPC3 at low levels was assessed through a lactate dehydrogenase (LDH) assay.

MKN-45 cells naturally express GPC3 at low levels. MKN-45 cells were digested and then resuspended in an RPMI1640 culture medium containing 2% serum, and the cell density was adjusted to 7 × 10⁴ cells/mL. Subsequently, the cells were seeded into 96-well plates at 50 µL/well, and the test antibodies (serially diluted, 50 µL) were added. An additional 50 µL of culture medium was added to each well. γδ T cells were collected and resuspended in RPMI1640 containing 2% fetal bovine serum, and the cell density was adjusted. The cells were seeded into the above assay plates at 50 µL/well, with the final effector-to-target ratios (γδ T cells:MKN-45 cells) being 1:1, 10:1, and 30:1. The plate was incubated in an incubator at 37 °C with 5% CO₂ for 24 h. The cell culture plates were taken out and centrifuged at 400 g for 5 min, and the cell culture supernatants were collected and assayed for LDH levels using a CytoTox 96^{®} Non-Radioactive Cytotoxicity Assay kit (Promega, G1780). Refer to the instructions for the reagent for specific procedures.

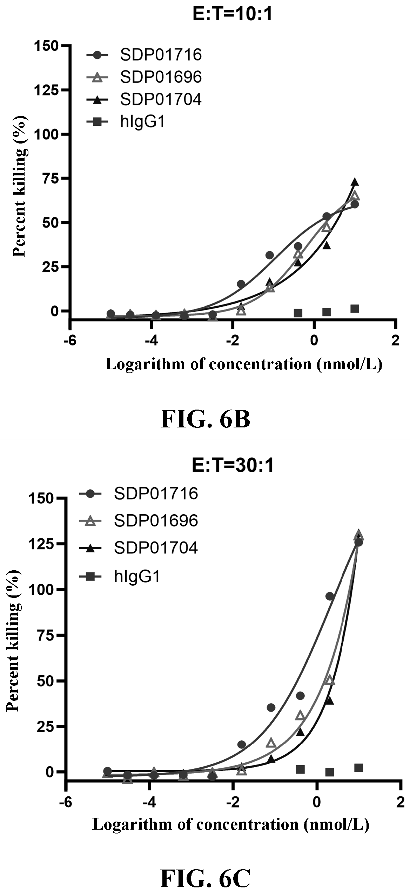

The results for SDP01716 are illustratively shown. As shown in FIG. 6A, when the effector-to-target ratio was 1:1, the killing activity of SDP01716 against cells with low antigen expression was weak, with the maximum killing value being 17%. As shown in FIG. 6B, after the effector-to-target ratio was increased to 10:1, SDP01716 could enhance the killing activity against cells with low antigen expression, with the maximum killing value reaching 64%. As shown in FIG. 6C, when the effector-to-target ratio was increased to 30:1, 100% killing was almost achieved. That is, the anti-GPC3/γδTCR bispecific antibodies of the present disclosure could kill target tumor cells in an effector-to-target ratio-dependent manner.

### Example 13. Increasing Proportion of γδ T cells in PBMCs to Enhance Killing Activity of Anti-GPC3/γδTCR Bispecific Antibodies and Cytokine Release Measurement

In this example, the antibody-mediated killing activity of γδ T cells against GPC3-expressing target cells was assessed through a lactate dehydrogenase (LDH) assay.

HepG2 cells naturally express GPC3 at high levels. HepG2 cells were digested and then resuspended in an RPMI1640 culture medium containing 2% serum, and the cell density was adjusted to 7 × 10⁴ cells/mL. Subsequently, the cells were seeded into 96-well plates at 50 µL/well, and the test antibodies (serially diluted, 50 µL) were added. An additional 50 µL of culture medium was added to each well. PBMCs were collected and resuspended in RPMI1640 containing 2% fetal bovine serum, and the cell density was adjusted. The cells were seeded into the above assay plates at 50 µL/well; or 30% γδ T cells were added to PBMCs, and the cells were seeded into the above assay plates at 50 µL/well. The plates were incubated in an incubator at 37 °C with 5% CO₂ for 24 h. The cell culture plates were taken out and centrifuged at 400 g for 5 min, and the cell culture supernatants were collected and assayed for LDH levels using a CytoTox 96^{®} Non-Radioactive Cytotoxicity Assay kit (Promega, G1780). Meanwhile, an IFNγ assay kit (Cisbio, S62HIFNGPEG) and a TNFα assay kit (Cisbio, 62HTNFAPEH) were used to determine the secretion levels of the corresponding cytokines. Refer to the instructions for the reagents for specific procedures.

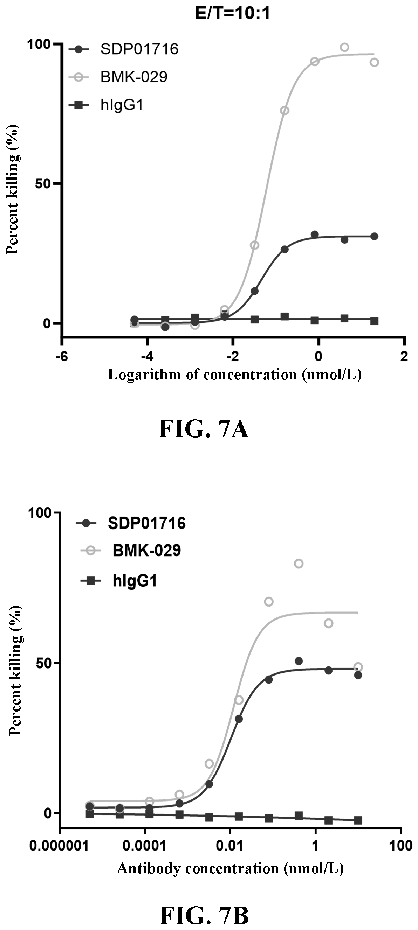

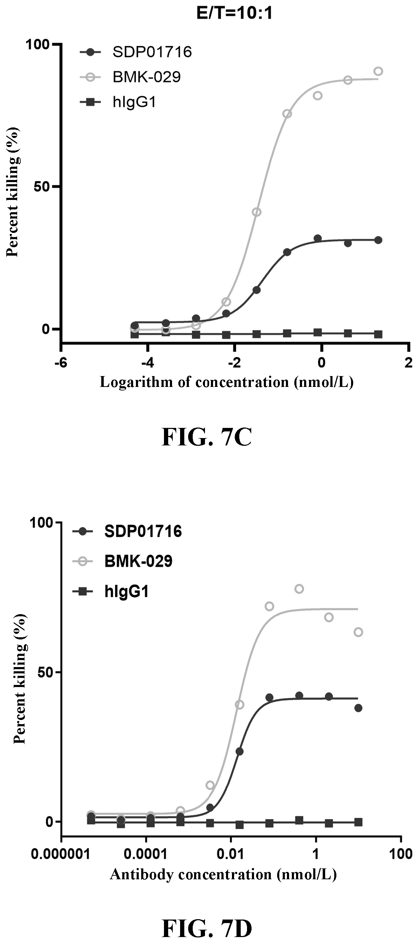

The results for SDP01716 are illustratively shown. The results are shown in FIGs. 7A to 7D. FIG. 7A shows killing by PBMCs of donor #XC11053, and FIG. 7B shows the killing activity after addition of 30% γδ T cells to PBMCs of donor #XC11053. FIG. 7C shows killing by PBMCs of donor #SC12392, and FIG. 7D shows the killing activity after addition of 30% γδ T cells to PBMCs of donor #SC12392.

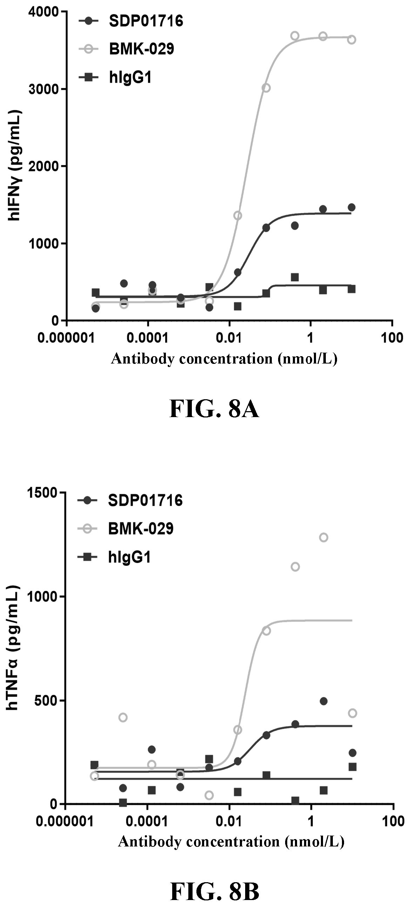

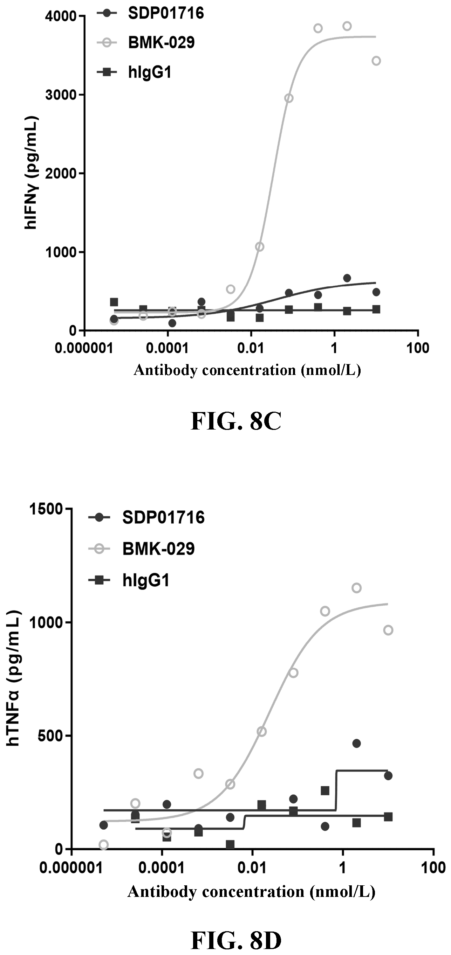

FIGs. 7A and 7C indicate that the ability of SDP01716 to kill tumor cells in a pure PBMC system was weaker than that of BMK-029 (ERY974 in US20220348658), which is a CD3 bispecific antibody against the same target, because the CD3 bispecific antibody can activate more T cells as effector cells to kill tumors. FIGs. 7B and 7D indicate that increasing the proportion of γδ T cells in PBMCs can improve their killing activity. Meanwhile, the cytokine release results corresponding to FIGs. 7A to 7D are shown in FIGs. 8A to 8D. SDP01716 induced less release of IFNγ and TNFα when killing tumor cells.

### Example 14. Measurement of γδ T Cell Proliferation-Promoting Activity of Anti-GPC3/γδTCR Bispecific Antibodies in PBMCs

In this example, the γδ T cell proliferation-inducing activity of anti-GPC3/γδTCR bispecific antibodies in PBMCs was assessed by flow cytometry.

PBMCs (800 µL, 1 × 10⁶ cells) were added to a 24-well plate. HepG2 cells were washed twice with PBS, and the cell density was then adjusted to 5 × 10⁵ cells/mL. 1 mL of the cell suspension was added to the corresponding well. Solutions of the test antibodies with the corresponding concentration were prepared using the culture medium, and 200 µL of each solution was then added to a well. The plate was incubated in an incubator at 37 °C with 5% CO₂, and the culture medium was refreshed every 3 days. After 6-7 days, the cells were washed twice with PBS and stained with a CD3 antibody (BD, Cat#564713), a Vδ2 TCR antibody (BD, Cat#555739), and a Vγ9 TCR antibody (BD, Cat#555732), and changes in the proportion of γδ T cells were identified.

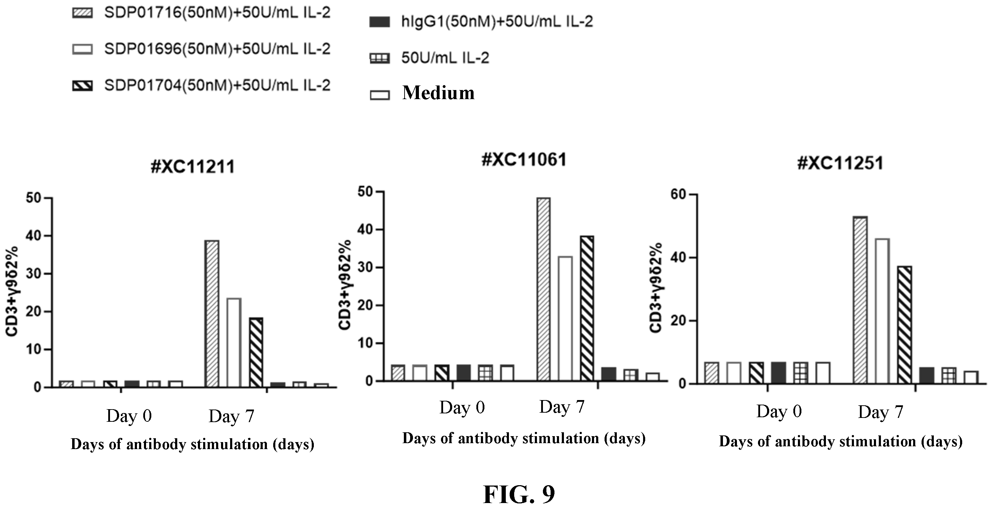

The results are shown in FIG. 9. In PBMCs derived from three donors: #XC11211, #XC11061, and #XC11251, after 6-7 days of stimulation, SDP01716, SDP01696, and SDP01704 could all significantly increase the proportion of γδ T cells.

### Example 15. Validation Using Human Liver Cancer Huh-7 Xenograft Tumor Model of Mice

1. Human liver cancer Huh-7 cells (Cell Bank of Chinese Academy of Sciences) were inoculated subcutaneously into NSG mice (female, 6-8 weeks old, provided by Vital River) at 5 × 10⁶ cells/100 µL/mouse, and the mice were randomly divided into 4 groups of 7 (G1-G4). Tumor cells were inoculated on the day of grouping, and γδ T cells and a drug were simultaneously infused. The groups were dosed according to the following regimens:
   G1: medium G2: γδ T cells + PBS
   G3: γδ T cells + SDP01716 (3 mg/kg)
   G4: γδ T cells + SDP01716 (0.3 mg/kg)

Thereafter, γδ T cells were infused and the drug was administered once weekly. During the administration and observation periods, the tumor volume was measured twice weekly, and the measurements were recorded. The calculation formulas are shown below (the same applies hereinafter):
The tumor volume (TV) was calculated using the formula TV = 1/2 × a × b², where a and
b represent the long and short diameters of the measured tumor, respectively.
The relative tumor proliferation rate T/C% = (T - T0)/(C - C0) × 100.
The tumor growth inhibition rate TGI% = 1 - T/C%.

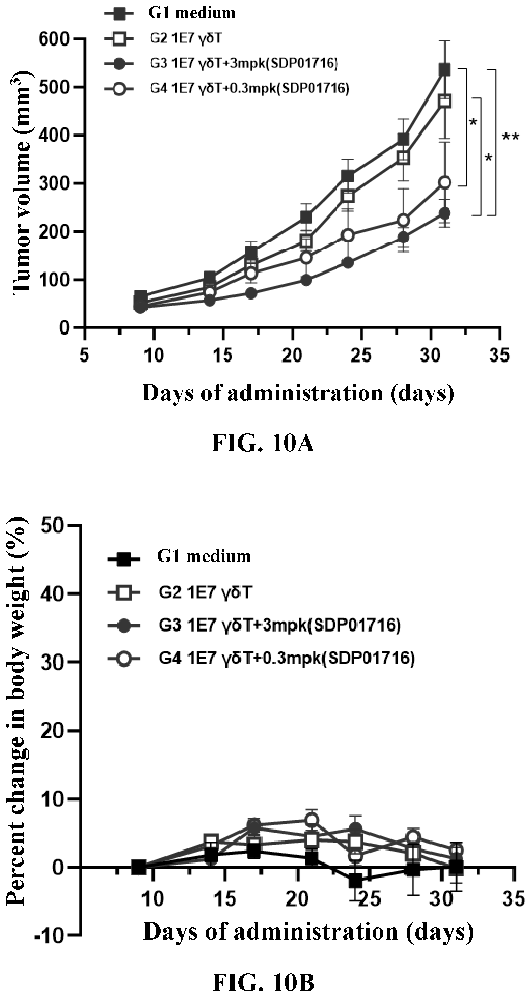

The results are shown in FIGs. 10A and 10B and Table 12. The γδ T cell monotherapy group (G2) exhibited a TGI of 11%; the inhibitory effect on tumor growth was limited. SDP01716 could inhibit tumor growth in a dose-dependent manner, with the TGI reaching 58% at a dose of 3 mpk, and no significant effect on mouse body weight was observed in each treatment group.

**Table 12. The anti-tumor activity of SDP01716 in a Huh-7 xenograft tumor model**

| Group | γδ T cell inoculation | Test drug | Administration dose | Route/frequency of administration | Tumor volume (mm³) | TGI (%) |
|---|---|---|---|---|---|---|
| G1 | PBS | PBS | / | ip, qw | 536.6±60 | - |
| G2 | 1E7 γδ T cells/injection, iv, qw | PBS | / | | 472.1±78 | 11 |
| G3 | | SDP01716 | 3 mpk | | 238.4±29 | 58** |
| G4 | | SDP01716 | 0.3 mpk | | 302.2±32 | 45* |

| | | | | | | |
|---|---|---|---|---|---|---|
| Note: * represents P < 0.05, and ** represents P < 0.01. | | | | | | |

2. Huh-7 cells were inoculated subcutaneously into NSG mice (female, 6-8 weeks old, provided by GemPharmatech) at 5 × 10⁶ cells/100 µL/mouse, and the mice were randomly divided into 6 groups of 7. Tumor cells were inoculated on the day of grouping, and γδ T cells and a drug were simultaneously infused. The groups were dosed according to the following regimens:
G1: Medium
G2: γδ T cells
G3: γδ T cells + SDP01716 (3 mg/kg)
G4: γδ T cells + SDP01696 (3.9 mg/kg)
G5: γδ T cells + SDP01704 (5.1 mg/kg).

Thereafter, γδ T cells were infused and the drug was administered once weekly; if γδ T cells were used in combination with an antibody, they were simultaneously administered. During the administration and observation periods, the tumor volume was measured twice weekly, and the measurements were recorded.

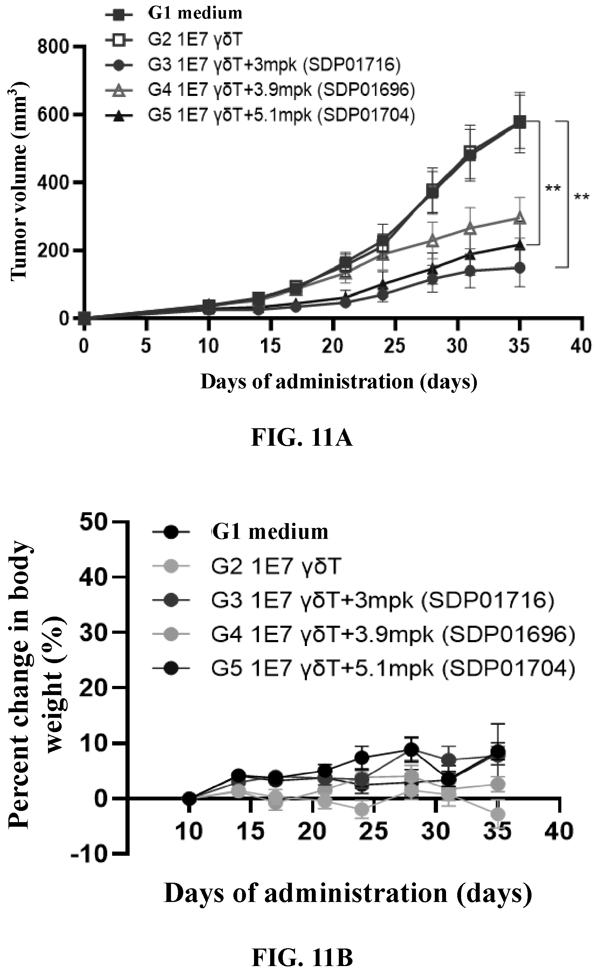

The results are shown in FIGs. 11A and 11B and Table 13. The bispecific antibodies of the present disclosure could all effectively inhibit tumors, and no significant change in mouse body weight was observed in each treatment group.

**Table 13. The anti-tumor activity of bispecific antibodies in a Huh-7 xenograft tumor model**

| Group | γδ T cell inoculation | Test drug | Administration dose | Route/frequency of administration | Tumor volume (mm³) | TGI (%) |
|---|---|---|---|---|---|---|
| G1 | PBS | PBS | / | ip, qw | 576.7±89 | - |
| G2 | 1E7 γδ T cells/injection, iv, qw | PBS | / | | 579.0±79 | -3 |
| G3 | | SDP01716 | 3 mpk | | 149.2±57 | 74** |
| G4 | | SDP01696 | 3.9 mpk | | 296.5±60 | 49 |
| G5 | | SDP01704 | 5.1 mpk | | 217.6±69 | 64** |

| | | | | | | |
|---|---|---|---|---|---|---|
| Note: ** represents P < 0.01. | | | | | | |

## Claims

1. A GPC3/TRGV9-binding protein, comprising:
a first antigen-binding domain that specifically binds to GPC3, and
a second antigen-binding domain that specifically binds to TRGV9,
wherein:
the first antigen-binding domain comprises a heavy chain variable region (VH1) and a light chain variable region (VL1);
the VH1 comprises a HCDR1, a HCDR2, and a HCDR3 from the amino acid sequence set forth in SEQ ID NO: 39;
the VL1 comprises a LCDR1, a LCDR2, and a LCDR3 from the amino acid sequence set forth in SEQ ID NO: 40;
the CDRs are defined according to the Kabat, IMGT, Chothia, AbM, or Contact numbering scheme;
preferably, the CDRs are defined according to the Kabat numbering scheme;
preferably, the amino acid sequences of the HCDR1, the HCDR2, and the HCDR3 are set forth in SEQ ID NOs: 41-43, respectively, and the amino acid sequences of the LCDR1, the LCDR2, and the LCDR3 are set forth in SEQ ID NOs: 44-46, respectively.

2. The GPC3/TRGV9-binding protein according to claim 1, wherein the second antigen-binding domain comprises an immunoglobulin single variable domain, or comprises a heavy chain variable region (VH2) and a light chain variable region (VL2), wherein:
the immunoglobulin single variable domain comprises a CDR1, a CDR2, and a CDR3 from the amino acid sequence set forth in any one of SEQ ID NOs: 29, 26-28, and 5;
the VH2 comprises a HCDR1, a HCDR2, and a HCDR3 from the amino acid sequence set forth in any one of SEQ ID NOs: 31, 9, 30, and 32, and the VL2 comprises a LCDR1, a LCDR2, and a LCDR3 from the amino acid sequence set forth in any one of SEQ ID NOs: 34, 10, 33, and 35;
the CDRs are defined according to the Kabat, IMGT, Chothia, AbM, or Contact numbering scheme;
preferably, the CDRs are defined according to the Kabat numbering scheme;
preferably, the immunoglobulin single variable domain comprises a CDR1, a CDR2, and a CDR3 whose amino acid sequences are set forth in SEQ ID NOs: 6-8, respectively;
the amino acid sequences of the HCDR1, the HCDR2, and the HCDR3 of the VH2 are set forth in SEQ ID NOs: 11-13, respectively, and the amino acid sequences of the LCDR1, the LCDR2, and the LCDR3 of the VL2 are set forth in SEQ ID NOs: 14-16, respectively.

3. The GPC3/TRGV9-binding protein according to claim 1 or 2, wherein:
the immunoglobulin single variable domain, the heavy chain variable region, and/or the light chain variable region are/is engineered by humanization, back mutation, affinity maturation, T cell epitope removal, reduction of antibody deamidation, and/or reduction of antibody isomerization;
preferably, framework regions of a human germline template used for humanization engineering of the immunoglobulin single variable domain are derived from IGHV3-64;
preferably, framework regions of a human germline template used for humanization engineering of the VH2 are derived from IGHV3-21, and/or framework regions of a human germline template used for humanization engineering of the VL2 are derived from IGKV1-12.

4. The GPC3/TRGV9-binding protein according to any one of claims 1 to 3, wherein:
the VH1 in the first antigen-binding domain comprises the amino acid sequence set forth in SEQ ID NO: 39 or an amino acid sequence having at least 80% or at least 90% identity thereto, and the VL1 comprises the amino acid sequence set forth in SEQ ID NO: 40 or an amino acid sequence having at least 80% or at least 90% identity thereto.

5. The GPC3/TRGV9-binding protein according to any one of claims 1 to 4, wherein:
the immunoglobulin single variable domain comprises the amino acid sequence set forth in any one of SEQ ID NOs: 29, 26-28, and 5 or an amino acid sequence having at least 80% or at least 90% identity thereto;
the VH2 in the second antigen-binding domain comprises the amino acid sequence set forth in any one of SEQ ID NOs: 9 and 30-32 or an amino acid sequence having at least 80% or at least 90% identity thereto, and the VL2 comprises the amino acid sequence set forth in any one of SEQ ID NOs: 10 and 33-35 or an amino acid sequence having at least 80% or at least 90% identity thereto.

6. The GPC3/TRGV9-binding protein according to any one of claims 1 to 5, further comprising an immunoglobulin Fc region, wherein:
preferably, the Fc region is a human IgG1, human IgG2, human IgG3, or human IgG4 Fc region;
more preferably, the Fc region is a human IgG1 Fc region;
most preferably, the Fc region is an IgG1 Fc region comprising mutation L234F and/or mutation L235E.

7. The GPC3/TRGV9-binding protein according to claim 6, wherein the Fc region comprises a first subunit and a second subunit;
preferably, the first subunit and the second subunit of the Fc region comprise knob-into-hole mutations;
preferably, the first subunit of the Fc region comprises a mutation at position 366, and the second subunit comprises a mutation selected from the group consisting of positions 366, 368, and 407 or any combination thereof; the first subunit of the Fc region comprises a mutation at position 354 or 356, and the second subunit comprises a mutation at position 349; or
the first subunit of the Fc region comprises a mutation at position 354 or 356, and the second subunit comprises a mutation at position 349, 366, 368, or 407 or any combination thereof;
more preferably, the first subunit of the Fc region comprises mutation 366W, and the second subunit comprises a mutation selected from the group consisting of 366S, 368A, and 407V or any combination thereof; the first subunit of the Fc region comprises mutation 354C or 356C, and the second subunit comprises mutation 349C; or the first subunit of the Fc region comprises mutations 354C/366W, and the second subunit comprises mutations 349C/366S/368A/407V;
mutation sites are numbered according to the Eu numbering scheme.

8. The GPC3/TRGV9-binding protein according to any one of claims 1 to 7, further comprising a linker, wherein:
preferably, the linker is represented by (GₘSₙ)ₕ or (GₘQₙ)ₕ or (GGNGT)ₕ or (YGNGT)ₕ or (EPKSS)ₕ or (AₘSₙ)ₕ, wherein m and n are each independently selected from the group consisting of integers of 1-8, and h is independently selected from the group consisting of integers of 1-20;
more preferably, the linker is A₃S or G₄S.

9. The GPC3/TRGV9-binding protein according to any one of claims 1 to 8, comprising any one combination of polypeptide chains selected from the group consisting of (1)-(3):
(1) a first heavy chain, a second heavy chain, and a light chain, wherein:
the first heavy chain, from the N-terminus to the C-terminus, is [immunoglobulin single variable domain]-[linker 1]-[Fc1],
the second heavy chain, from the N-terminus to the C-terminus, is [VH1]-[linker 2]-[CH1]-[linker 3]-[Fc2], and
the light chain, from the N-terminus to the C-terminus, is [VL1]-[linker 4]-[CL];
wherein the linker 1, the linker 2, the linker 3, and the linker 4 may be identical or different, may independently be present or absent, and may be independently selected from the group consisting of the linker defined in claim 8;
preferably, the linker 1 is AAAS, and the linker 2, the linker 3, and the linker 4 are absent;
(2) a first heavy chain, a first light chain, a second heavy chain, and a second light chain, wherein:
the first heavy chain, from the N-terminus to the C-terminus, is [VH2]-[linker 1]-[Obscurin-O chain]-[linker 2]-[Fc1],
the first light chain, from the N-terminus to the C-terminus, is [VL2]-[linker 3]-[Titin-T chain],
the second heavy chain, from the N-terminus to the C-terminus, is [VH1]-[linker 4]-[CH1]-[linker 5]-[Fc2], and
the second light chain, from the N-terminus to the C-terminus, is [VL1]-[linker 6]-[CL]; or
the first heavy chain, from the N-terminus to the C-terminus, is [VH1]-[linker 1]-[Obscurin-O chain]-[linker 2]-[Fc1],
the first light chain, from the N-terminus to the C-terminus, is [VL1]-[linker 3]-[Titin-T chain],
the second heavy chain, from the N-terminus to the C-terminus, is [VH2]-[linker 4]-[CH1]-[linker 5]-[Fc2], and
the second light chain, from the N-terminus to the C-terminus, is [VL2]-[linker 6]-[CL];
wherein the linker 1, the linker 2, the linker 3, the linker 4, the linker 5, and the linker 6 may be identical or different, may independently be present or absent, and may be independently selected from the group consisting of the linker defined in claim 8;
preferably, the linker 1 and the linker 3 are G₄S, and the linker 2, the linker 4, the linker 5, and the linker 6 are absent;
(3) a first heavy chain, a first light chain, a second heavy chain, and a second light chain, wherein:
the first heavy chain, from the N-terminus to the C-terminus, is [VH2]-[linker 1]-[Obscurin-O chain]-[linker 2]-[Fc1],
the first light chain, from the N-terminus to the C-terminus, is [VL2]-[linker 3]-[Titin-T chain],
the second heavy chain, from the N-terminus to the C-terminus, is [VH1]-[linker 4]-[CH1]-[linker 5]-[VH1]-[linker 6]-[CH1]-[linker 7]-[Fc2], and
the second light chain, from the N-terminus to the C-terminus, is [VL1]-[linker 8]-[CL]; or
the first heavy chain, from the N-terminus to the C-terminus, is [VH1]-[linker 1]-[Obscurin-O chain]-[linker 2]-[Fc1],
the first light chain, from the N-terminus to the C-terminus, is [VL1]-[linker 3]-[Titin-T chain],
the second heavy chain, from the N-terminus to the C-terminus, is [VH2]-[linker 4]-[CH1]-[linker 5]-[VH2]-[linker 6]-[CH1]-[linker 7]-[Fc2], and
the second light chain, from the N-terminus to the C-terminus, is [VL2]-[linker 8]-[CL];
wherein the linker 1, the linker 2, the linker 3, the linker 4, the linker 5, the linker 6, the linker 7, the linker 8, the linker 9, and the linker 10 may be identical or different, may independently be present or absent, and may be independently selected from the group consisting of the linker described in claim 8;
preferably, the linker 1 and the linker 3 are G₄S, the linker 5 is (G₄S)₂, and the linker 2, the linker 4, the linker 6, the linker 7, and the linker 8 are absent;
wherein in the above (1)-(3), - represents a peptide bond;
preferably, the amino acid sequences of the Obscurin-O chain and the Titin-T chain are set forth in SEQ ID NOs: 47 and 48, respectively;
preferably, the CL is a Cκ, and the amino acid sequences of the CH1 and the Cκ are set forth in SEQ ID NOs: 49 and 50, respectively;
preferably, the amino acid sequences of the Fc1 and the Fc2 are set forth in SEQ ID NOs: 52 and 53, respectively.

10. The GPC3/TRGV9-binding protein according to any one of claims 1 to 9, comprising any one combination of polypeptide chains selected from the group consisting of (1)-(3):
(1) a first heavy chain whose amino acid sequence is set forth in SEQ ID NO: 54 or has at least 80% or at least 90% identity thereto, a second heavy chain whose amino acid sequence is set forth in SEQ ID NO: 55 or has at least 80% or at least 90% identity thereto, and a light chain whose amino acid sequence is set forth in SEQ ID NO: 56 or has at least 80% or at least 90% identity thereto;
(2) a first heavy chain whose amino acid sequence is set forth in SEQ ID NO: 57 or has at least 80% or at least 90% identity thereto, a first light chain whose amino acid sequence is set forth in SEQ ID NO: 58 or has at least 80% or at least 90% identity thereto, a second heavy chain whose amino acid sequence is set forth in SEQ ID NO: 59 or has at least 80% or at least 90% identity thereto, and a second light chain whose amino acid sequence is set forth in SEQ ID NO: 56 or has at least 80% or at least 90% identity thereto; or
(3) a first heavy chain whose amino acid sequence is set forth in SEQ ID NO: 57 or has at least 80% or at least 90% identity thereto, a first light chain whose amino acid sequence is set forth in SEQ ID NO: 58 or has at least 80% or at least 90% identity thereto, a second heavy chain whose amino acid sequence is set forth in SEQ ID NO: 60 or has at least 80% or at least 90% identity thereto, and a second light chain whose amino acid sequence is set forth in SEQ ID NO: 61 or has at least 80% or at least 90% identity thereto;
wherein preferably, the GPC3/TRGV9-binding protein comprises any one combination of polypeptide chains selected from the group consisting of (1)-(3):
(1) polypeptide chains whose amino acid sequences are set forth in SEQ ID NOs: 54-56;
(2) polypeptide chains whose amino acid sequences are set forth in SEQ ID NOs: 56-59; and
(3) polypeptide chains whose amino acid sequences are set forth in SEQ ID NOs: 57, 58, 60, and 61;
more preferably, the GPC3/TRGV9-binding protein comprises any one combination of polypeptide chains selected from the group consisting of (1)-(3):
(1) polypeptide chains whose amino acid sequences are set forth in SEQ ID NOs: 54-56 in a molar ratio of 1:1:1;
(2) polypeptide chains whose amino acid sequences are set forth in SEQ ID NOs: 56-59 in a molar ratio of 1:1:1:1; and
(3) polypeptide chains whose amino acid sequences are set forth in SEQ ID NOs: 57, 58, 60, and 61 in a molar ratio of 1:1:1:2.

11. The GPC3/TRGV9-binding protein according to any one of claims 1 to 10, being an antibody or an antigen-binding fragment thereof, preferably an anti-GPC3/TRGV9 antibody or an antigen-binding fragment thereof.

12. A TRGV9-binding protein, comprising:
(1) an immunoglobulin single variable domain, comprising a CDR1, a CDR2, and a CDR3 from the amino acid sequence set forth in any one of SEQ ID NOs: 29, 26-28, and 5, preferably a CDR1, a CDR2, and a CDR3 from the amino acid sequences set forth in SEQ ID NOs: 6-8; or
(2) a heavy chain variable region (VH) and/or a light chain variable region (VL), wherein the VH comprises a HCDR1, a HCDR2, and a HCDR3 from the amino acid sequence set forth in any one of SEQ ID NOs: 9 and 30-32, and the VL comprises a LCDR1, a LCDR2, and a LCDR3 from the amino acid sequence set forth in any one of SEQ ID NOs: 10 and 33-35;
wherein the CDRs are defined according to the Kabat, IMGT, Chothia, AbM, or Contact numbering scheme, for example, according to the Kabat numbering scheme.

13. The TRGV9-binding protein according to claim 12, wherein the immunoglobulin single variable domain, the VH, and/or the VL are/is engineered by humanization, back mutation, affinity maturation, T cell epitope removal, reduction of antibody deamidation, and/or reduction of antibody isomerization;
preferably, framework regions of a human germline template used for humanization engineering of the immunoglobulin single variable domain are derived from IGHV3-64; preferably, framework regions of a human germline template used for humanization engineering of the VH are derived from IGHV3-21, and/or framework regions of a human germline template used for humanization engineering of the VL are derived from IGKV1-12.

14. The TRGV9-binding protein according to claim 12 or 13, wherein:
(1) the immunoglobulin single variable domain comprises the amino acid sequence set forth in any one of SEQ ID NOs: 29, 26-28, and 5 or an amino acid sequence having at least 80% or at least 90% identity thereto; or
(2) the VH comprises the amino acid sequence set forth in any one of SEQ ID NOs: 9 and 30-32 or an amino acid sequence having at least 80% or at least 90% identity thereto, and the VL comprises the amino acid sequence set forth in any one of SEQ ID NOs: 10 and 33-35 or an amino acid sequence having at least 80% or at least 90% identity thereto; preferably, the TRGV9-binding protein is an anti-TRGV9 antibody or an antigen-binding fragment thereof;
more preferably, when the TRGV9-binding protein comprises an immunoglobulin single variable domain, the immunoglobulin single variable domain is an anti-TRGV9 single-domain antibody or VHH.

15. The TRGV9-binding protein according to any one of claims 12 to 14, further comprising an immunoglobulin Fc region, wherein:
preferably, the Fc region is a human IgG1, human IgG2, human IgG3, or human IgG4 Fc region; more preferably, the Fc region is a human IgG1 Fc region;
preferably, the Fc region is an IgG1 Fc region comprising mutation L234F and/or mutation L235E.

16. The TRGV9-binding protein according to any one of claims 12 to 15, further comprising a tumor-associated antigen (TAA)-binding domain or a tumor-specific antigen-binding domain, preferably an antigen-binding domain that specifically binds to GPC3.

17. The GPC3/TRGV9-binding protein according to any one of claims 1 to 11 or the TRGV9-binding protein according to any one of claims 12 to 16, having any one or more properties or functions selected from the group consisting of (a)-(d):
(a) specific binding to a γ9 chain of a TCR, preferably specific binding to a γ9δ1 TCR or a γ9δ2 TCR;
(b) undetectable binding to a γ8 chain of the TCR, preferably undetectable binding to a γ8δ2 TCR;
(c) specific binding to a variable region of the γ9 chain of the TCR (TRGV9); and
(d) specific binding to a VγδCαβ chimeric TCR and undetectable binding to a VαβCγδ chimeric TCR.

18. A polynucleotide, encoding the GPC3/TRGV9-binding protein according to any one of claims 1 to 11 or the TRGV9-binding protein according to any one of claims 12 to 16, or a combination thereof, wherein:
preferably, the polynucleotide is a DNA or RNA.

19. A vector, comprising the polynucleotide according to claim 18, wherein:
preferably, the vector expresses the polynucleotide according to claim 18.

20. A host cell, comprising or expressing the polynucleotide according to claim 18 or the vector according to claim 19.

21. A method for preparing the GPC3/TRGV9-binding protein according to any one of claims 1 to 11 or the TRGV9-binding protein according to any one of claims 12 to 16, comprising:
expressing the polynucleotide according to claim 18 or the vector according to claim 19 in the host cell according to claim 20, and isolating an expressed GPC3/TRGV9-binding protein or TRGV9-binding protein from the host cell,
wherein optionally, the method further comprises a step of purifying the GPC3/TRGV9-binding protein or the TRGV9-binding protein.

22. A pharmaceutical composition, comprising the GPC3/TRGV9-binding protein according to any one of claims 1 to 11, the TRGV9-binding protein according to any one of claims 12 to 16, the polynucleotide according to claim 18, and/or the vector according to claim 19, wherein:
preferably, the pharmaceutical composition further comprises a T cell, and the T cell is preferably a γδ T cell;
preferably, the pharmaceutical composition further comprises one or more pharmaceutically acceptable excipients, diluents, or auxiliary materials.

23. A combination or kit, comprising:
(1) the GPC3/TRGV9-binding protein according to any one of claims 1 to 11 and a T cell, or
(2) the TRGV9-binding protein according to any one of claims 12 to 16 and a T cell, wherein the T cell is preferably a γδ T cell.

24. Use of the GPC3/TRGV9-binding protein according to any one of claims 1 to 11, the TRGV9-binding protein according to any one of claims 12 to 16, the polynucleotide according to claim 18, the vector according to claim 19, or the combination or kit according to claim 23 in the manufacture of a medicament for treating a disease, wherein:
preferably, the disease is cancer;
more preferably, the disease is liver cancer or GPC3-positive cancer; most preferably, the disease is GPC3-positive liver cancer.

25. Use of the GPC3/TRGV9-binding protein according to any one of claims 1 to 11, the TRGV9-binding protein according to any one of claims 12 to 16, the polynucleotide according to claim 18, or the vector according to claim 19 in the manufacture of a medicament for treating a disease, wherein:
the GPC3/TRGV9-binding protein, the TRGV9-binding protein, the polynucleotide, or
the vector is used in combination with a T cell;
the T cell is preferably a γδ T cell;
preferably, the disease is cancer; more preferably, the disease is liver cancer or GPC3-positive cancer; most preferably, the disease is GPC3-positive liver cancer.

26. Use of a T cell for the manufacture of a medicament for treating a disease, wherein:
the T cell is used in combination with the GPC3/TRGV9-binding protein according to any one of claims 1 to 11, the TRGV9-binding protein according to any one of claims 12 to 16, the polynucleotide according to claim 18, or the vector according to claim 19;
the T cell is preferably a γδ T cell;
preferably, the disease is cancer; more preferably, the disease is liver cancer or GPC3-positive cancer; most preferably, the disease is GPC3-positive liver cancer.

27. A method for treating or alleviating a disease, comprising administering to a subject in need thereof a therapeutically or palliatively effective amount of the GPC3/TRGV9-binding protein according to any one of claims 1 to 11, the TRGV9-binding protein according to any one of claims 12 to 16, the polynucleotide according to claim 18, the vector according to claim 19, the pharmaceutical composition according to claim 22, or the combination or kit according to claim 23, wherein:
preferably, the disease is cancer; more preferably, the disease is liver cancer or GPC3-positive cancer; most preferably, the disease is GPC3-positive liver cancer.
